# EUROPEAN PATENT SPECIFICATION

(11) **EP 2 021 356 B1**
(45) Date of publication and mention of the grant of the patent: **27.10.2010**
(21) Application number: 07722719.7
(22) Date of filing: 01.06.2007
(51) Int. Cl.: C07K 14/16, A61K 38/16, A61K 39/21

(54) **HIV VACCINE**
HIV-IMPFSTOFF
VACCIN CONTRE LE VIH

(30) Priority: 01.06.2006 DK 200600750
(43) Date of publication of application: 11.02.2009
(73) Proprietor: Statens Serum Institut, DK-2300 Copenhagen S (DK)
(72) Inventor: FOMSGAARD, Anders, 2800 Lyngby (DK)
(74) Representative: Plougmann & Vingtoft A/S
(86) International application number: PCT/DK2007/050066
(87) International publication number: WO 2007/137591

(56) References cited:
- WO-A-01/24810
- WO-A-01/55177
- US-A1- 2002 182 222
- CORBET S ET AL.: "Optimization and immune recognition of multiple novel conserved HLA-A2, human immunodeficiency virus type 1-specific CTL epitopes" JOURNAL OF GENERAL VIROLOGY, vol. 84, no. 9, September 2003 (2003-09), pages 2409-2412, XP002460046 ISSN: 0022-1317 cited in the application
- THORN M ET AL.: "Sequence conservation of subdominant HLA-A2-binding CTL epitopes in HIV-1 clinical isolates and CD8+ T-lymphocyte cross-recognition may explain the immune reaction in infected individuals" APMIS, vol. 115, 2007, pages 757-768, XP002460047

## Description

### FIELD OF INVENTION

The present invention relates to compositions of amino acid sequences derived from human immunodeficiency virus, the compositions being in the form of for instance a vaccine to a method of producing the composition and to the use of the composition. The invention further relates to nucleic acid molecules encoding the amino acid sequences, to a composition comprising these nucleic acid molecules, as well as to the use of the composition. Finally the invention relates to a method of treating an individual being infected with a human immunodeficiency virus or reducing the risk of infection with a human immunodeficiency virus, the method comprising administering to the individual a composition comprising an amino acid sequence corresponding to a CTL epitope from a virus isolate from the individual and/or a CTL epitope which is found in virus isolates from more than 50% of a group of infected individuals, or a composition comprising a nucleic acid sequence coding for such an amino acid sequence.

### BACKGROUND OF THE INVENTION

Since HIV was identified as the cause of AIDS in 1983, multiple candidates for a vaccine to prevent HIV infection and AIDS have been tested in human trials. The international AIDS vaccine initiative, IAVI, maintains a database of these vaccines and trials (www.IAVI.org). Nearly all of these trials have been very early (phase I) tests of vaccine safety and preliminary immune response. Only one vaccine (two formulations of the same basic gp120 vaccine) has been tested in large-scale Phase III studies. The VaxGen gp120 subtype B protein was found ineffective in a phase III trial that was completed in 2003 in USA, Canada and Netherlands. Later in 2003, a second trial of AIDSVAX was completed in Thailand. Both trials found the candidates to be ineffective. It has previously been difficult to prepare protein vaccines against HIV which may be due to the high diversity in the envelope protein, the differences between the envelope of the laboratory adapted strain used and the clinical isolates, the monomeric nature of the gp120 in the vaccine and the trimeric organisation in the virus, and in particular because only antibodies and not cellular immunity are induced. A combination of AIDSVAX protein (from VAXGene) with *genes* delivered in canary pox (ALVAC from Aventis Pasteur) is also in phase III (www.IAVI.org for further information) and a fourth large scale trial is expected to begin testing Merck's adenovirus-based candidate. Cytotoxic T-lymphocytes (CTL) are considered a critical component of immune control of virus including HIV-1 and relevant CTL immunogens are considered for therapeutic vaccines. CTLs are the first virus-specific immune effectors to be detected in HIV-1 infected individuals with primary infection and their expansion is associated with virus control (Koup *et al.,* 1994; Borrow *et al.,* 1994; Musey *et al.,* 1997). Although partial control of infection is the likely reason for different rates of progression the natural HIV-1 infection does not give life-long protective immunity. Therefore, a therapeutic vaccine must target different and multiple epitopes present in the patients virus in a more potent way to induce a broader and stronger immunity than the natural infection does. In order to prevent and/or control HIV-1 infections via vaccine-induced activating CTL, the identification of relevant CTL target epitopes, and the rational selection and design of corresponding CTL epitope immunogens and suitable mixtures of relevant CTL epitope immunogens is of great importance. A broad CTL response helps to control emerging virus variants and is a critical factor in determining the rate of progression to disease (Chouquet *et al.,* 2002). Moreover, the epitopes from highly conserved regions identified so far has been found to have little or no amino acid identity to the most prevalent circulating HIV-1 strains (McKinney *et al.,* 2004). HIV-1 variation and escape from specific CTL to frequently targeted immunodominant epitopes represents a significant obstacle in the development of therapeutic vaccines able to control viral replication in infected individuals. Immunodominant epitopes were the first to be identified and are subjected to vaccine approaches (McMichael *et al.,* 2001). The effect of such vaccines remains to be seen.

Epitopes that are infrequently targeted during infection and represent poorly immunogenic subdominant epitopes may be more conserved. Epitopes infrequently targeted during the infection will provide new targets if a vaccine can induce immunity towards them. The infection may induce CTL against only a limited number of epitopes that increase the risk of the development of escape mutations and in HIV proteins that may be less optimal targets during the virus life cycle in the infected cells. Therefore, a different approach in HIV-1 therapeutic vaccination would be attempts to force the immune system to recognise and target multiple and subdominant HIV-1 epitopes. Broad CTL responses to structural, accessory and early regulatory proteins are preferable to meet the high diversity of HIV-1 and target the virus during different stages of its replication cyclus. However, the low sequence conservation of epitopes in these short and variable proteins render their identification particularly difficult. As the epitopes may not be identified by traditional methods "reverse immunology" has previously been employed in the form of Artificial Neural Network predictions of MHC-I binding (WO0155177, Corbet et al., 2003). In Corbet *et al.* it was further proposed that novel HIV vaccines should contain multiple subdominant epitopes that are globally more conserved, within certain subtypes or within a defined geographical area. Moreover, Corbet et al. suggested including variants of natural epitopes that have been anchor optimised, as some anchor optimised epitopes were found to have improved immunogenicity.

It was observed by Corbet *et al.* that their identified epitopes were conserved to a certain degree among the various circulating HIV subtypes and the authors were further able to demonstrate that certain anchor optimised variants of an HIV-1 epitope could cross-react with the naturally occurring epitope. Nevertheless, when designing tailor-made vaccines one would expect to have to develop a large repertoire of epitopes in order to target as many HIV-proteins as possible and to assure an adequately broad coverage.

### SUMMARY OF THE INVENTION

In order to investigate further the possibility of developing a tailor-made HIV vaccine tailored to HLA-A2 positive individuals whose virus strain contain relevant target epitopes only weakly or not yet targeted full-length HIV-1 genomes from A2 positive untreated patients were sequenced and the presence and conservation of the identified new putative HLA-A2-restricted HIV-1 CTL epitopes have been investigated in the clinical isolates. The results have been compared with the patients' corresponding CD8⁺ T-cell immune response to these epitopes raised during the infection. Also the possibility of broadly covering such clinically relevant HIV-1 target epitopes and their variants by more immunogenic variant or modified epitope immunogens has been investigated.

The present invention is based on the observation that sequence conservation of a number of identified HLA-A2-binding HIV-1 infrequently targeted CTL epitopes was high among virus isolates from patients. In addition, cross-reacting variants were identified that resulted in a surprisingly broad coverage of the variant target antigens. The target epitopes present were infrequently targeted during the infection since no recall immune respons could be induced and thus will provide new targets for a vaccine containing more immunogenic and cross-reacting versions of the epitope. Optimal target epitopes could be identified in Gag, Pol, Env, Vif, Vpr, and Vpu, based on this sequence conservation and measured patient CTL immune recognition. It is therefore possible to combine a few selected peptides comprising immune optimised versions of epitopes from these proteins in a composition which, when used as a vaccine targeting these epitopes, has a broad coverage. It is seen that the coverage is broad in the newly sequenced Danish patient HIV isolates as well as in HIV sequences in the HIV databases representing different HIV subtypes from different geographical areas. Such a vaccine may be effective even though including only relatively few immunogens identified among natural rare epitopes or modified artificially anchor-optimized epitopes. The vaccine may be contemplated for tailor-made prophylactic and/or therapeutic vaccinations e.g. of a specific group of patients. Such group could comprise HLA-A2 positive individuals whose HIV strains or HIV strains predominantly circulating in the particular geographical area contain several of the identified target epitopes.

In particular, the invention is based on a selection of 11 amino acid sequences representing minimal epitopes selected among those identified in WO0155177, Corbet et al., 2003 and on peptide variants of these epitopes. The epitopes were selected for their targets epitopes ability to induce cytotoxicity and/or IFN-γ in groups of immunized HLA-A2 transgenic mice and/or they were selected as being recognised by CD8⁺ T cells in HIV-1 infected A2+ and A2- patients as measured by intracellular flow cytometry (Fluorescence Activated Cell Sorting), IC-FACS IFN-γ. The 11 epitopes were also selected because they represents minimal epitope versions that may be optimised to induce a stronger immunity than the target epitope and still induce cross-reaction to the less immunogenic target epitope or target epitope variant. In addition, the 11 vaccine epitopes were selected because each of their corresponding target epitope was found to be present by sequencing in HIV-1 clade B and C isolates from at least one individual within a group of HLA-A2 patients or new variants of the epitope were found present that the vaccine epitope was shown to induce cross-reaction to. Furthermore the epitopes fulfilled the criteria of being infrequently targeted within the same group of patients. Moreover the vaccine epitope mix selected will target epitopes that are frequently "coupled" or present simultaneously within the clinical HIV isolate. Thus, the selected mix of 11 vaccine epitopes will result in targeting the clinical HIV isolate on multiple areas (from 4-9 epitopes in 3-5 different proteins) simultaneously.

Therefore, a main aspect of the invention provides a composition comprising at the most 20 amino acid sequences selected from the group consisting of:
X₁-X₂-ADQLIH-X₃ (SEQ ID NO: 1)
   wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
   wherein X₁ is selected from the group consisting of: V, I, T and R;
X₁-X₂-VEMGHH-X₃ (SEQ ID NO: 3)
   wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
   wherein X₁ is selected from the group consisting of: T and L;
FLG-X₁-IW-X₂-X₃ (SEQ ID NO: 5)
   wherein X₁ is selected from the group consisting of K and R; X₂ is selected from the group consisting of: P and S; and X₃ is selected from the group consisting of: V and S;
K-X₁-GKAGYV-X₂ (SEQ ID NO: 6)
   wherein X₁ is selected from the group consisting of: L, M, I and T; and X₂ is selected from the group consisting of: T and V;
N-X₁-WATHACV (SEQ ID NO: 7)
   wherein X₁ is selected from the group consisting of: V and I;
VLAEAMSQ-X₁ (SEQ ID NO: 8)
   wherein X₁ is selected from the group consisting of: V, M and A;
N-X₁-VATLYCV (SEQ ID NO: 9)
   wherein X₁ is selected from the group consisting of: T and L;
Y-X₁-X₂-IFIMI-X₃ (SEQ ID NO: 10)
   wherein X₁ is selected from the group consisting of: I and L; X₂ is selected from the group consisting of: K and R; and X₃ is selected from the group consisting of: V and I;
X₁-LGQHIY-X₂-T (SEQ ID NO: 11)
   wherein X₁ is selected from the group consisting of: S, G and N; and X₂ is selected from the group consisting of: E and N
   and wherein said composition comprises the amino acid sequences:
X1-X₂-ADQLIH-X₃ (SEQ ID NO: 1)
   wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
   wherein X₁ is selected from the group consisting of: V, I, T and R;
X₁-X₂-VEMGHH-X₃ (SEQ ID NO: 3)
   wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
   wherein X₁ is selected from the group consisting of: T and L;
FLG-X₁-IW-X₂-X₃ (SEQ ID NO: 5)
   wherein X₁ is selected from the group consisting of K and R; X₂ is selected from the group consisting of: P and S, and X₃ is selected from the group consisting of: V and S;
K-X₁-GKAGYV-X₂ (SEQ ID NO: 6)
   wherein X₁ is selected from the group consisting of: L, M, I and T; and X₂ is selected from the group consisting of: T and V; and
N- X₁-WATHACV (SEQ ID NO: 7)
   wherein X₁ is selected from the group consisting of: V and I.

Another aspect of the invention provides a nucleic acid molecule consisting of a sequence of nucleic acids encoding at the most 20 and at the least 7 of the amino acid sequences as defined above.

Further provided is a composition comprising a nucleic acid molecule according to the invention.

Yet another aspect of the invention pertains to a composition or a nucleic acid molecule according to the invention for use in medicine.

The invention further relates to the use of a composition or a nucleic acid sequence according to the invention in the manufacture of a medicament for the prevention and/or treatment of human immunodeficiency virus infections/acquired immunodeficiency syndrome.

### DEFINITIONS

In the context of the present invention the amino acids are designated using the conventional one-letter code. Thus, A designates alanine, R designates arginine, N designates asparagine, D designates aspartic acid, C designates cysteine, E designates glutamic acid, Q designates glutamine, G designates glycine, H designates histidine, I designates isoleucine, L designates leucine, K designates lysine, M designates methionine, F designates phenylalanine, S designates serine, T designates threonine, W designates tryptophan, Y designates tyrosine and V designates valine.

The short term CTL refers to cytotoxic T-lymphocytes.

The term "target epitope" refers in the context of the present invention to an epitope of a HIV protein.

### DETAILED DESCRIPTION OF THE INVENTION

### Composition comprising at least 7 amino acid sequences

A total of 11 antigenic epitopes were selected in 6 HIV-1 proteins, namely 2 in Vif, 1 in Vpu, 4 in Gag, 1 in Pol, 2 in Env, 1 in Vpr. By sequencing viral isolates from infected individuals and examining the corresponding immune recognition in the individuals several variants of the target epitopes were found. Examination of cross recognition of variant epitopes in patients and cross reactions induced by the optimal vaccine immunogens in HLA-A2 transgenic mice revealed that such variants may also be used in composition which is suitable as a vaccine thus broadening the coverage of a vaccine. In this context the term "cross recognition" refers to that the immune system of a patient is able to recognize an epitope which is different from the epitope expressed by the HIV virus by which the patient is infected.

**Table 1. Selected immunogens and their variants**

| | | Optimized immunogen (SEQ ID NO:) | Epitope occurring with the highest frequency (SEQ ID NO:) | Variants in the Position 1 | Variants in Position 2 (Primary MHC-I anchor) |
|---|---|---|---|---|---|
| 1 | Vif101 | GLADQLIHL (12) | GLADQLIHM (23) | S,E,N,D,H | M,I,T,V |
| 2 | Vif23 | SLVKHHMYV (13) | SLVKHHMYI (24) | | |
| 3 | Vpu66 | ALVEMGHHV (14) | ALVEMGHHA (25) | I | L,M, |
| 4 | Gag150 | RLLNAWVKV (15) | RTLNAWVKV (26) | | L,T |
| 5 | Gag433 | FLGKIWPV (16) | FLGKIWPS (27) | | |
| 6 | Pol606 | KLGKAGYVV (17) | KLGKAGYVT (28) | | M,I,T |
| 7 | Env67 | NIWATHACV (18) | NVWATHACV (29) | | V,I,L |
| 8 | Gag362 | VLAEAMSQV (19) | VLAEAMSQA (30) | | |
| 9 | Gag80 | NTVATLYCV (20) | NTVATLYCA (31) | | L |
| 10 | Env681 | YIKIFIMIV (21) | YIKIFIMIV (32) | | L |
| 11 | Vpr41 | SLGQHIYET (22) | SLGQHIYET (22) | G,N | 8N |
| | | | | G,S,E,N,D,I | L,M,I,T,V |
| | | | | | |

| | | Variants in the Position 1 | Variants in Position 2 (Primary MHC-I anchor) | Variants in the positions 3-8 (TCR recognition site) | Variants in the C-terminal position (primary MHC-I anchor) |
|---|---|---|---|---|---|
| 1 | Vif101 | S,E,N,D,H | M,I,T,V | | L,M,Q |
| 2 | Vif23 | | | | V,I,T,R |
| 3 | Vpu66 | I | L,M, | | V,R, A |
| 4 | Gag150 | | L,T | | |
| 5 | Gag433 | | | 7S, 4R | V,S |
| 6 | Pol606 | | M,I,T | | T,V |
| 7 | Env67 | | V,I,L | | |
| 8 | Gag362 | | | | V,M,A |
| 9 | Gag80 | | L | | A,V |
| 10 | Env681 | | L | 3R | I,V |
| 11 | Vpr41 | G,N | 8N | | |
| | | G,S,E,N,D,I | L,M,I,T,V | 3R,4R,7S | L,M,Q,V,I,T,A, S, |

In Table 1 the variants at the different positions indicate those variations of the epitope which occurred with the highest frequency in the patients, which were observed in some patients.

By vaccinating with minimal epitopes one can direct the immunity toward the selected epitopes and exclude possibility for induction of irrelevant and unwanted immune targeting. If a vaccine contains a whole HIV protein or a gene encoding it may induce CTL to some and not other CTL epitopes and target only a limited number of potential immune targets. Moreover the more immunogenic immune dominant epitopes that are more frequently targeted during infection may be selected. Same limited immune induction is seen during an HIV infection and may vary depending on the stage of the infection. Immunisation with selected minimal epitopes on the other hand can help to direct the immunity only to the selcted targets and thus towards other epitopes in selected HIV proteins that is only weakly or not yet targeted. The use of minimal epitopes and the use of immune optimised versions of the target epitopes that can cross-react to the natural epitopes helps this targeting. Moreover a set or mixture of suitable targets in preferred HIV proteins can be selected for providing multiple targets which will minimise the possibility of immune escape. It is an advantage that the target epitopes are present in the patient isolate and that mixtures can be defined that target as many as possible epitopes present in the same virus isolate for simultaneous targeting at different areas of the virus isolate. According to the present invention a combination of 7 of the amino acid sequences represented in table 1 is sufficient to create a composition which is suitable as a prophylactic and/or therapeutic vaccine against HIV, wherein the composition comprises amino acid sequences SEQ ID NOs : 1, 2, 3, 4, 5, 6 and 7. Therefore, in a main aspect the present invention pertains to a composition comprising at at least 7, at least 8, at least 9, at least 10, at least 11, at least 12, at least 13, at least 14, at least 15, at least 16, at least 17, at least 18 or at least 19 amino acid sequences selected from the group consisting of:
X₁-X₂-ADQLIH-X₃ (SEQ ID NO: 1)
   wherein X₁ is selected from the group consisting of: G, S, E, N, D and H; X₂ is selected from the group consisting of: L, M, I, T and V; and X₃ is selected from the group consisting of: L, Q and M;
SLVKHHMY-X₁ (SEQ ID NO: 2)
   wherein X₁ is selected from the group consisting of: V, I, T and R;
X₁-X₂-VEMGHH-X₃ (SEQ ID NO: 3)
   wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
   wherein X₁ is selected from the group consisting of: T and L;
FLG-X₁-IW-X₂-X₃ (SEQ ID NO: 5)
   wherein X₁ is selected from the group consisting of K and R; X₂ is selected from the group consisting of: P and S; and X₃ is selected from the group consisting of: V and S;
K-X₁-GKAGYV-X₂ (SEQ ID NO: 6)
   wherein X₁ is selected from the group consisting of: L, M, I and T; and X₂ is selected from the group consisting of: T and V;
N- X₁-WATHACV (SEQ ID NO: 7)
   wherein X₁ is selected from the group consisting of: V and I;
VLAEAMSQ-X₁ (SEQ ID NO: 8)
   wherein X₁ is selected from the group consisting of: V, M and A;
N-X₁-VATLYCV (SEQ ID NO: 9)
   wherein X₁ is selected from the group consisting of: T and L;
Y-X₁-X₂-IFIMI- X₃ (SEQ ID NO: 10)
   wherein X₁ is selected from the group consisting of: I and L; X₂ is selected from the group consisting of: K and R; and X₃ is selected from the group consisting of: V and I;
X₁-LGQHIY- X₂-T (SEQ ID NO: 11)
   wherein X₁ is selected from the group consisting of: S, G and N; and X₂ is selected from the group consisting of: E and N.

If the composition of the present invention is used as a vaccine against HIV in an individual patient the composition may in particular comprise at least 7 amino acid sequences of the present invention which corresponds to epitopes found in the HIV proteins present in the patient.

If the composition of the present invention is used as a vaccine against HIV in group of patients the combination of amino acid sequences in the composition may in particular be such that it corresponds to at least 7 epitopes found in HIV proteins present in each of the patients.

As can be inferred from the above, the composition may be a pharmaceutical composition, an immunologic composition, a formulation or a vaccine, such as a prophylactic and/or a therapeutic vaccine. It will be understood that in order to provide optimal coverage the composition according to the invention may be composed of amino acid sequences representing as many of the 6 HIV-1 proteins, Vif, Vpu, Gag, Pol, Env and Vpr as possible. Thus each of the amino acid sequences may in particular represent different HIV-1 proteins. Thus, the amino acid sequences may be those represented by SEQ ID NO: 1 (representing Vif) with X₁, X₂ and X₃ as defined above, SEQ ID NO: 3 (representing Vpu) with X₁, X₂ and X₃ as defined above, SEQ ID NO: 4 (representing Gag) with X₁ as defined above, SEQ ID NO: 6 (representing Pol) with X₁ and X₂ as defined above, SEQ ID NO: 7 (representing Env) with X₁ as defined above and SEQ ID NO: 11 (representing Vpr) with X₁ and X₂ as defined above. Similarly, for a composition comprising 11 amino acid sequences as described above the composition may advantageously include one amino acid sequence represented by SEQ ID NO: 1 with X₁, X₂ and X₃ as defined above, one amino acid sequence represented by SEQ ID NO: 2 with X₁ as defined above, one amino acid sequence represented by SEQ ID NO: 3 with X₁, X₂ and X₃ as defined above, one amino acid sequence represented by SEQ ID NO: 4 with X₁ as defined above, one amino acid sequence represented by SEQ ID NO: 5 with X₁, X₂ and X₃ as defined above, one amino acid sequence represented by SEQ ID NO: 6 with X₁ and X₂ as defined above, one amino acid sequence represented by SEQ ID NO: 7 with X₁ as defined above, one amino acid sequence represented by SEQ ID NO: 8 with X₁ as defined above, one amino acid sequence represented by SEQ ID NO: 9 with X₁ as defined above, one amino acid sequence represented by SEQ ID NO: 10 with X₁, X₂ and X₃ as defined above, and one amino acid sequence represented by SEQ ID NO: 11 with X₁ and X₂ as defined above.

While inclusion of sequences derived from all of the 6 genes in the composition may be optimal, it is contemplated that compositions according to the present comprising amino acid sequences representing e.g. 3, 4 or 5 of the 6 different HIV proteins may provide sufficient coverage of particular HIV strains when the composition is used as a vaccine.

In a preferred embodiment the present invention provides a composition comprising the amino acid sequences:
X₁-X₂-ADQLIH-X₃ (SEQ ID NO: 1)
   wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
   wherein X₁ is selected from the group consisting of: V, I, T and R;
X₁-X₂-VEMGHH-X₃ (SEQ ID NO: 3)
   wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
   wherein X₁ is selected from the group consisting of: T and L;
FLG-X₁-IW-X₂-X₃ (SEQ ID NO: 5)
   wherein X₁ is selected from the group consisting of K and R; X₂ is selected from the group consisting of: P and S, and X₃ is selected from the group consisting of: V and S;
K-X₁-GKAGYV-X₂ (SEQ ID NO: 6)
   wherein X₁ is selected from the group consisting of: L, M, I and T; and X₂ is selected from the group consisting of: T and V; and
N- X₁-WATHACV (SEQ ID NO: 7)
   wherein X₁ is selected from the group consisting of: V and I.

For some vaccination procedures like dendritic cell vaccination it may be an advantage if no more than 2, such as 0, 1 or 2, different amino acid sequences representing the same HIV proteins is present in the composition. Without being bound by any theory the inventors of the present invention believe that this may reduce the risk of immune competition, cost and concentration of each epitope peptide in various mixtures. To cover most of the Danish patients in a regional-specific vaccine the inventors of the present invention sequenced virus isolates from a number of patients and established which epitopes are most conserved. In a preferred embodiment the amino acid sequences present in a composition of the present invention corresponds to the epitopes or their variants which have been found in 4 or more of the 11 isolates (more than one third of the isolates).

Table 2 shows the presence of identical epitope sequences in 11 Danish patient HIV-1 isolates and the patient's immune recognition (significant IFNg IC-FACS) of the corresponding epitope. +/+ indicate the presence of an epitope sequence and a positive immune recognition of that epitope; +/- indicate the presence of an epitope but no immune recognition; -/+ indicate the absence of a 100% identical epitope sequence but a positive recall immune reaction to the epitope; -/- indicate the absence of the epitope sequence and the immune reaction to it. Right column summarise among the 11 patients how many patient HIV-1 isolates has the target epitope sequence and how many of the patients which had a positive immune recognition of that epitope. Although an epitope or a cross-reacting epitope is frequently present only few patients recognizes it during the infection (infrequently recognized). At the bottom is summarized how many epitopes are simultaneously present in each patient HIV-1 isolate (from 4-8).

**Table 2**

| **Epitope** | **Sequence** **(SEQ ID NO:)** | **SEQ ID NO:** | **Pt15 subty pe C** | **Pt16 Clade B** | **Pt17 Clade B** | **Pt18 Clade B** | **Pt21 Clade B** | **Pt23 Clade B** |
|---|---|---|---|---|---|---|---|---|
| **Gag 80** | NTVATLYCV (20) | 20 | +/+ | +/- | | +/- | | |
| **Gag 150** | RTLNAWVKV (26) | 23 | +/+ | +/- | +/- | +/- | +/- | +/- |
| **Gag 362(9V)** | VLAEAMSQV (19) | 19 | | +/- | | +/- | +/- | +/+ |
| ***Gag 362(9A)^{var}*** | *VLAEA MSQA* (30) | 24 | +/- | | | -/+ | | -/+ |
| **Gag 433** | FLGKIWPS (16) | 16 | +/- | -/+ | +/- | +/- | +/- | +/- |
| **Pol 606** | KLGKAGYVT (28) | 25 | | | | +/- | +/- | +/- |
| **Env 67(2V)** | NVWATHACV (29) | 26 | +/- | +/- | +/- | +/- | +/- | +/- |
| ***Env 67(2I)^{var}*** | *NIWATHACV (18)* | 18 | | | | | | -/+ |
| **Env 681** | YIKIFIMIV (21) | 21 | +/- | +/+ | | +/- | +/- | -/+ |
| **Vif 23(91)** | *SLVKHHMYI* (24) | 27 | | | +/- | | | +/- |
| ***Vif 23(9V)^{var}*** | SLVKHHMYV (13) | 13 | | | | +/- | | |
| **Vif 101 (9L)** | GLADQLIHL (12) | 12 | | -/+ | | | | -/+ |
| ***Vif 101(9M)^{va} r*** | *GLADQLIHM* (23) | 28 | | | | | | |
| ***Vif 101 (9 I)*** | | 29 | +/- | | | | | |
| ***Vif 101 (1 S)*** | | 30 | | | | | | |
| ***Vif 101 (1 D)*** | | 31 | | | | | +/- | +/+ |
| ***Vif 101 (1 N)*** | | 32 | | +/+ | | | | |
| **Vpr 41** | SLGQHIYET (22) | 22 | | | | | -/nd | +/- |
| **Vpu 66** | ALVEMGHHA (25) | 33 | | | | +/- | +/- | |
| | | | | | | | | |
| **Numbers of vaccine targets per HIV-1 isolate** | | | **7** | **6** | **4** | **9** | **8** | **8** |
| **Numbers of different HIV-1 proteins containing a target epitope** | | | **3** | **3** | **3** | **5** | **5** | **5** |
| **Control Gag 77** | SLYNTVATL | 34 | -/+ | +/+ | -/- | -/+ | | -/+ |
| | | | | | | | | |

| **Epitopes** | **Sequence** | **SEQ ID NO:** | **Pt30 Clade B** | **Pt33 Clade B** | **Pt35 Clade B** | **Pt41 Clade B** | **Pt43 Clade B** | **Total** |
|---|---|---|---|---|---|---|---|---|
| **Gag 80** | NTVATLYCV (20) | 20 | +/- | +/- | +/- | | | **6/1** |
| **Gag 150** | RTLNAWVKV (26) | 23 | +/- | +/- | +/- | +/- | +/- | **11/1** |
| **Gag 362(9V)** | VLAEAMSQV (19) | 19 | +/- | | +/- | +/- | +/- | **8/1** |
| ***Gag 362(9A)^{var}*** | *VLAEAMSQA* (30) | 24 | | +/- | | | | **2/2** |
| **Gag 433** | FLGKIWPS (16) | 16 | +/+ | +/+ | +/- | +/+ | -/+ | **9/4** |
| **Pol 606** | KLGKAGYVT (28) | 25 | +/+ | +/- | -/+ | +/- | +/- | **7/2** |
| **Env 67(2V)** | NVWATHACV (29) | 26 | | +/- | +/- | +/- | +/- | **10/0** |
| ***Env 67(2I)^{var}*** | *NIWATHACV (18)* | 18 | | | | | | **0/1** |
| **Env 681** | YIKIFIMIV (21) | 21 | +/- | +/- | | | +/- | **7/2** |
| **Vif 23(91)** | *SLVKHHMYI* (24) | 27 | | | | | +/+ | **3/1** |
| ***Vif 23(9V)^{var}*** | SLVKHHMYV (13) | 13 | | | | | -/+ | **1/1** |
| **Vif 101 (9L)** | GLADQLIHL (12) | 12 | | | | | | **7/5** |
| ***Vif 101(9M)^{var}*** | *GLADQLIHM*(23) | 28 | | | | -/+ | +/- | |
| ***Vif 101* (9 *I)*** | | 29 | | | | | | |
| ***Vif 101* (1 *S)*** | | 30 | | | +/- | | | |
| ***Vif 101* (1 *D)*** | | 31 | | +/- | | | | |
| ***Vif 101 (1 N)*** | | 32 | | | | | | |
| **Vpr 41** | SLGQHIYET (22) | 22 | | | | | | **1/0** |
| **Vpu 66** | ALVEMGHHA (25) | 33 | +/- | +/- | | | +/- | **5/0** |
| | | | | | | | | |
| **Numbers of vaccine targets per HIV-1 isolate** | | | **7** | **9** | **6** | **5** | **8** | |
| **Numbers of different HIV-1 proteins containing a target epitope** | | | **4** | **5** | **5** | **3** | **5** | |
| **Control Gag 77** | SLYNTVATL | 34 | | | +/- | -/+ | -/+ | **2/6** |

As will be recognised by the person skilled in the art, peptides naturally presented by MHC class I molecules are 8 to 10 amino-acid long and contain specific anchor residues that share properties like hydrophobicity or charge. The anchor residue side chains are deeply embedded within the antigen-presenting groove of the MHC molecule, suggesting that anchor residues are primarily involved in MHC class I binding. Because anchor residue side chains are buried in the peptide binding groove, it is believed that anchor residues are not directly involved in the T-cell response. This suggests that anchor residues can be replaced without affecting T-cell recognition.

In the case of the HLA complex, it is generally accepted that the binding affinity of an HLA-A2 peptide epitope can be improved by replacing non-optimal amino acids in primary anchor positions with more optimal amino acids in said primary anchor positions. It is contemplated that this is the case for all the amino acid sequences according to the invention.

Although this may be a general rule changing of amino acids in an epitope may alter its interaction with T cell receptors so that for example its ability to elicit a T-cell response is altered. Thus it is an advantage to test the effect that an amino acid modification of a natural occurring epitope has on the epitopes interaction with T cell receptors. Thus, using HLA-A2 as an example, the primary anchor positions are defined as the second amino acid in the epitope and the terminal amino acid in the epitope (Ω). For example, the primary anchor positions of a 9 amino acid long HLA-A2 binding motif are located at position 2 and position 9. Hence in a preferred embodiment of the present invention, one or more of the amino acid sequences of the present invention has in anchor position 2 a Leucine, Methionine, Glutamine, or Isoleucine. The most preferred optimal primary anchor amino acid in this context is Leucine at position 2. In another embodiment of the invention, the one or more of the amino acid sequences of the present invention has in anchor position Ω a Valine, Isoleucine, Leucine, or Alanine. The most preferred optimal primary anchor amino acid in this context is a Valine at position 9.

In particular one or more of the amino acid sequences of the present invention has a Leucine, Methionine, Glutamine, or Isoleucine in anchor position 2 and a Valine, Isoleucine, Leucine, or Alanine in anchor position Ω. Even more particularly one or more of the amino acid sequences of the present invention has a Leucine in anchor position 2 and a Valine in anchor position Ω.

It has been found that immunisation with a modified CTL epitope with improved binding affinity often has a higher probability of inducing a CTL response than the natural epitope or that it may induce a higher CTL response than that induced with the natural CTL epitope. Thus, it may in particular be relevant to use peptide epitopes which have been modified to comprise optimal anchor residues when the peptide epitopes are so-called sub-dominant epitopes, i.e. epitopes which are less frequently recognised by the immune system of infected individuals. Furthermore T-cell Receptor (TCR)binding to variant epitopes is relatively unaffected by amino acid substitutions at positions other than the primary MHC-I anchors, such as at the TCR contact residues as a considerable promiscuity of the TCR has been acknowledged. A "TCR contact residue" is an amino acid residue in an epitope that is understood to be bound by a T-cell receptor; these are defined herein as not being any primary MHC anchor.

Since the natural epitopes may not necessarily serve as optimal immunogens the composition may in particular comprise at least one amino acid sequence wherein an optimal primary anchor amino acid is present at either or both of the two primary anchor position of the amino sequences according to the invention. Accordingly, a preferred embodiment of the invention provides a composition according to the invention, wherein at least one, such as at least 2, at least 2, at least 4, at least 5, at least 6, at least 7, at least 8, at least 9, at least 10 or at least 11 of said amino acid sequences is selected from the group consisting of: GLADQLIHL (SEQ ID NO: 12), SLVKHHMYV (SEQ ID NO: 13), ALVEMGHHV (SEQ ID NO: 14), RLLNAWVKV (SEQ ID NO: 15), FLGKIWPS (SEQ ID NO: 16), KLGKAGYVV (SEQ ID NO: 17), NIWATHACV (SEQ ID NO: 18), VLAEAMSQV (SEQ ID NO: 19), NTVATLYCV (SEQ ID NO: 20), YIKIFIMIV (SEQ ID NO: 21), SLGQHIYET (SEQ ID NO: 22).

In equally preferred embodiments the composition according to the invention comprises the amino acid sequences: GLADQLIHL (SEQ ID NO: 12), SLVKHHMYV (SEQ ID NO: 13), ALVEMGHHV (SEQ ID NO: 14), RLLNAWVKV (SEQ ID NO: 15), FLGKIWPV (SEQ ID NO: 16), KLGKAGYVV (SEQ ID NO: 17), NIWATHACV (SEQ ID NO: 18).

Sequence identifiers 12 -14 all represent optimised immunogens. In a vaccine, it may be preferred to replace or supplement one or more of these optimised immunogens with one of its variants cf. table 1, preferably the variant occurring with the highest frequency. In yet a preferred embodiment, the composition according to the invention thus comprises the amino acid sequences: GLADQLIHL (SEQ ID NO: 12) and/or GLADQLIHM (SEQ ID NO: 23); SLVKHHMYV (SEQ ID NO: 13) and/or SLVKHHMYI (SEQ ID NO: 24); ALVEMGHHV (SEQ ID NO: 14) and/or ALVEMGHHA (SEQ ID NO: 25); RLLNAWVKV (SEQ ID NO: 15) and/or RTLNAWVKV (SEQ ID NO: 26); FLGKIWPV (SEQ ID NO: 16) and/or FLGKIWPS (SEQ ID NO: 27); KLGKAGYVV (SEQ ID NO: 17) and/or KLGKAGYVT (SEQ ID NO: 28); NIWATHACV (SEQ ID NO: 18) and/or NVWATHACV (SEQ ID NO: 29).

In a further preferred embodiment of the invention the composition comprises the amino acid sequences: GLADQLIHL (SEQ ID NO: 12), SLVKHHMYV (SEQ ID NO: 13), ALVEMGHHV (SEQ ID NO: 14), RLLNAWVKV (SEQ ID NO: 15), FLGKIWPS (SEQ ID NO: 27), KLGKAGYVV (SEQ ID NO: 17), NIWATHACV (SEQ ID NO: 18), VLAEAMSQV (SEQ ID NO: 19), NTVATLYCV (SEQ ID NO: 20), YIKIFIMIV (SEQ ID NO: 21), SLGQHIYET (SEQ ID NO: 22).

As an alternative to this embodiment the composition may comprise the amino acid sequences: GLADQLIHL (SEQ ID NO: 12), SLVKHHMYV (SEQ ID NO: 13), ALVEMGHHV (SEQ ID NO: 14), RLLNAWVKV (SEQ ID NO: 15), FLGKIWPV (SEQ ID NO: 16), KLGKAGYVV (SEQ ID NO: 17), NIWATHACV (SEQ ID NO: 18), VLAEAMSQV (SEQ ID NO: 19), NTVATLYCV (SEQ ID NO: 20), YIKIFIMIV (SEQ ID NO: 21), SLGQHIYET (SEQ ID NO: 22).

For the purpose of the present invention the amino acid sequences described above may each be comprised within a separate molecular entity. The term "molecular entity" is meant to comprise peptides, conjugates of two or more peptides and conjugates of one or more peptides and one or more non-peptide molecules. The term "peptide" denotes both short peptides with a length of at least eight amino acid residues and at most 10 amino acid residues, oligopeptides (11-100 amino acid residues), and longer peptides. The peptide may be chemically modified by being glycosylated, by being lipidated, or by comprising prosthetic groups.

Alternatively, the composition according to the invention may comprise a so-called polyepitope in the meaning that at least 2, such as at least 3, at least 4, at least 5, at least 6, at least 7, at least 8, at least 9, at least 10 or at least 11 of the amino acids sequences described above are included in the same molecular entity.

In some embodiments the composition of the invention, all amino acid sequences are present within the same molecular entity. Accordingly, if the composition comprises 11 amino acid sequences as described above, it may be preferred that 11 amino acid sequences is included in the same molecular entity.

It is apparent that the length and size of the molecular entity will depend on the number of amino acid sequences according to the invention that are contained within the said molecular entity. According to some embodiments of the invention, therefore, the said molecular entity comprises at least 8 amino acid residues, such as at least 9, at least 10, at least 11, at lest 12, at least 13, at least 14, at least 1, at least 20, at least 30, at least 40, at least 50, at least 60, at least 70, at least 80, at least 90, at least 100, at least 125, at least 150, at least 175 or at least 200 amino acid residues. In general, it will be desirable to keep the length of the molecular entities as short as possible. Thus, it may be preferred that the molecular entity has a length of at the most 9 amino acid residues, such as at the most 10, at the most 11, at the most 12, at the most 13, at the most 14, at the most 15, at the most 16, at the most 17, at the most 18, at the most 19, at the most 20, at the most 25, at the most 30, at the most 40, at the most 50, at the most 60, at the most 70, at the most 80, at the most 90, at the most 100, at the most 125, at the most 150, at the most 175 or such as at the most 200 amino acid residues.

If the molecular entity comprises only a single epitope or amino acid sequence according to the invention, the preferred length of the molecular entity may be 9, 10, 11, 12, 13, 14, 15, 16, 17, 18, 19 or 20 amino acids residues. Conversely, if the molecular entity comprises 11 epitopes, the preferred length may be 102, 105, 110, 115, 120, 125, 130, 135, 140, 145, 150, 160, 170, 180, 190 or 200 amino acid residues.

In a currently preferred embodiment of the invention the molecular entity is a polypeptide comprising from 100 to 150 amino acids, such as from 105 to 145, from 110 to 140, from 115-135 or such as from 120-130 amino acids residues. According to this embodiment of the invention the molecular entity comprises, preferably, 11 amino acid sequences as described above.

If the amino acid sequence as described above are combined into a polyepitope peptide that contains four or more of the amino acid sequences, i.e. a molecular entity, it is an advantage that the polyepitope is constructed in such a way that new epitopes do not arise in the region overlapping two adjacent epitope amino acid sequences. This is assured by testing the polyepitope amino acid sequence composition for the appearance of new putative HLA-A2 binding epitope sequences. Spacing amino acid sequences may be used to separate adjacent epitope amino acids but may preferably not exceed 6 amino acid residues. Accordingly, in a specific embodiment of the invention the composition comprises a molecular entity wherein said amino acids sequences is present a position and a mutual distance allowing for processing of the amino acid sequences without creating additional unwanted epitopes. Methods for generating polyepitopes and constructs encoding such polyepitopes are described in WO 0147641, which is incorporated in its entirety herein by reference.

The composition according to the invention may be combined with other relevant vaccine components. These additional components may be other vaccines with a capability of stimulating other parts of the immune system, including a humoral (antibody) response and/or CD4+ T cell activation, which may also act as an adjuvant for the amino acid sequences according to the invention. Thus, in addition to the amino acid sequences described above the composition may further comprise a T-helper epitope. As the skilled person will be aware, the ability of a component to induce a CD4+ T cell response may be determined by standard proliferation assays or cytokine production as determined by Flow cytometry or ELISA.

In particular embodiments of the invention the T-helper epitope is selected from the group consisting of: TPPAYRPPNAPIL (SEQ ID NO: 58) (synthetic T-helper peptide from hepatitis B virus), KRWIILGLNKIVRMY (SEQ ID NO: 59) (synthetic T-helper peptide from hiv-1 Gag 298), VWGIKQLQARVLAVERYLKD (SEQ ID NO: 60) (synthetic T-helper peptide from hiv-1, gp41), aK(X)VAAWTLKAAa (SEQ ID NO: 61), X=cyclohexylalanine a=D-alanine (PADRE), AKXVAAWTLKAAaZC X=L-cyclohexylalanine, Z=aminocaproic acid.

As mentioned above the amino acid sequences of the present invention has been selected on the basis of whether or not they fulfilled a number of criteria. In further preferred embodiments of the invention it is therefore a feature of the composition that at least one of said amino acid sequences as present in the composition, is:
a) capable of eliciting a CTL response in HLA-A2 transgenic mice e.g. C57-A2K^{b}tg mice as determined by > 10% lysis at Effector:Target (E:T) cell ratio 50:1 in a chromium release assay; and/or
b) capable of causing formation of > 10 spot forming units (s.f.u.) per 10⁶ splenocytes from HLA-A2 transgenic mice, e.g. C57-A2K^{b}tg mice, when assayed in IFN-γ ELISPOT assays; and/or
c) recognised by a CD8+ T cell recall response in at least one HIV positive HLA-A2+ and/or HLA-A2- patient in a group of 17 patients as determined in an intracellular-IFNγ FACS assay.

It is to be understood that all the amino acid sequences in said composition can be demonstrated to have at least one of the functional effects a) through c) as defined above prior to being combined in the composition. However, as present in the composition some of the amino acid sequences may not have any of these effects due for instance immune competition between the epitopes.

Whether an amino acid sequence according to the invention conforms to the criteria recited in a) may be determined in an assay conducted as in an assay as described by Corbet et al. 2003. Briefly, C57-A2K^{b}tg mice, 6-12 weeks old, are injected subcutaneously with 100 µg peptide plus 120 µg of an I-A-b binding synthetic T-helper peptide from hepatitis B virus, TPPAYRPPNAPIL (SEQ ID NO: 58), emulsified in 100 µl Freund's incomplete adjuvants. At day 10 post-infection, spleen cells from primed mice are re-stimulated using peptide-loaded mitomycin C-treated C57-A2K^{b}tg B-cell blasts. After 5 days of re-stimulation, effector cells are washed and resuspended to 5 × 10⁶ cells/ml and a standard 5h 51Cr-release assay is performed assaying the effector cells against either peptide-pulsed or unpulsed HLA-A2*0201/hβ2m-transfected EL4 target cells as described by Pascolo et al. 1997.

For evaluation of the amino acid sequences in relation to the criteria in b), ELISPOT assays may be conducted as described by Corbet et al. 2003. Spleen cells are plated on nitrocellulose microtiterplates that have been coated overnight (O.N.) with 50 µl per plate of Anti-IFM-γ monoclonal antibody (18181D Pharmingen) at 0.5 µg/ml in 0.1 M sodium bicarbonate buffer, pH 9.6. Peptides are added to a final concentration of 10 µg/ml in complete RPMI-1640 medium supplemented with 5 IU recombinant IL-2 per ml. After incubation O.N. the number if specific INF-γ-secreting T-lymphocytes are counted by direct visualisation.

Recognition by a CD8+ T cell recall response according to c) may be determined as described in Corbet et al. 2003. In brief, T-lymphocytes to the amino acid sequences are measured by flow cytometry. Cells are stimulated with 2 µg peptide as described by Kern et al. 1998, except that 400 µl of whole heparinized blood is used. Blood is treated with red blood cell-lysing solution and lymphocyte permeabilization solution according to the manufacturer's instructions. Cells are stained with appropriate antibodies for 30 minutes and fixed in 2% paraformaldehyde solution. Five-parameter flowcytometric analysis are performed using a FASCAN (Beckton Dickinson) using 150,000-400,000 events per analysis. CD3+CD8+ cells are examined for IC-FCN-γ and data are analysed using CELLQUEST software.

The C57-A2K^{b}tg mice which are a suitable model system for the above analysis are described by Epstein et al. 1989.

It is known within the field of HIV research that each individual will react only to a few of the potentially immunogenic target epitopes that the individual is exposed to. Thus, chronically infected HIV-1 patients on antiviral therapy have been reported to react to an average of only 4 epitopes, and in some single patients a single epitope may even account for 90% of the response (Goulder *et al.* 2001).

As mentioned above, it has been demonstrated in relation to the present invention that a composition comprising only a few peptide epitopes may provide adequately broad coverage. Therefore, it may be preferred that the composition according to the invention does not comprise any amino acid sequence (e.g. of at least 4 amino acids, such as of at least 5 amino acids or of at least 6 amino acids or of at least 7 amino acids or of at least 8 amino acids or of at least 9 amino acids or of at least 10 amino acids) from a protein selected from the group consisting of structural or accessory proteins of a human immunodeficiency virus other than an amino acid sequence as defined above.

In view hereof and for practical reasons, it may be desirable to include as few amino acid sequences in the composition according to the invention. Thus, in preferred embodiments the composition comprises at the most 4 amino acid sequences, such as at the most 5, at the most 6, at the most 7, at the most 8, at the most 9, at the most 10 or such as at the most 11 amino acid sequences selected from the group consisting of structural (e.g. the Gag, Pol and Env proteins) or accessory proteins (e.g. the Vif, Vpu and Vpr proteins) of a human immunodeficiency virus.

Also some preferred embodiments pertain to a composition according to the invention, wherein said composition does not comprise any amino acid sequence (e.g. of at least 4 amino acids, such as of at least 5 amino acids or of at least 6 amino acids or of at least 7 amino acids or of at least 8 amino acids or of at least 9 amino acids or of at least 10 amino acids) or molecular entity derived from a human immunodeficiency virus other than an amino acid sequence or molecular entity as defined above.

Further preferred embodiments of the invention provide a composition which does not comprise any synthetic or recombinantly produced amino acid sequences as defined above and/or any synthetic or recombinantly produced molecular entity other than a molecular entity as defined above.

The term "recombinantly produced" refers to an artificial combination usually accomplished by either chemical synthesis means, recursive sequence recombination of nucleic acid segments or other diversity generation methods (such as, e.g. shuffling) of nucleotides, or manipulation of isolated segments of nucleic acids, e.g. by genetic engineering techniques known to those of ordinary skill in the art. "Recombinantly expressed" typically refers to techniques for the production of a recombinant nucleic acid in vitro and transfer of the recombinant nucleic acid into cells in vivo, in vitro, or ex vivo, where it may be expressed or propagated.

In the present context, the term "synthetic peptide" refers to a peptide, including a short peptide that has been synthesized in vitro. The term further encompasses peptides or short peptides that have been modified by substitution with unusual or non-natural amino acids.

Preparation of vaccines which contain peptide sequences as active ingredients is generally well understood in the art, as exemplified by U.S. Patents 4,608,251; 4,601,903; 4,599,231 and 4,599,230. Typically, such vaccines are prepared as injectables either as liquid solutions or suspensions; or solid forms suitable for solution in, or suspension in, liquid prior to injection may also be prepared. The preparation may also be emulsified.

The manner of application may be varied widely. Any of the conventional methods for administration of a vaccine are applicable. These are believed to include oral application on a solid physiologically acceptable base or in a physiologically acceptable dispersion, parenterally, by injection or the like. The dosage of the vaccine will depend on the route of administration and will vary according to the age of the person to be vaccinated and, to a lesser degree, the size of the person to be vaccinated.

The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired. Suitable dosage ranges are of the order of more than 100 microgram/g active ingredient per vaccination. In particular embodiments the invention thus provides a composition, wherein each of said amino acid sequences are present in an amount of from 20-400 µg/dose, such as from 50-300 µg/dose, such as from 75-250 µg/dose, such as from 80-200 µg/dose, such as from 90-150 µg/dose, such as from 95-125 µg/dose. Currently preferred is a dose having a content of, 100 µg/dose of an amino acid sequence as defined above. It is to be understood that a dose may constitute a volume of from 50 µl-1 ml, such as from 100-750 µl, such as from 150-600 µl, such as from 200-500 µl, such as from 250-400 µl, or such as from 50-100 µl, such as from 100-200 µl, such as from 200-300 µl, such as from 300-400 µl, such as from 400-500 µl, such as from 500-600 µl, or such as from 700-800 µl.

In many instances, it may an advantage to have multiple administrations of the vaccine.
Suitable regimens for initial administration and booster shots are also variable but are generally typified by an initial administration followed by subsequent inoculations or other administrations. Usually, the regiment will not be exceeding six vaccinations, more usually not exceeding four vaccinations and preferably one or more, usually at least about three vaccinations. The vaccinations may normally be at from two to twelve week intervals, more usually from three to five week intervals. Periodic boosters at intervals of 1-5 years, usually three years, may be desirable.

Some of the polypeptides of the vaccine are sufficiently immunogenic by themselves, but for some of the others the immune response will be enhanced if the vaccine further comprises an adjuvant substance. Therefore, some aspects of the invention provide a composition which further comprises an adjuvant substance.

Various methods of achieving adjuvant effect for the vaccine include use of agents such as aluminum hydroxide or phosphate (alum), commonly used as 0.05 to 0.1 percent solution in phosphate buffered saline, admixture with synthetic polymers of sugars (Carbopol) used as a 0.25 percent solution, aggregation of the protein in the vaccine by heat treatment with temperatures ranging between 70°C to 101°C for 30 second to 2 minute periods respectively, Freund's complete or incomplete adjuvants, QuilA and Quil A derivates, RIBI, Carbopol, physiologically acceptable oil vehicles including mannide mono-oleate (Aacel A), perfluorocarbon (Fluosol-DA), dimethyldioctadecylammonium bromide (DDA), endotoxins or lipopolysaccharide or Lipid A components of Gram-negative bacteria. Further possibilities are monophosphoryl lipid A (MPL), and muramyl dipeptide (MDP).

Currently preferred adjuvants are mycobacterial derivatives including the bacille Calmette-Guerin BCG developed as a tuberculosis vaccine in the 19^{th} century, and the Ag85 and ESAT-6 antigens derived from mycobacterium tuberculosis. Another particularly preferred adjuvant inducing strong Th1 responses is the LipoVac system based on stable cationic liposomes comprising dimethyldioctadecylammonium-bromide and a total lipid extract of a mycobacterium. Details on the LipoVac adjuvant are provided in WO 2005/004911.

Further preferred adjuvants are immune stimulatory CpG oligonucleotides containing certain CpG motifs. The term "CpG motif" refers to a nucleic acid having a cytosine followed by a guanine linked by a phosphate bond. Preferably, the CpG motif is unmethylated, meaning that methylation on the cytosine pyrimidine ring is absent. Methylation, partial removal, or removal of an unmethylated CpG motif in an oligonucleotide included as adjuvant is believed to reduce its effect.

Finally, other examples of suitable adjuvants are cytokines including but not limited to IL-15, GM-CSF and IL-2

In addition to possibly being mixed with one or more adjuvants, the active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents and pH buffering agents.

Recently a therapeutic dendritic-cell based HIV vaccine was contemplated by Lu and co-workers (Lu *et al.* 2004). The rationale of Lu *et al.,* however, is different from the concept of the present invention in that the Lu *et al.* vaccine was based on the use of autologous inactivated HIV virus. In a currently most preferred embodiment of the invention the composition further comprises monocyte-derived dendritic cells.

According to this embodiment of the invention the monocyte-derived dendritic cells may be present in an amount of from 10⁶ to 10⁹ cells per ml, such as from 10⁷-10⁹, or such as from 5 x 10⁷ to 5 x 10⁸ cells per ml.

The composition according to the invention may be administered parenteral, by injection, for example, either subcutaneously or intramuscularly. Additionally, examples of formulations which are suitable for other modes of administration include suppositories and oral formulations. For suppositories, traditional binders and carriers may be included, for example, polyalkalene glycols or triglycerides. Oral formulations generally include normally employed excipients as, for example, pharmaceutical grades of mannitol, lactose, starch, magnesium stearate, sodium saccharine, cellulose, magnesium carbonate, and the like. These compositions may take the form of solutions, suspensions, tablets, pills, capsules, sustained release formulations or powders.

The amino acid sequences of the present invention may be formulated into the composition as neutral forms or salt forms. Pharmaceutically acceptable salts include acid addition salts (formed with the free amino groups of the peptide) and which are formed with inorganic acids such as, for example, hydrochloric or phosphoric acids, or such organic acids as acetic oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

### Nucleic acid sequences

The present invention also relates to a composition comprising at the most 20 and at the least 7, such as at least 8, such as at least 9, such as at least 10 such as at least 11 nucleic acid sequences encoding amino acid sequences selected from the group consisting of:
X₁-X₂-ADQLIH-X₃ (SEQ ID NO: 1)
   wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
   wherein X₁ is selected from the group consisting of: V, I, T and R;
X₁-X₂-VEMGHH-X₃ (SEQ ID NO: 3)
   wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
   wherein X₁ is selected from the group consisting of: T and L;
FLG-X₁-IW-X₂-X₃ (SEQ ID NO: 5)
   wherein X₁ is selected from the group consisting of K and R; X₂ is selected from the group consisting of: P and S; and X₃ is selected from the group consisting of: V and S;
K-X₁-GKAGYV-X₂ (SEQ ID NO: 6)
   wherein X₁ is selected from the group consisting of: L, M, I and T; and X₂ is selected from the group consisting of: T and V;
N- X₁-WATHACV (SEQ ID NO: 7)
   wherein X₁ is selected from the group consisting of: V and I;
VLAEAM5Q-X₁ (SEQ ID NO: 8)
   wherein X₁ is selected from the group consisting of: V, M and A;
N-X₁-VATLYCV (SEQ ID NO: 9)
   wherein X₁ is selected from the group consisting of: T and L;
Y-X₁-X₂-IFIMI- X₃ (SEQ ID NO: 10)
   wherein X₁ is selected from the group consisting of: I and L; X₂ is selected from the group consisting of: K and R; and X₃ is selected from the group consisting of: V and I;
X₁-LGQHIY- X₂-T (SEQ ID NO: 11)
   wherein X₁ is selected from the group consisting of: S, G and N; and X₂ is selected from the group consisting of: E and N, wherein the composition comprises the 7 nucleic acid sequences encoding amino acid sequences SEQ ID NOs 1 to 7.

A further aspect of the invention provides a nucleic acid molecule comprising a sequence of nucleic acids encoding at least 7, such as at least 8, such as at least 9, such as at least 10 such as at least 11 of the amino acid sequences as defined above.

When the term nucleotide is used in the present application, it should be understood in the broadest sense. A "nucleotide" thus refers to the phosphate ester polymeric form of ribonucleosides ("RNA molecules") or deoxyribonucleosides ("DNA molecules"), or any phosphoester analogs thereof, such as phosphorothioates and thioesters, in either single stranded form, or a double-stranded helix. The term "nucleic acid" includes double-stranded DNA, Inter alia, in linear (e.g., restriction fragments) or circular DNA molecules. Furthermore, the term nucleotide should also be considered as synthetic DNA or RNA analogues such as PNA and LNA.

Apart from their use as starting points for the synthesis of polypeptides of the invention and for hybridization probes (useful for direct hybridization assays or as primers in e.g. PCR or other molecular amplification methods), the nucleic acid fragments of the invention may be used for effecting *in vivo* expression of antigens, *i.e*. the nucleic acid fragments may be used in so-called DNA vaccines. Recent research have revealed that a DNA fragment cloned in a vector which is non-replicative in eukaryotic cells may be introduced into an animal (including a human being) by e.g. intramuscular injection or percutaneous administration (the so-called "gene gun" approach). The DNA is taken up by e.g. muscle cells and professional antigen presenting dendritic cells and the gene of interest is expressed by a promoter which is functioning in eukaryotes, e.g. a viral promoter, and the gene product thereafter stimulates the immune system. These newly discovered DNA vaccination methods are reviewed in Ulmer et al., 1993, which is incorporated herein by reference.

Other delivery methods for the DNA vaccine encoding the peptides may be used, e.g. recombinant Adenovirus, adenoassociated virus, alphavirus, pox virus such as vacciniavirus or modified vaccinia virus Ankara (MVA), recombinant influenzavirus or bacteria such as *lactococcis lactis, lactobacillus* species, *salmonella* and *shigella* species. The DNA vaccine can also be delivered inside apoptotic cells. The DNA vaccine encoding the vaccinepeptides can also be delivered ex vivo to autologous antigen presenting dendritic cells by transfection, electroporation or coupled to nanoparticles that are injected into an individual.

In a preferred embodiment of this aspect of the invention the nucleic acid molecule comprises a sequence of nucleic acids encoding the amino acid sequences:
X₁-X₂-ADQLIH-X₃ (SEQ ID NO: 1)
   wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
   wherein X₁ is selected from the group consisting of: V, I, T and R;
X₁-X₂-VEMGHH-X₃ (SEQ ID NO: 3)
   wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
   wherein X₁ is selected from the group consisting of: T and L;
FLG-X₁-IW-X₂-X₃ (SEQ ID NO: 5)
   wherein X₁ is selected from the group consisting of K and R; X₂ is selected from the group consisting of: P and S, and X₃ is selected from the group consisting of: V and S;
K-X₁-GKAGYV-X₂ (SEQ ID NO: 6)
   wherein X₁ is selected from the group consisting of: L, M, I and T; and X₂ is selected from the group consisting of: T and V; and
N-X₁-WATHACV (SEQ ID NO: 7)
   wherein X₁ is selected from the group consisting of: V and I.

It will be apparent the sequence of said nucleic acid molecule may be derived by simple back-translation of the amino acid sequences that the nucleic acid molecule is to encode. The sequence may in the simplest embodiments be construed from the list of possible codons representing the relevant amino acid residues.

| **Amino acid** | **Possible codons** |
|---|---|
| Alanine | GCA,GCC,GCG,GCT |
| Asparagine or Aspartic acid | AAC, AAT, GAC, GAT |
| Cysteine | TGC,TGT |
| Aspartic acid | GAC,GAT |
| Glutamic acid | GAA,GAG |
| Phenylalanine | TTC, TTT |
| Glycine | GGA,GGC,GGG,GGT |
| Histidine | CAC,CAT |
| Isoleucine | ATA,ATC,ATT |
| Lysine | AAA,AAG |
| Leucine | CTA, CTC, CTG, CTT, TTA, TTG |
| Methionine | ATG |
| Asparagine | AAC,AAT |
| Proline | CCA,CCC,CCG,CCT |
| Glutamine | CAA,CAG |
| Arginine | AGA, AGG, CGA, CGC, CGG, CGT |
| Serine | AGC, AGT, TCA, TCC, TCG, TCT |
| Threonine | ACA, ACC, ACG, ACT |
| Valine | GTA, GTC, GTG, GTT |
| Tryptophan | TGG |
| stop codon | TAA, TAG, TGA |
| Tyrosine | TAC, TAT |
| Glutamine or Glutamic acid | CAA,CAG,GAA,GAG |

The frequencies with which different codons are used vary significantly between different organisms, between proteins expressed at high or low levels within the same organism, and sometimes even within the same operon. Codon usage is a critical factor in prokaryotic gene expression, which without being bound by any theory is believed to be because preferred codons correlate with the abundance of cognate tRNAs available within the cell. This relationship serves to optimize the translational system and to balance codon concentration with isoacceptor tRNA concentration. To reduce heterologous expression problems it is therefore preferred to eliminate codons that are used infrequently by the desired host organism. Any codon that is used less than 5% to 10% of the time by an organism may cause problems. It may be particularly detrimental to expression if two or more of such infrequently used codons are close or adjacent to each other. It is generally preferred to incorporate codons at approximately the same frequency as what is found in the host's natural genes. This ensures that the tRNA pools remain balanced, even for a highly expressed protein.

One very successful application of codon optimization has been in enhancing the expression of viral genes in mammalian cell lines. Virus codon choices are often highly constrained because their dense information load is frequently accommodated using overlapping reading frames. For DNA vaccine research and development or other areas where high expression of individual viral proteins is important, gene resynthesis using the host's codon bias has resulted in very large increases in expression.

The nucleic acid molecule according to the invention is preferably obtained by back-translation of the amino acid sequence using codons that are preferentially used by highly-expressed human gene. Small characters in the sequence indicate silent mutations we made either to add selected endonuclease restriction sites or to remove nucleotides repeats that might have generated unwanted secondary structure and/or interfered with de NOVO construction by overlapping PCR. The modifications in the gene sequence of this polytope are made by choosing the human codons with the second highest frequency in available codon usage table such as that disclosed at www.kasuka.or.jp or in Vinner et al 1999.

It should be understood that for the purpose of the present invention the nucleic acid molecule may be in the form of a single stranded nucleic acid or in the form of a plasmid, such as a naked plasmid, a single stranded plasmid, a double stranded plasmid or a virus. The nucleic acid molecule may be designed with the purpose of mediating transcription of the sequence of nucleic acids in vivo and may thus be regarded as a DNA vaccine or part of a DNA vaccine. In the present context, a "naked" plasmid means that it is free from any delivery vehicle that can act to facilitate entry into the cell.

Numerous strategies for development of constructs useful in delivering the nucleic acid sequences of the invention have been described in the art. Suitable vector constructs for delivery of DNA vaccines are described, for instance in US2006051839.

In embodiments of this aspect of the invention where the nucleic acid sequence encodes several of the amino acid sequences as described above the nucleic acid sequence may in particular be prepared by using overlapping long synthetic oligonucleotides in PCR. The PCR product may be purified and cloned using a conventional vector for cloning and expression.

In view hereof, a further preferred embodiment of this aspect of the invention provides a nucleic acid molecule which comprises one or more of the following nucleic acid sequences:
GGC CTG GCC GAC CAG CTG ATC CAC CTG (SEQ ID NO: 63) (encoding Vif101(9L) amino acids GLADQLIHL; i.e. SEQ ID NO: 12);
AGC CTG GTG AAG CAC CAC ATG TAC GTG (SEQ ID NO: 64) (encoding Vif23 amino acids SLVKHHMYV; i.e. SEQ ID NO: 13);
GCA CTA GTG GAG ATG GGA CAT CAC GTG (SEQ ID NO: 65) (encoding Vpu66 amino acids ALVEMGHHV; i.e. SEQ ID NO: 14);
CGC CTG CTG AAC GCC TGG GTG AAA GTG (SEQ ID NO: 66) (encoding Gag150(2L) amino acids RLLNAWVKV; i.e. SEQ ID NO: 15);
TTC CTG GGC AAG ATC TGG CCA TCT (SEQ ID NO: 67) (encoding Gag433(8S) amino acids FLGKIWPS; i.e. SEQ ID NO: 16);
AAG CTT GGC AAG GCC GGC TAC GTG GTG (SEQ ID NO: 68) (encoding Pol606 amino acids KLGKAGYVV; i.e. SEQ ID NO: 17);
AAC ATC TGG GCA ACA CAC GCC TGC GTC (SEQ ID NO: 69) (encoding Env67(2i) amino acids NIWATHACV; i.e. SEQ ID NO: 18);
GTG CTG GCC GAA GCT ATG AGC CAG GTC (SEQ ID NO: 70) (encoding Gag362 amino acids VLAEAMSQV; i.e. SEQ ID NO: 19);
AAC ACC GTG GCT ACT CTG TAC TGT GTC (SEQ ID NO: 71) (encoding Gag80 amino acids NTVATLYCV; i.e. SEQ ID NO: 20);
TAC ATC AAG ATT TTC ATC ATG ATC GTG (SEQ ID NO: 72) (encoding Env681 amino acids YIKIFIMIV; i.e. SEQ ID NO: 21);
AGC TTG GGA CAA CAT ATC TAC GAG ACC (SEQ ID NO: 73) (encoding Vpr41 amino acids SLGQHIYET; i.e. SEQ ID NO: 22)

In relation to these embodiments it may be preferred that the nucleic acid molecule comprises at least 2, such as at least 3, at least 4, at least 5, at least 6, at least 7, at least 8, at least 9, at least 10 or at least 11 of these nucleic acid sequences.

In an additional embodiment the nucleic acid molecule comprises a sequence of nucleic acids encoding a molecular entity as defined above. Where the molecular entity comprises several of the amino acid sequences as defined above said molecular entity may be referred to as a polyepitope. As mentioned above, approaches to generating constructs encoding functional polyepitopes are provided in WO 0147541.

According to a third main aspect of the invention is provided a composition comprising a nucleic acid molecule as described above. The composition may in particular be regarded as a DNA vaccine. DNA vaccines are generally injected into host tissues in the form of plasmid DNA or RNA molecules via needle or particle bombardment. Once delivered, the DNA induces expression of antigenic proteins within transfected cells. U.S. Patent No. 6,194,389 describes methods for transferring DNA to vertebrate cells to produce physiological immune-response producing protein in an animal subject.

In the present context the term "DNA vaccine" and variations thereof refer to a nucleotide sequence that encodes an antigenic peptide, such as an amino acid sequence according to the invention, and may be directly introduced into a subject to induce an immune response in the subject against the antigenic peptide.

The amount of DNA to be used in a vaccine recipient depends, e.g., on the strength of the promoter used in the DNA construct, the immunogenicity of the expressed tumor antigen, the condition of the mammal intended for administration (e.g., weight or age), the mode of administration, and the type of formulation. In general, a therapeutically effective dose from about 1 µg to about 1 mg, preferably, from about 10 µg to about 800 µg and, more preferably, from about 25 µg to about 250 µg, can be administered to human adults. The administration can be achieved in a single dose or repeated at intervals.

The route of administration may be any conventional route used in the vaccine field. As general guidance, a nucleic acid of the invention may be administered via a parenteral route, e.g., by an intradermal, intraepidermal, or intramuscular route. The choice of administration route depends on the formulation that is selected. For example a polynucleotide formulated in association with bupivacaine is advantageously administered into muscles. When a neutral or anionic liposome or a cationic lipid, such as DOTMA or DC-Chol, is used, the formulation can be advantageously injected via intramuscular or intradermal routes. A polynucleotide in a naked form can advantageously be administered via the intramuscular, intradermal, or subcutaneous routes.

### Use of a composition of the present invention

Another main aspect of the invention pertains to a composition comprising the amino acid sequences and/or molecular entities as described above for use in medicine. This aspect of the invention also provides a composition comprising the nucleic acid sequences or a nucleic acid molecule as described above for use in medicine. It will be apparent that all embodiments or features described above in relation to the amino acid sequences of the invention and all the embodiments and features relating to the nucleic acid sequences or molecules and the composition thereof also apply to this aspect of the invention.

In a related aspect the present invention also relates to the use of a composition comprising the amino acid sequences and/or molecular entities as described above in the manufacture of a medicament for the prevention and/or treatment of human immunodeficiency virus infections/acquired immunodeficiency syndrome. This aspect of the invention also provides the use of a composition comprising the nucleic acid sequences or a nucleic acid molecule as described above in the manufacture of a medicament for the prevention and/or treatment of human immunodeficiency virus infections/acquired immunodeficiency syndrome. Also in relation to this aspect it is to be understood that all embodiments or features described above in relation to the amino acid sequences of the invention and all the embodiments and features relating to the nucleic acid sequences or molecules and the composition thereof also apply to this aspect of the invention.

Whereas the primary goal when selecting and characterising the amino acid sequences according to the invention was to develop compositions that would be useful in relation to subjects having the HLA-A2 tissue type it has been observed that the amino acids sequences and the nucleic acid sequences according to the invention may also be effective when administered to individuals having a different tissue type. Therefore, according to an interesting and currently promising embodiment of the invention the medicament is for administration to an individual having the HLA-A2 tissue type and/or to an individual a non-HLA-A2 tissue type. In particular the non-HLA-A2 tissue type may be selected from the group consisting of: HLA-A1, HLA-A3, HLA-A11, HLA-A24, HLA-B7, HLA-B27, HLA-B44, HLA-B58 AND HLA-B62.

In a currently most preferred embodiment of the invention, however, said medicament is for administration to an individual having the HLA-A2 tissue type.

Based on phylogenetic studies, HIV-1 viruses are classified into 3 genetic groups; group M for Major, group O for Outer and group N for New. While HIV-1 group M viruses are responsible for the majority of HIV-infections world-wide, HIV-1 group O and N have mainly been identified in restricted areas in Central Africa (mostly Cameroon). HIV-1 group M is currently subdivided into 8 reference genetic subtypes: A, B, C, D, F, G, H, J (available at http://hiv-web.lanl.gov/).

The amino acid sequences and nucleic acid sequences according to the invention have been developed with the primary goal of providing compositions that were useful in individuals infected with a subtype B human immunodeficiency virus. However, the minimal epitopes identified have proved to be surprisingly well conserved cross-clade. According to one interesting embodiment, therefore, the medicament is for administration to an individual being infected with a human immunodeficiency virus-1 of group M, selected from the group of subtypes consisting of: subtype A, subtype B, subtype C, subtype D, subtype E, subtype F, subtype G, subtype H, subtype J, subtype K and recombinants of these. Also, the medicament may be for administration to an individual being infected with a human immunodeficiency virus-1 belonging to any one of groups N and O.

It has previously been established that the HIV virus has the ability to recombine and therefore one will expect a number of recombinants of the known subtypes to appear in the future. A list of currently existing circulating forms (CRFs) is found at the Los Alamos National Laboratory website which is operated by the University of California for the US department of Energy, http://hiv.lanl.gov/content/hiv-db/CRFs/CRFs.html. At present the list describes the following CRFs:

| **Name** | **Reference strain** | **Subtypes** |
|---|---|---|
| CRF01 AE | CM240 | A, E |
| CRF02 AG | IbNG | A, G |
| CRF03 AB | Kal153 | A, B |
| CRF04 cpx | 94CY032 | A, G, H, K, U |
| CRF05 DF | VI1310 | D, F |
| CRF06 cpx | BFP90 | A, G, J, K |
| CRF07 BC | CN54 | B', C |
| CRF08 BC | GX-6F | B', C |
| CRF09 cpx | 96GH2911 | CRF02_AG, A, U |
| CRF10 CD | TZBF061 | C, D |
| CRF11 cpx | GR17 | A, CRF01_AE, G, J |
| CRF12 BF | ARMA159 | B, F |
| CRF13 cpx | 96CM-1849 | A, CRF01_AE, G, J, U |
| CRF14 BG | X397 | B, G |
| CRF15 01B | 99TH.MU2079 | CRF01_AE, B |
| CRF16 A2D | KISII5009 | A2, D |

The list is maintained and is regularly updated to include newly identified CRFs. A more detailed description of the CRFs and their sequences is found at the website and in the HIV Sequence Compendium published annually by the Los Alamos National Laboratory, See for instance Kruiken C et al. HIV sequence Compendium. Theoretical Biology and Biophysics Group, Los Alamos National Laboratory, Los Alamos, New Mexico, 2002.

It is within the scope of the present invention to provide medicaments, wherein each medicament is composed for a specific group of HIV infected individuals. Therefore, according to one embodiment of the invention the medicament is for administration to an individual infected with a human immunodeficiency virus, the proviral DNA or viral RNA of said virus comprising at least one such as at least 2, at least 3, at least 4, at least 5, at least 6, at least 7, at least 8, at least 9, at least 10, or at least 11 nucleic acid sequence encoding an amino acid sequence as defined above or an amino acid sequence variant shown to cross-react with said amino acid sequence.

For the purpose of determining cross-reactivity, experiments may be conducted using for instance HLA-A2 tg HHD mice (described by Pascolo *et al.,* 1997), essentially as described in the examples section. HHD mice are H-2D^{b-/-} and β2m^{-/-} double knockout mice. In brief, isolated bone marrow cells from HHD transgenic mice are kept overnight in culture medium (RPMI 1640 with 10 % FCS, penicillin, streptomycin, 2-mercapta-ethanol). The next day, the non-adhering cells are isolated and cultured with GM-CSF, 10 ng/ml, and IL-4, 20 ng/ml. At day 3, fresh culture medium with GM-CSF and IL-4 is supplied. At day 6, the cells are harvested and cultured overnight with GM-CSF, IL-4 and LPS, 1 µg/ml. At day 7, mature dendritic cells are harvested and used for vaccination. HHD transgenic mice, 8-12 weeks old, are injected s.c. with syngenic mature dendritic cells pulsed with the test peptide and the T-helper peptide. At day 11 after vaccination, the mice are sacrificed and the primed splenocytes are pooled and re-stimulated with the vaccination-peptide. Briefly, the splenocytes (100 × 10⁶/ml) are loaded with the vaccination-peptide, 100 µg/ml, for 3 hours at 37 °C and at day 1 after re-stimulation, IL-2, 100 IU/ml, is added. After 5 days of re-stimulation, the CTLs are harvested and used for the Cr-release assay, or kept another 6 days in culture media with IL-2 and tested in the ELISPOT assay.

As explained above the amino acid sequences of the present invention correspond to selected subdominant epitopes of proteins from the HIV-1 virus. Thus when a composition of the present invention comprising said amino acid sequences is used as a vaccine it may force the immune system of an individual to respond to epitopes that have not previously been recognised in the individual or to which only few individuals within a group normally react to. Therefore, in a preferred embodiment the medicament may be administered to an individual whose CD8+ T-cells react weakly or do not react with one or more of the amino acid sequences of the present invention, i.e. those described in any of claims 1-12 when said amino acid sequences are present in a HIV protein of the virus by which the patient is infected. The reaction of CD8+ T-cell may be determined by the previously described method c); i.e. the CD8+ T-cell recall response.

It may thus be preferred that the medicament is administered to an individual who does not have a or only has a weak CD8+ T-cell response to the naturally occurring variant of at least one of the amino acid sequences included in the composition or the medicament, such as at least 2, at least 3, at least 4, at least 5, at least 6, at least 7, at least 8, at least 9, at least 10 or at least 11 of the amino acid sequences included in the composition or the medicament. In this context the term "variant" refers to any of the amino acid sequences that may be represented by the generic sequences of SEQ ID NOs: 1-11. In particular, a naturally occurring variant may be the variant of the amino acid sequence which is found in the candidate individual.

According to these preferred embodiments the medicament may also be contemplated for administration to a group of patients wherein an amino acid sequence as defined above or an amino acid sequence variant shown to cross-react with said amino acid sequence is infrequently recognised. In the context of the present invention the term "infrequently recognised" means that the amino acid sequence is recognised in no more than 3 individuals in a group of 10, such as no more than 2 individuals, such as no more than 1 individual in a group of 10.

As a therapeutic the medicament may be contemplated as a supplement to conventional anti-retroviral therapy and in preferred embodiments the medicament is therefore for administration to an individual or a group of individuals receiving anti-retroviral therapy. Currently the anti-retroviral therapy comprises nucleoside RT inhibitors, non-nucleoside RT inhibitors, protease inhibitors, fusion inhibitors and integrase inhibitors.

Alternatively, the medicament may serve its purpose when administered to an individual or a group of individuals which do not receive anti-retroviral therapy. In particular, it may be contemplated that use of the medicament according to the invention may allow discontinuation of the anti-retroviral therapy.

### Method of producing a composition of the present invention

According to another aspect the invention provides a method of producing a composition such as a vaccine according to the invention. In a main embodiment the method comprises formulating at least 7, at least 8, at least 9, at least 10 or at least 11 of the amino acid sequences according to the invention or a nucleic acid sequence according to the invention with an appropriate component selected from the group consisting of: a carrier, an excipient, a binder, an adjuvant, and a pharmaceutically acceptable salt. Examples of such suitable components are recited above. As also explained above dendritic cell based vaccines provide a promising approach in the development of subunit vaccines against HIV and, accordingly the method for producing a composition according to the invention may comprise the step of pulsing dendritic cells with an amino acid sequence and/or a molecular entity and/or a composition as defined above. It may be contemplated that the dendritic cells may be pulsed with at least 2, such as at least 3, at least 4, at least 5, at least 6, at least 7, at least 8, at least 9, at least 10 or at least 11 amino acid sequences as provided by the invention. Methods for pulsing of dendritic cells are described in details in Lu et al. 2004 and in the examples illustrating the present invention.

Specific steps in a procedure for pulsing dendritic cells may be any of the steps of:
a) lysing any red blood cells present in a sample taken from an individual, said sample containing mononuclear cells;;
b) concentrating the monocytes present in said sample, e.g. by allowing said monocytes to adhere to a solid support;
c) culturing said monocytes under conditions allowing immature dendritic cells to differentiate from said monocytes;
d) culturing said immature dendritic cells under conditions allowing further maturation of the cells.

In step a) the sample may e.g. be a blood sample or a tissue sample.

In step c) examples of conditions allowing immature dendritic cells to differentiate from said monocytes include those provided in the examples; in particular, the dendritic cells are cultured in an appropriate medium, e.g. the X-VIVO15 medium (available from e.g. Cambrex) or the CellGro DC medium (CellGenix)). The medium is preferably supplemented with autologous plasma, preferably in a relative amount of approximately 1%, and recombinant cytokines, GM-CSF, preferably in an amount of 1000 U/ml (Leukine) and IL4, preferably in an amount of approximately 250 U/ml (Cellgro).

In step d) the dendritic cells may in particular be allowed to further matured, preferably in the X-VIVO15 medium or the CellGro DC medium supplemented with approximately 25 ng/ml IL-1β, approximately 50 ng/ml TNF-α, approximately 3000 U/ml IFN-α, approximately 1000 U/ml IFN-γ, 12,5 µg/ml Polyinosin: polycytidin.

In the procedure described above mononuclear cells are preferentially isolated from the sample by leukapheresis.

In a further preferred aspect of the disclosure the dendritic cells are of autologous origin, that is, the cells are derived from the individual to whom the composition is to be administered.

According to an aspect of the disclosure the immature dendritic cells are pulsed with a composition of the present invention or an amino acid sequence of the present invention. I relation to this embodiment it is preferred that the dentritic cells are pulsed at a point in the process after the cells have been allowed to mature. Referring to the process steps above, it may thus be preferred that the cells are pulsed at a point close to the completion of step e) or after completion of step e).

According to the present disclosure dendritic cells may be pulsed with a composition comprising at least 4 amino acid sequences according to the invention, such as at least 5, at least 6, at least 7, at least 8, at least 9, at least 10 or at least 11 amino acid sequences according to the invention. However, in order to avoid or minimize competition between the epitopes/amino acid sequences it may be preferred to generate aliquots of dendritic cells, to pulse each aliquot with a preparation/formulation of a single peptide species and to subsequently prepare a mix of said aliquots.

According to another main aspect of the disclosure is provided a method of treating an individual being infected with a human immunodeficiency virus, such as the HIV-1 or reducing the risk of infection with a human immunodeficiency virus, the method comprising administering to said individual a composition selected from the group consisting of:
i) a composition comprising an amino acid sequence corresponding to a CTL epitope from a virus isolate from said individual and/or a CTL epitope which is found in virus isolates from more than 50% of a group of infected individuals to which the individual belongs and/or an amino acid sequence shown to cross-react with said CTL epitope;
ii) a composition comprising a nucleic acid sequence coding for an amino acid sequence corresponding to a CTL epitope from a virus isolate from said individual and/or a CTL epitope which is found in virus isolates from more than 50% of a group of infected individuals and/or a nucleic acid sequence coding for an amino acid sequence shown to cross-react with one of said CTL epitope;

In the context of the present disclosure the group of infected individuals may be infected individuals within a certain geographic area or a certain social group.

Alternatively, the composition of i) may be one that comprises an amino acid sequence corresponding to a CTL epitope, and/or an amino acid sequence shown to cross-react with a CTL epitope from a virus isolate which is found in more than 50% of a group of infected individuals within the geographical area or within the social group to which the individual that is to receive the composition, belongs. Similarly, for the composition in ii) this composition may be one that comprises a nucleic acid sequence coding for an amino acid sequence corresponding to a CTL epitope, and/or an amino acid sequence shown to cross-react with a CTL epitope from a virus isolate which is found in more than 50% of a group of infected individuals within the geographical area or within the social group to which the individual that is to receive the composition, belongs.

In order to identify individuals to which it will be considered feasible to administrate the composition a step may be included of analysing proviral DNA or viral RNA from a virus isolate from said individual and/or from a virus isolate which is found in more than 50% of a group of infected individuals in order to identify nucleic acid sequences coding for HLA binding CTL epitopes. As a skilled person will understand conventional methods for nucleic acid sequencing may be employed in the analysis.of the individuals and their virus isolates. Illustrations of such sequencing technology are provided in the examples.

In preferred aspect of the disclosure method the method comprises administering to said individual a composition selected from the group consisting of:
i) a composition comprising at least 2, such as at least 3, at least 4, at least 5, at least 6, at least 7, at least 8, at least 9, at least 10 or at least 11 amino acid sequences, each amino acid sequence corresponding to or cross-reacting with a CTL epitope from a virus isolate from said individual and/or a CTL epitope which is found in virus isolates from more than 50% of a group of infected individuals to which the individual belongs and/or each sequence being an amino acid sequence shown to cross-react with said CTL epitope and;
ii) a composition comprising at least 2 nucleic acid sequences, such as at least 3, at least 4, at least 5, at least 6, at least 7, at least 8, at least 9, at least 10 or at least 11 nucleic acid sequences, each nucleic acid sequence coding for an amino acid sequence which corresponds to or has been shown to cross-react with a CTL epitope from a virus isolate from said individual and/or which corresponds to or has been shown to cross-react with a CTL epitope which is found in virus isolates from more than 50% of a group of infected individuals;

As discussed above is will be preferred that the CTL epitope may in particular be one that reacts only weakly or do not react with the CD8+ T-cells of one or more of the individuals to which the composition is administered.

It may be equally preferred that the candidate individual miss a CD8+ T-cell response to the naturally occurring variant of at least one of the amino acid sequences included in the composition or the medicament, such as at least 2, at least 3, at least 4, at least 5, at least 6, at least 7, at least 8, at least 9, at least 10 or at least 11 of the amino acid sequences included in the composition or the medicament. In this context the term "variant" is to be construed as broadly as possible to comprise amino acid sequences that differ from those present in the composition by substitution of one or more amino acid residues. In particular, "naturally occurring variants" may be amino acid sequences that have less optimal amino acid residues at the primary anchor positions.

In relation to some of the following more specific aspects of the disclosure the term "variant" may refer specifically to any of the amino acid sequences that may be represented by the generic sequences of SEQ ID NOs: 1-11. In particular, a naturally occurring variant may be the variant of the amino acid sequence which is found in the candidate individual.

According to some aspects of the disclosure the virus isolate is one which is is found in more than 50% of a group of infected individuals having a tissue type selected from the group consisting of: HLA-A1, HLA-A2, HLA-A3, HLA-A11, HLA-A24, HLA-B7, HLA-B27, HLA-B44, HLA-B58 AND HLA-B62.

In more specific aspects, the virus isolate is one which is found in more than 50% of a group of infected individuals having the HLA-A2 tissue type. In further specific embodiments, said CTL epitope is specific for the HLA-A2 allele.

In specific embodiments relating to this aspect of the invention the amino acid sequence is a sequence as described above. In particular, the amino acid sequence may comprise any one of the sequences represented by SEQ ID NOs: 1-11. According to similar specific embodiments the nucleic acid molecule is a nucleic acid molecule as described above. In particular the nucleic acid sequence may comprise any one of the following sequences:
GGC CTG GCC GAC CAG CTG ATC CAC CTG (SEQ ID NO: 63) (encoding Vif101(9L) amino acids GLADQLIHL; i.e. SEQ ID NO: 12);
AGC CTG GTG AAG CAC CAC ATG TAC GTG (SEQ ID NO: 64) (encoding Vif23 amino acids SLVKHHMYV; i.e. SEQ ID NO: 13);
GCA CTA GTG GAG ATG GGA CAT CAC GTG (SEQ ID NO: 65) (encoding Vpu66 amino acids ALVEMGHHV; i.e. SEQ ID NO: 14);
CGC CTG CTG AAC GCC TGG GTG AAA GTG (SEQ ID NO: 66) (encoding Gag150(2L) amino acids RLLNAWVKV; i.e. SEQ ID NO: 15);
TTC CTG GGC AAG ATC TGG CCA TCT (SEQ ID NO: 67) (encoding Gag433(8S) amino acids FLGKIWPS; i.e. SEQ ID NO: 16);
AAG CTT GGC AAG GCC GGC TAC GTG GTG (SEQ ID NO: 68) (encoding Pol606 amino acids KLGKAGYVV; i.e. SEQ ID NO: 17);
AAC ATC TGG GCA ACA CAC GCC TGC GTC (SEQ ID NO: 69) (encoding Env67(2i) amino acids NIWATHACV; i.e. SEQ ID NO: 18);
GTG CTG GCC GAA GCT ATG AGC CAG GTC (SEQ ID NO: 70) (encoding Gag362 amino acids VLAEAMSQV; i.e. SEQ ID NO: 19);
AAC ACC GTG GCT ACT CTG TAC TGT GTC (SEQ ID NO: 71) (encoding Gag80 amino acids NTVATLYCV; i.e. SEQ ID NO: 20);
TAC ATC AAG ATT TTC ATC ATG ATC GTG (SEQ ID NO: 72) (encoding Env681 amino acids YIKIFIMIV; i.e. SEQ ID NO: 21);
AGC TTG GGA CAA CAT ATC TAC GAG ACC (SEQ ID NO: 73) (encoding Vpr41 amino acids SLGQHIYET; i.e. SEQ ID NO: 22)

With analogy to the description above the composition may in further preferred embodiments comprise dendritic cells. According these embodiments the method comprises the step of pulsing dendritic cells with an amino acid sequence and/or a molecular entity and/or a composition according to the invention.

Relying on the use of dendritic cells, the method may further comprise any of the steps of:
a) lysing any red blood cells present in a sample taken from an individual, said sample containing mononuclear cells;;
b) concentrating the monocytes present in said sample, e.g. by allowing said monocytes to adhere to a solid support;
c) culturing said monocytes under conditions allowing immature dendritic cells to differentiate from said monocytes;
d) culturing said immature dendritic cells under conditions allowing further maturation of the cells.

In step a) the sample may e.g. be a blood sample or a tissue sample.

In step c) examples of conditions allowing immature dendritic cells to differentiate from said monocytes include those provided in the examples; in particular, the dendritic cells are cultured in an appropriate medium, e.g. the X-VIVO15 medium (commercially available from Cambrex) or the CellGro DC medium (CellGenix)). The medium is preferably supplemented with autologous plasma, preferably in a relative amount of approximately 1%, and recombinant cytokines, GM-CSF, preferably in an amount of 1000 U/ml (Leukine) and IL4, preferably in an amount of approximately 250 U/ml (Cellgro).

In step d) the dendritic cells may in particular be allowed to further matured, preferably in the X-VIVO15 medium or the CellGro DC medium supplemented with approximately 25 ng/ml IL-1ß, approximately 50 ng/ml TNF-α, approximately 3000 U/ml IFN-α, approximately 1000 U/ml IFN-γ, 12,5 µg/ml Polyinosin:polycytidin.

Suitable conditions allowing monocytes to differentiate into dendritic cells and conditions allowing immature dendritic cells to mature include those previously described and those described in the examples.

In preferred embodiments of this aspect of the disclosure the method comprises administration of a composition according to the invention to a single HIV-1 infected individual or a group of individual's which full fill one or more of the following criteria:

For a single HIV-1 infected individual:
1) The patient has the HLA-A2 tissue type
2) An amino acid sequence obtained from the patient's HIV-1 from either proviral DNA or viral RNA contains at least one of the target epitope sequence or an epitope variant sequence to which the corresponding vaccine epitope can induce cross-reaction
3) Before the therapeutic vaccination, the CD8+ T-cells of the infected individual reacts only weakly or not at all with the target epitopes present in his virus

For a group of HIV-1 infected individuals:
1) The individuals in the group have the HLA-A2 tissue type
2) Amino acid sequences from representative HIV-1 strains from the group of. HLA-A2+ individuals or HLA-A2+ HIV-1 infected individual in the same geographic area contain at least one of the target epitope sequence to which the optimal immunogen (the vaccine epitope) induce CD8+ T-cell response or an epitope variant sequence to which the vaccine epitope can induce cross-reaction.

Thus, for individual treatment for therapeutic purposes at least 4 vaccine epitopes fitting the patient's targets should be selected among the amino acid sequences of as described herein. For treating a group of individuals a mix of 4 to 11 of the amino acid sequences should be used to cover as broad as possible.

It is contemplated that the individual may have to meet several additional criteria for in order to qualify for therapeutic vaccination with the compositions according to the invention. Some relevant functional criteria are
a) The individual has a functional immune system, for instance as measured by CD4+ T-cell count >400 and/or
b) The individual has a viral-load < 100,000 and/or
c) The individual does not have other immune defects, wherein examples of other immune defects include but is not limited to HIV-2 infection

The therapeutic vaccine may be administrated as:
1) as peptides in an adjuvant delivered by injection
2) as peptide pulsed autologous dendritic cells generated from mononuclear cells and matured in the presence of a cytokine and/or a growth factor in vitro before pulsing with vaccine immunogens (peptides) for injections
3) as DNA vaccines e.g. naked plasmids or linear expressions cassettes (DNA or RNA) coupled to nanoparticles or delivered by injections and/or by transfection of dendritic cells, by recombinant virus or bacteria
4) mixed prime-boost regimes of the above

When administered as a therapeutic vaccination the composition according to the present invention will be effective in
1) inducing better immunological control with the virus (lower viral-load and/or improvement in CD8+ and/or CD4+ T-cell count or function
2) postponing the need for anti-retroviral therapy medicine
3) allowing withdrawal of anti-retroviral medicine e.g. initiated during the acute HIV-1 infection, or withdraw of anti-retroviral therapy because of side-effects or virus resistance mutations
4) aiding in suboptimal anti-retroviral treatment.

It should be noted that, according to the present invention, embodiments and features described in the context of one of the aspects of the present invention also apply to the other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

The following examples are included to demonstrate particular embodiments of the invention. The following examples are offered by way of illustration and are not intended to limit the invention in any way.

### BRIEF DESCRIPTION OF THE DRAWINGS

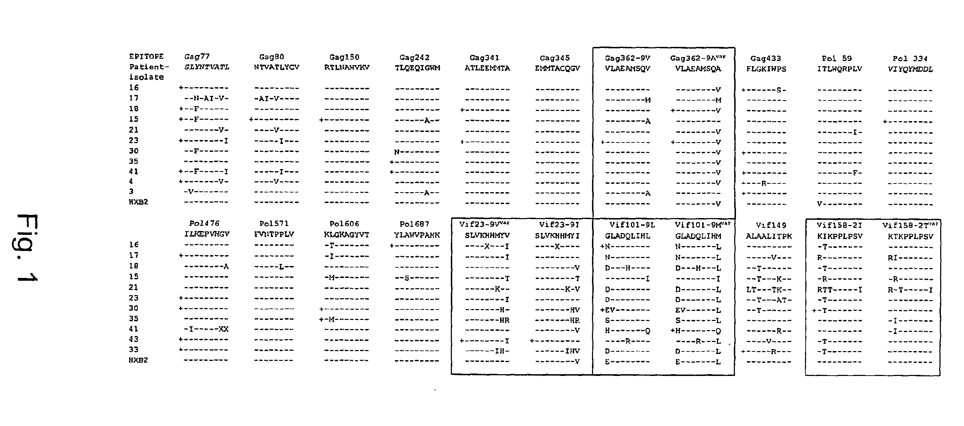

**Figure 1****.** Amino acid sequence alignment of 5 known (italic) and 28 new putative HLA-A2 restricted CTL epitopes including 5 variant epitopes (boxed) in HIV-1 isolates from 11 Danish HLA-A2⁺ HIV-1 infected patients and the HXB2 reference strain. The patient number is indicated. A positive CTL reaction (IC-IFNγ) to the test epitope peptide shown at the top is indicated by a "+" in front of the patient HIV-1 isolate sequence that may be identical to the epitope sequence shown in the top or contain mutations..

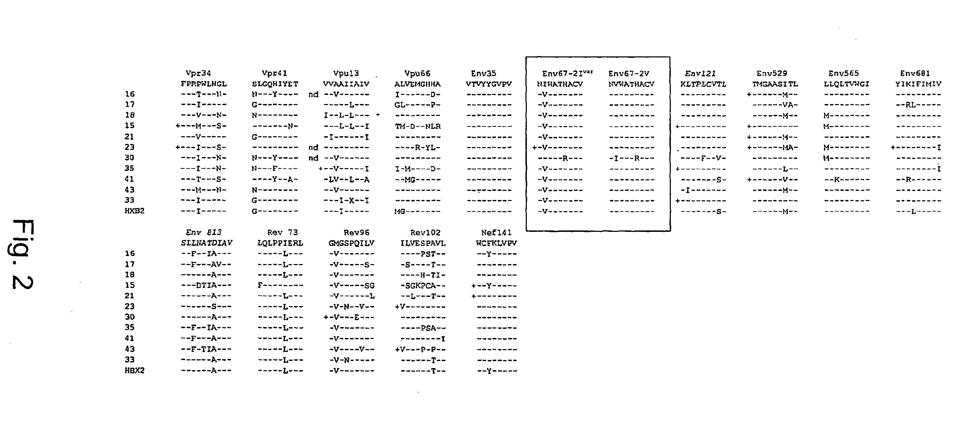

**Figure 2****.** Amino acid sequence alignment of known (italic) and new putative HLA-A2 restricted CTL epitopes, continued.

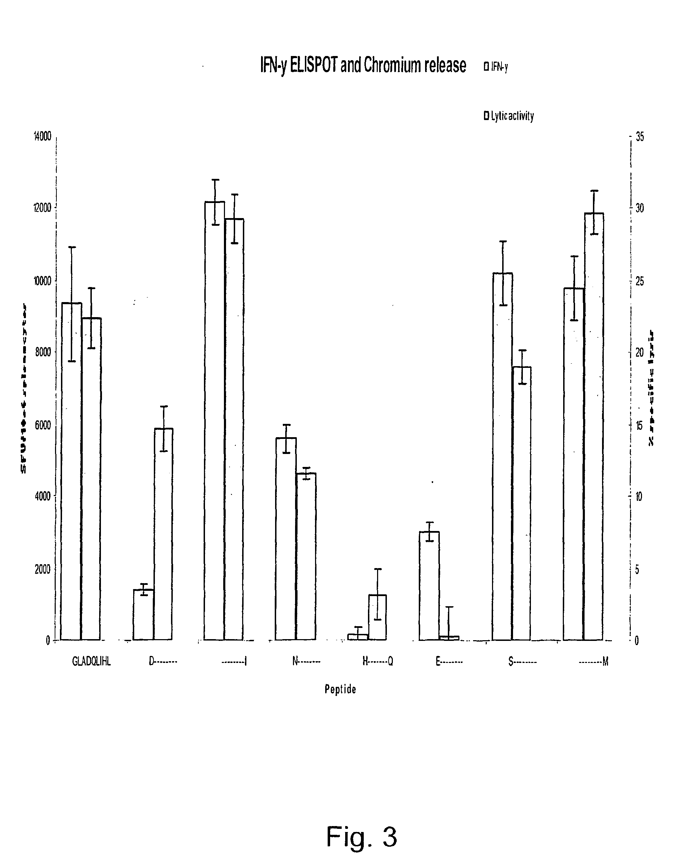

**Figure 3****.** Vif101(9L) specific splenocytes recognize variants of the parental Vif101 epitope. A group of 3 mice were immunized with 10 mill. syngeneic dendritic cells each pulsed with Vif₁₀₁ (9L) (10 µg/ml) and PADRE (5 µg/ml). 10 days post immunization mice were sacrificed and the pooled splenocytes were pulsed with the vaccination peptide (100 µg/ml) at 100 mill/ml and expanded *in vitro* for 5 days receiving 50 iU/ml IL-2 at day 1. At day 5 splenocytes were tested in ELISPOT wells for IFN-γ production measured as spot forming units (sfu) per 10⁶ splenocytes (light shaded bars) when receiving the immunization peptide, the cross-reacting variant peptides or an irrelevant mock peptide (the mock peptide is not shown, but subtracted 44 sfu/10⁶ splenocytes).
   At day 5 splenocytes were also tested in a cytotoxicity assay (% specific lysis showed as dark shaded bars) against EL-4s3 cells pulsed with the immunization peptide, the cross-reacting variant peptides or an irrelevant mock peptide Gag77 (the mock peptide value is not shown, but subtracted 10,8 % lysis). The effector target ratio shown is 50:1. Values shown under the peptide sequence is the *in vitro* IC₅₀ [nM] value for the HLA-A*0201 molecue (the lower value the higher affinity). Bars indicate 95 % confidence limits (n = 3).
   Cross reaction were noted to Vif101(1D), Vif101(9 I), Vif101(1N), Vif101(1S), Vif101(9M), respectively.

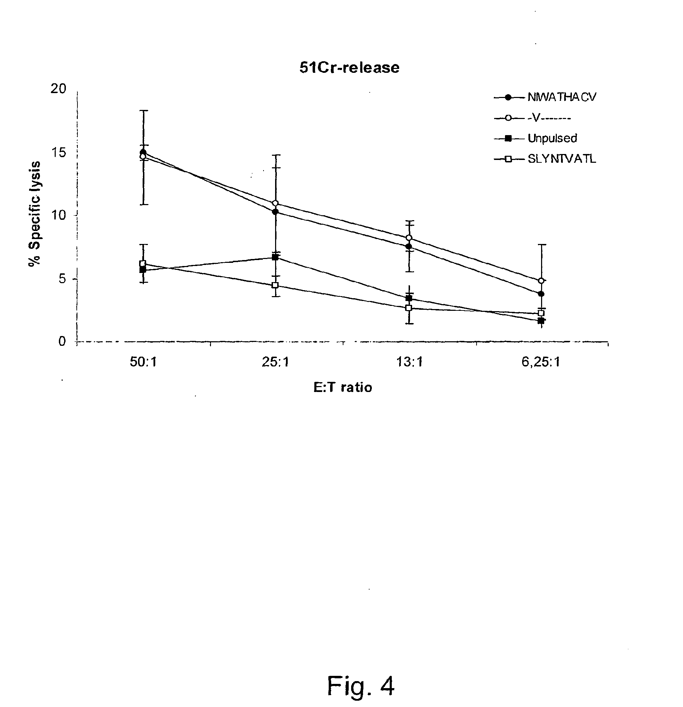

**Figure 4****.** Env67(2I) specific splenocytes recognize its Env67(2V) variant in a cytotoxicity assay. A group of 4 mice were immunized with 4 mill. syngeneic dendritic cells each pulsed with the vaccine immunogen peptide Env67(2I) (10 µg/ml) and PADRE peptide (5 µg/ml). 10 days post immunization mice were sacrificed and the pooled splenocytes were pulsed with the vaccination peptide (100 µg/ml) at 100 mill/ml and expanded *in vitro* for 5 days receiving 50 IU/ml IL-2 at day 1. At day 5 splenocytes were depleted for NK (CD49bDX5) and B-cells (CD19) and tested in a cytotoxicity assay (% specific lysis) against EL-4s3 cells pulsed with the immuization peptide, the cross-reacting variant peptide Env67(2V), an irrelevant mock peptide (Gag77) or unpulsed cells at different effector:target ratios (E:T). Bars indicate 95 % confidence limits (n = 3).

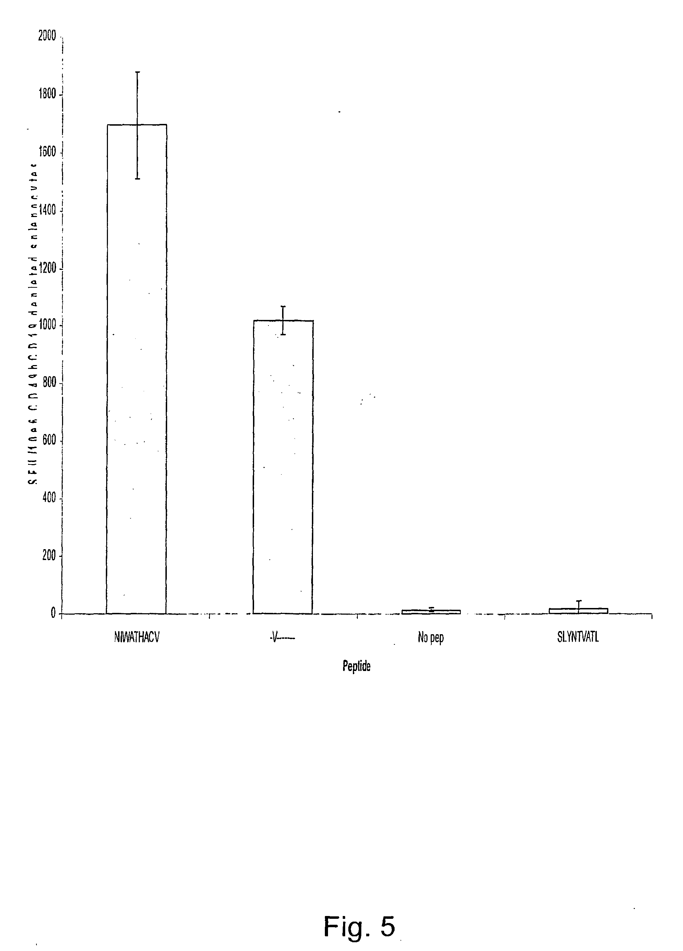

**Figure 5****.** Env67(2I) specific splenocytes recognize its Env67(2V) variant in an IFN-γ ELISPOT assay. A group of 4 mice were immunized with 4 mill. syngeneic dendritic cells each pulsed with Env67(2I) (10 µg/ml) and PADRE (5 µg/ml). 10 days post immunization mice were sacrificed and the pooled splenocytes were pulsed with the vaccination peptide (100 µg/ml) at 100 mill/ml and expanded *in vitro* for 5 days receiving 50 iU/ml IL-2 at day 1. At day 5 splenocytes were depleted for NK (CD49bDX5) and B-cells (CD19) and tested in ELISPOT wells for IFN-γ production (spot forming unite (sfu) per 10⁶ splenocytes) when receiving the immunization peptide, the cross-reacting variant peptide Env67(2V), an irrelevant mock peptide (Gag77 SLYNTVATL) or media only (no pep). Values shown under the peptide sequence is the *in vitro* IC₅₀ [nM] value for the HLA-A*0201 molecue (the lower value the higher affinity).

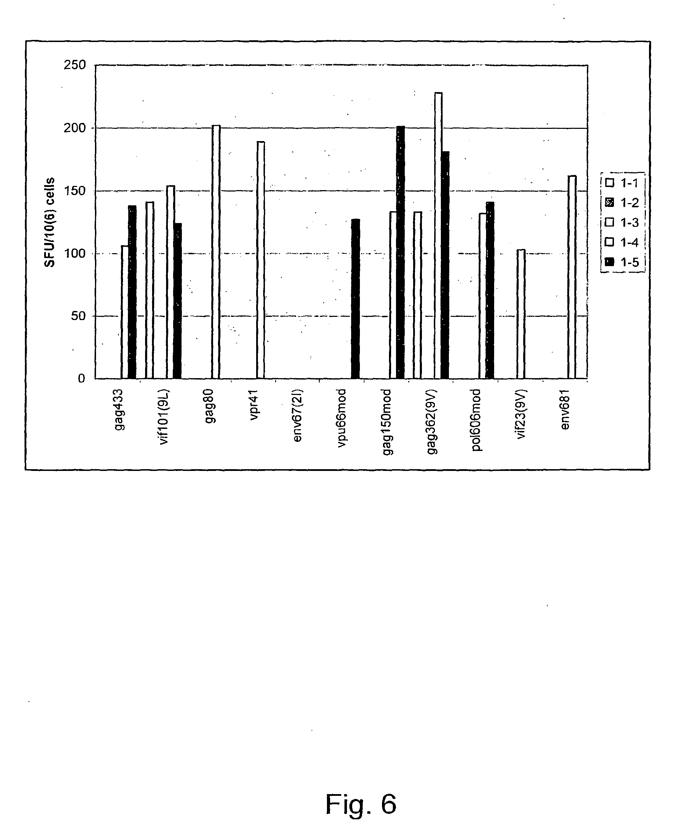

**Figure 6****.** ELISPOT results of peptide mix (11 vaccine epitope peptides plus PADRE) immunisation of 5 animals after 5 days of in vitro stimulation. Results are shown as reactivities of individual mice on each peptide. 10 of the 11 epitopes are recognised after immunization in the group of 5 HLA-A2 tg mice.

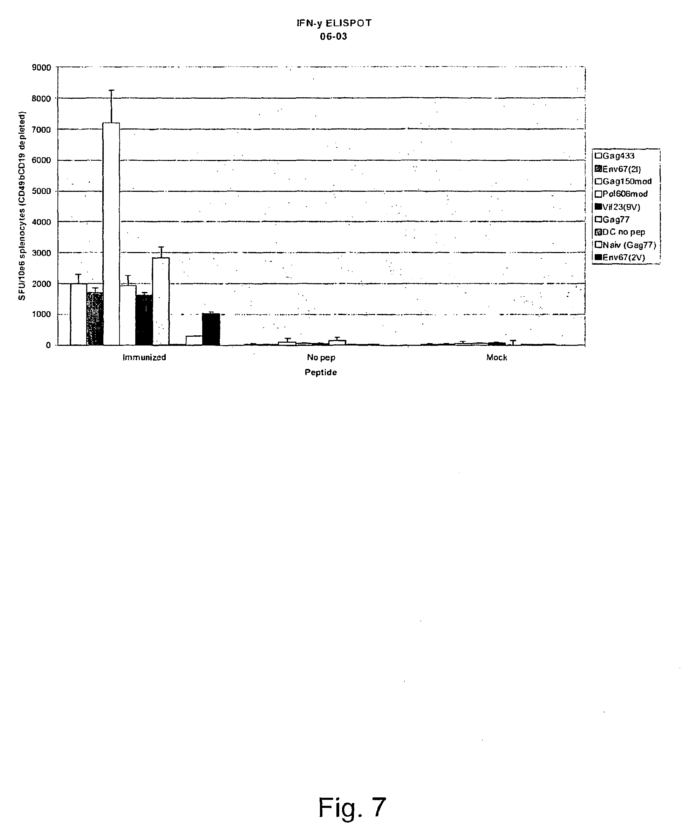

**Figure 7****.** Test of immunogenicity in IFN-γ ELISPOT assay of 5 newly identified HIV-1 CTL epitopes and the control epitope Gag77 (SLYNTVATL) by dendritic cell immunization in HLA-A*0201 positive transgenic mice.
   A group of 4 mice were immunized with 4 mio. syngeneic dendritic cells each pulsed with a T helper epitope PADRE (5 µg/ml) and either of the following test peptides: Gag433 (FLGKIWPS), Gag150mod (RLLNAWVKV), Env67(2I) (NIWATHACV), Pol606mod (KLGKAGYVV), Vif23(9V) (SLVKHHMYV), Gag77 (SLYNTVATL), unpulsed DCs (DC no pep.) or a naïve group *in vitro* expanded with Gag77 (SLYNTVATL). 10 days post immunization mice were sacrificed and the pooled splenocytes were pulsed with the immunization peptide (100 µg/ml) at 100 mio/ml and expanded *in vitro* for 5 days receiving 50 IU/ml IL-2 at day 1. At day 5 splenocytes were depleted for NK cells by anti CD49bDX5 antibodies and B cells by anti CD19 antibodies and tested in IFN-γ ELISPOT (sfu/10⁶ splenocyte) for immune recognition when receiving the immunization peptide, an irrelevant mock peptide (Gag77) or media only (no pep).
   Bars indicate 95 % confidence limits (n = 3).

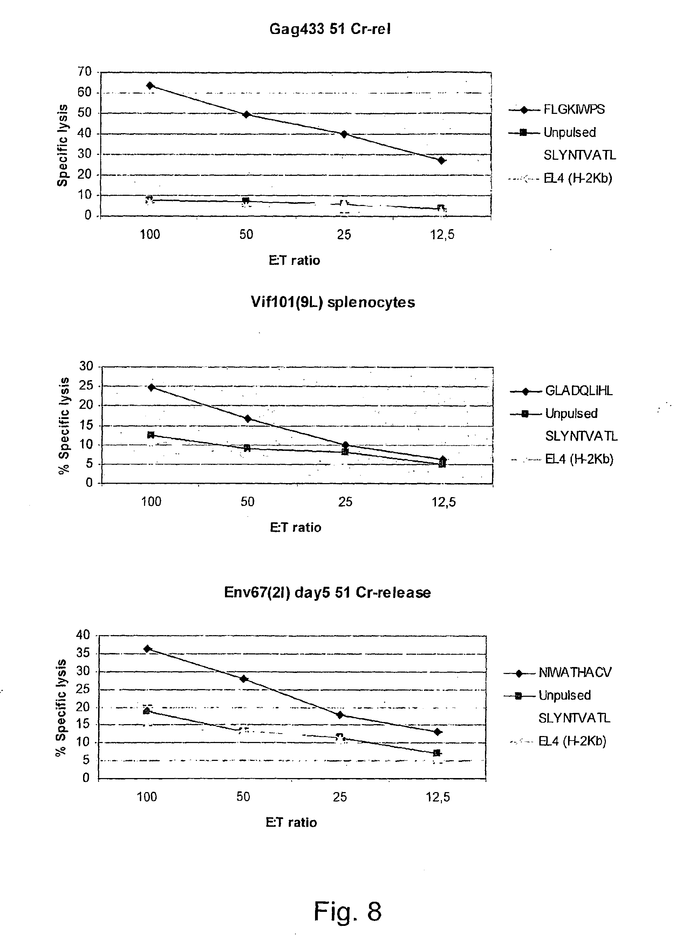

**Figure 8****.** Test of immunogenicity in ⁵¹Chromium release assay of 6 newly identified HIV-1 CTL epitopes and the known control Gag77 (SLYNTVATL) by dendritic cell immunization technology in HLA-A*0201 positive transgenic mice. Groups of 4 mice were immunized with 4 mio. syngeneic dendritic cells each pulsed with a T helper epitope PADRE (5 µg/ml) and either of the following test peptides: Gag433 (FLGKIWPS), Vif101(9L) (GLADQLIHL), Env67(2I) (NIWATHACV),

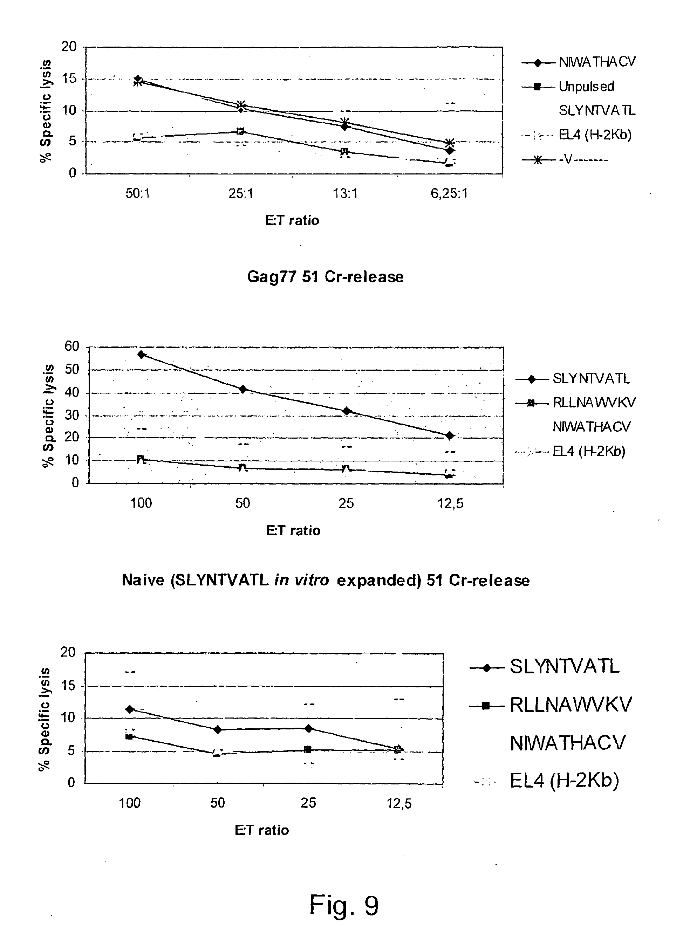

**Figure 9****.** (continuation of fig 8) Env67(2I) (NIWATHACV), Gag77 (SLYNTVATL), or a naïve group *in vitro* expanded with Gag77 (SLYNTVATL)

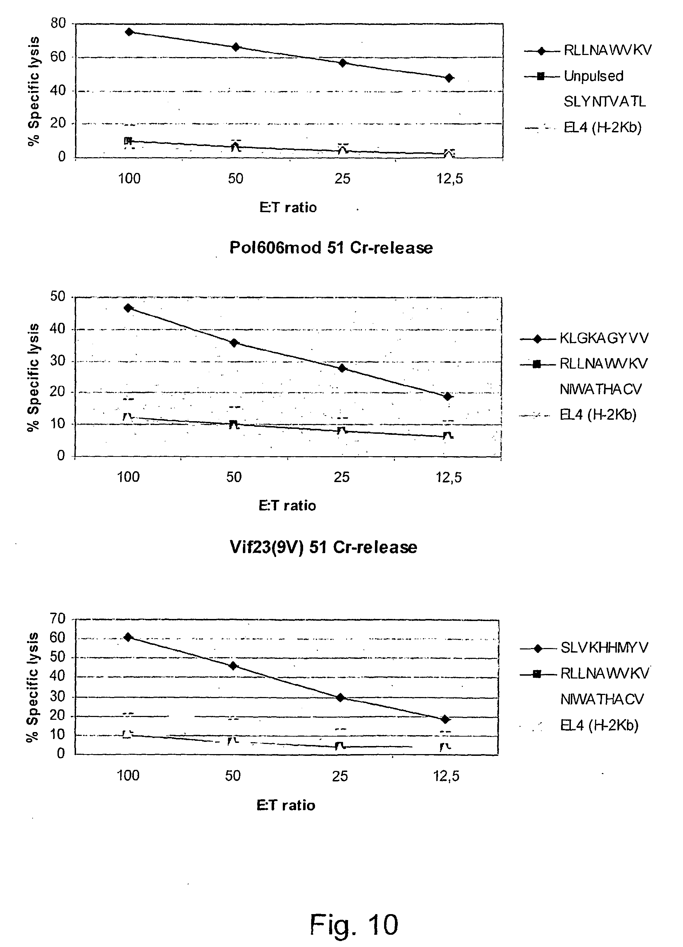

**Figure 10** (continuation of fig 8) Gag150mod (RLLNAWVKV), Pol606mod (KLGKAGYVV), Vif23 (9V) (SLVKHHMYV)

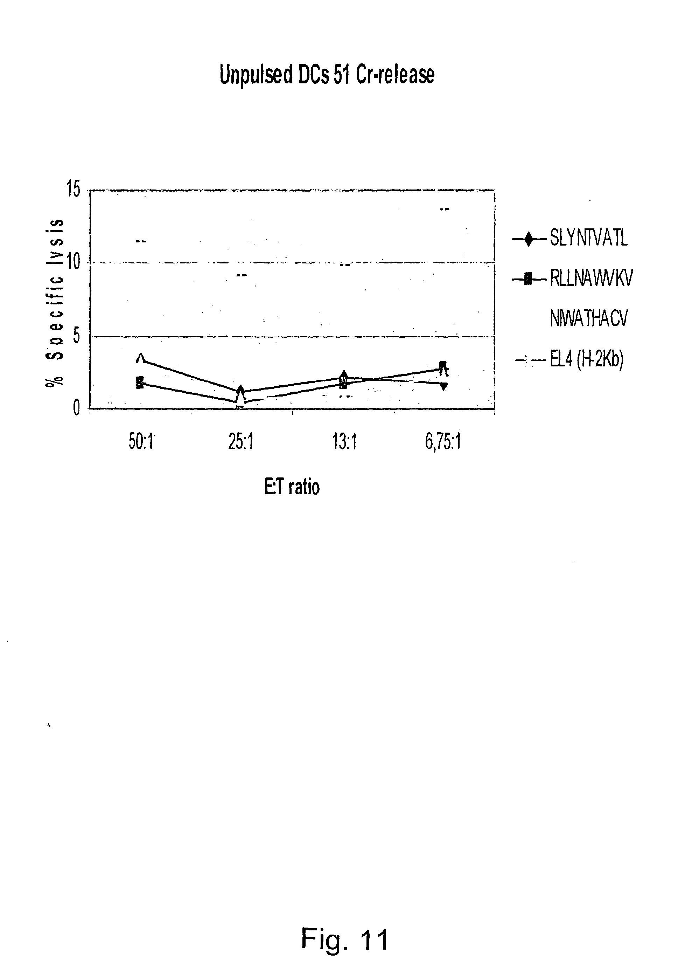

**Figure 11** (continuation of fig 8) unpulsed DCs as negative control. 10 days post immunization mice were sacrificed and the pooled splenocytes were pulsed with the immunization peptide (100 µg/ml) at 100 mio/ml and expanded *in vitro* for 5 days receiving 50 IU/ml IL-2 at day 1. At day 5 splenocytes were depleted for NK cells by anti CD49bDX5 antibodies and B cells by anti CD19 antibodies and tested in a cytotoxic assay (% specific lysis) against EL-4s3 cells pulsed with the immunization peptide, an irrelevant mock peptide, unpulsed target cells and finally EL-4 H-2Kb target cells at different effector:target ratios (E:T).
   Bars indicate Std. dev (n = 3).

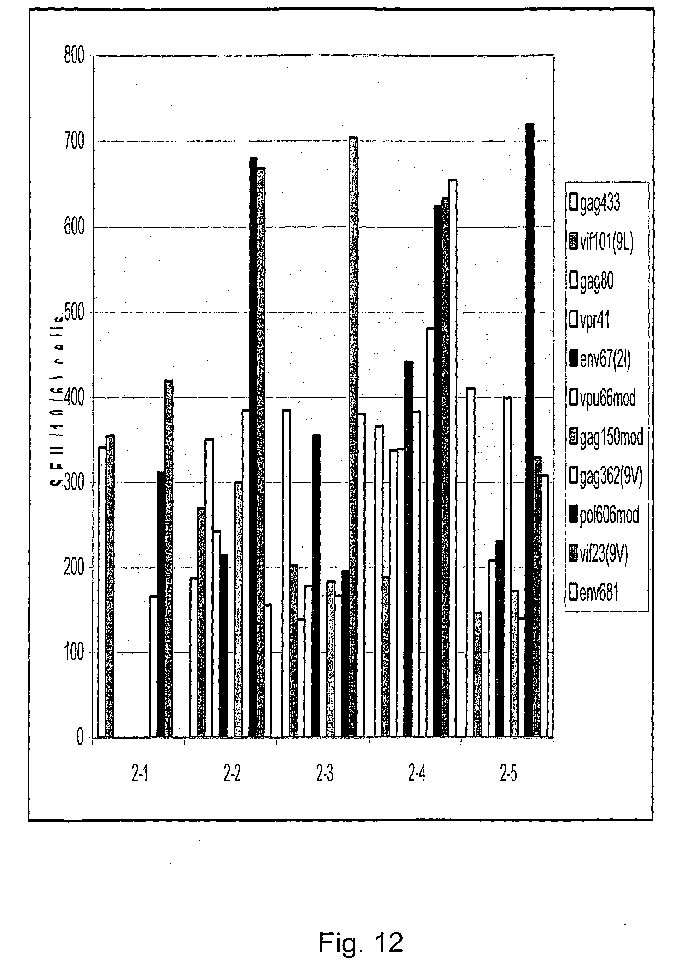

**Figure 12****.** ELISPOT results of pcDNA3.1-poly11 DNA immunisation of 5 animals after 5 days of in vitro stimulation. Results are shown as the reactivity of each mouse on individual peptides.

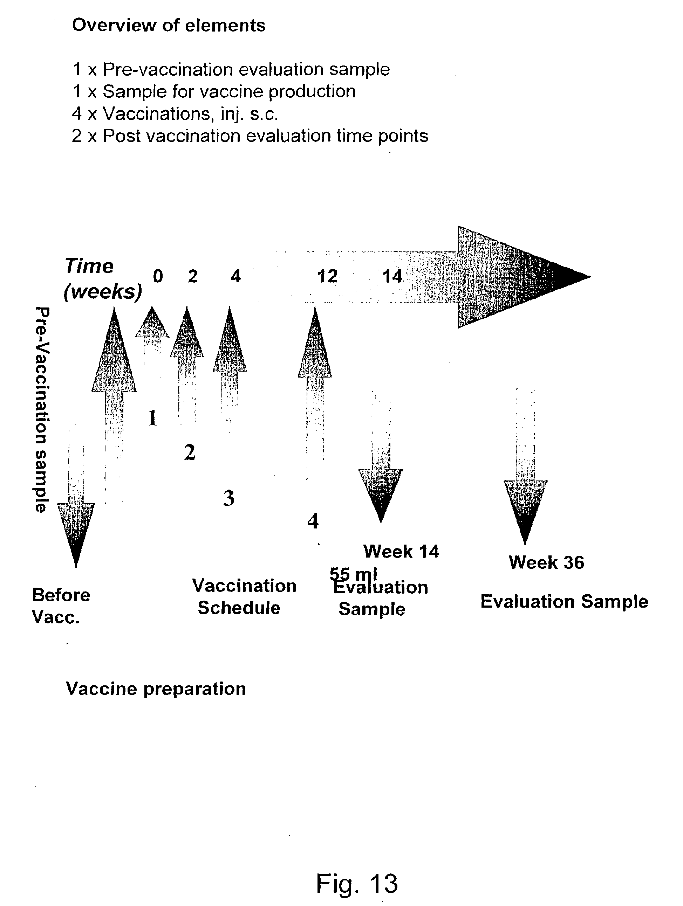

**Figure 13****.** Study design employed in the HIV-peptide pulsed Dendritic Cell (DC) therapeutic vaccination trial. The total scheduled number of patients enrolled in the study was 12. The complete design includes eight elements, including a pre-enrollment baseline evaluation for naturally occurring responses against HIV-peptides included in the vaccine. "Vaccine preparation" designates collection of 500 ml peripheral blood from the enrolled patient, for use in denditic cells production. The resulting DC cells were afterwards pulsed using the vaccine peptides and the pulsed cells were aliquoted into 4 vaccination vials for subsequent use in the vaccination phase. The vaccination phase consisted of 4 vaccinations over a period of 12 weeks, including individual vaccinations at 0, 2, and 4 weeks, plus a "booster" vaccination at week 12. After the completion of the vaccine phase an evaluation sample was collected in order to investigate whether the patient showed a response in terms of novel immunological responses to the vaccine peptides as well as the native HIV wild type equivalents of the vaccine peptides. Finally, a long term follow up evaluation was performed after 36 weeks with intentions of evaluating parameters such as CD4 count, viral load and persistence of novel induced immunological responses to the vaccine peptides.

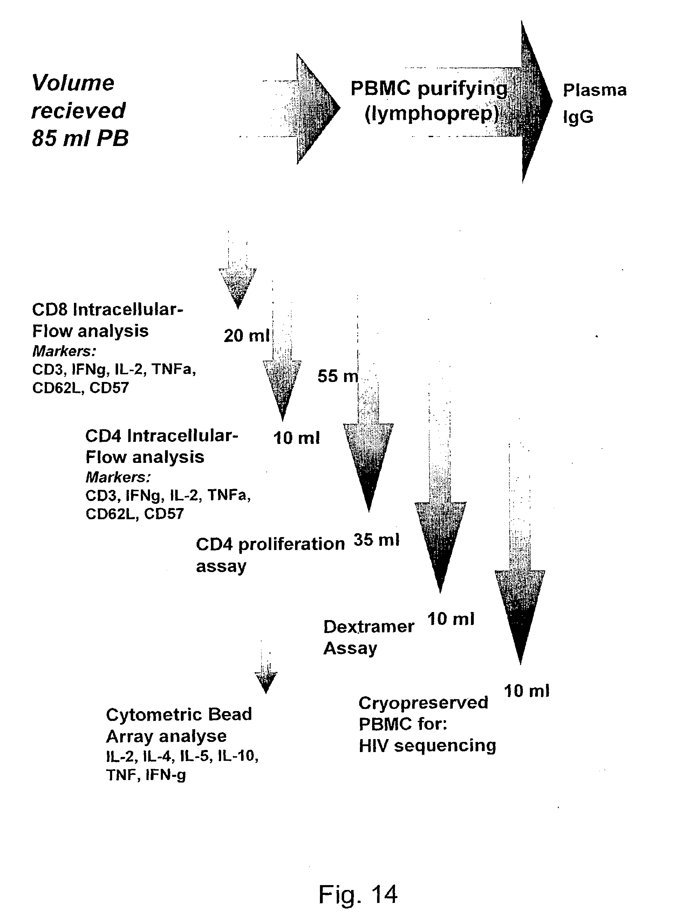

**Figure 14****.** Organization of individual assay-systems performed for the pre-enrollment base line sample, the week 14 evaluation (post-vaccination) sample, and the week 36 post-vaccination evaluation. A total volume of 85 ml peripheral blood was received from the patient. A CD8 and a CD4 in vitro peptide stimulation aimed at showing biological response to the vaccine peptides are performed, evaluating the response in terms of intra-cellular cytokine production. An in vitro proliferation assay was performed using the CD4 "helper" epitopes to show potential activation of the patients CD4 cells using cell divisions in vitro as a measure of the cells ability to respond biologically to the CD4 elements of the vaccine. From both the intracellular CD4 and CD8 assays as well as from the CD4 proliferation assays, supernatant was harvested for subsequent cytometric bead array analysis, evaluating the generation of IL-2, IL-4, IL-5, IL-10, TNF-a and IFN-g from the in vitro stimulated cells. This assay shows the immunological competence of any CD4 and CD8 cells, respectively, responding to the vaccine and hence measures the proficiency of the immunological response generated by the vaccine. Finally, a dextramer assay was performed to show specific generation and responsiveness of cytotoxic CD8 cells, evaluated on a peptide by peptide basis.
   Furthermore, cells were cryopreserved for later ex vivo expansion and full length sequencing of the patient specific HIV genome.

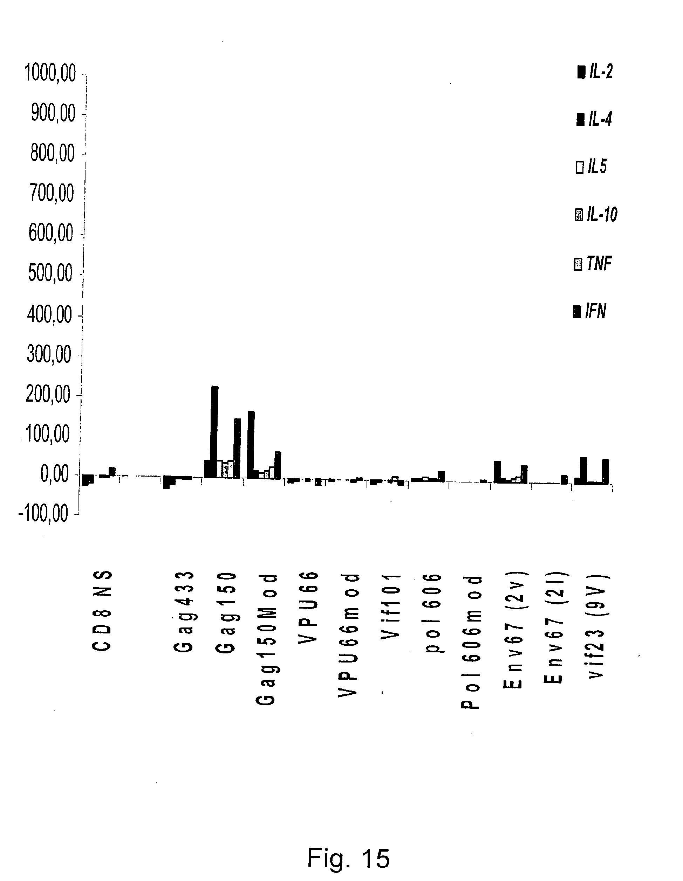

**Figure 14** and **15** demonstrates the immunological response to therapeutic HIV vaccination in a HIV-patient. Figure 14 shows the response to HIV-wild type as well as to the corresponding vaccine variants in the pre-vaccination baseline sample. This particular patient shows a faint CD8 mediated response to one HIV peptide, the Gag150, as well as to the corresponding vaccine epitope Gag150(Modified). The response is regarded to be a natural response to a HIV derived peptide, with a weak cross reaction to a corresponding modified peptide. "NS" indicates the background as found when subjecting the CD8 cells to a "mock" culture with no peptide present.
Figure 15 shows the corresponding response to the same panel of HIV-wild type peptides and vaccine variants of the peptides. A noticeably response in this post-vaccination evaluation sample was seen after ex vivo stimulation with Gag433, Gag150, VPU66, Pol606, and Env67 (2V). A slightly lower response was seen in Vif101, Pol606 (Modified), and Env67 (2L, Modified). Overall, this indicates that in this patient, DC based therapeutic vaccination induced novel immunological responses toward HIV-wild type and vaccine variants of the employed peptides.

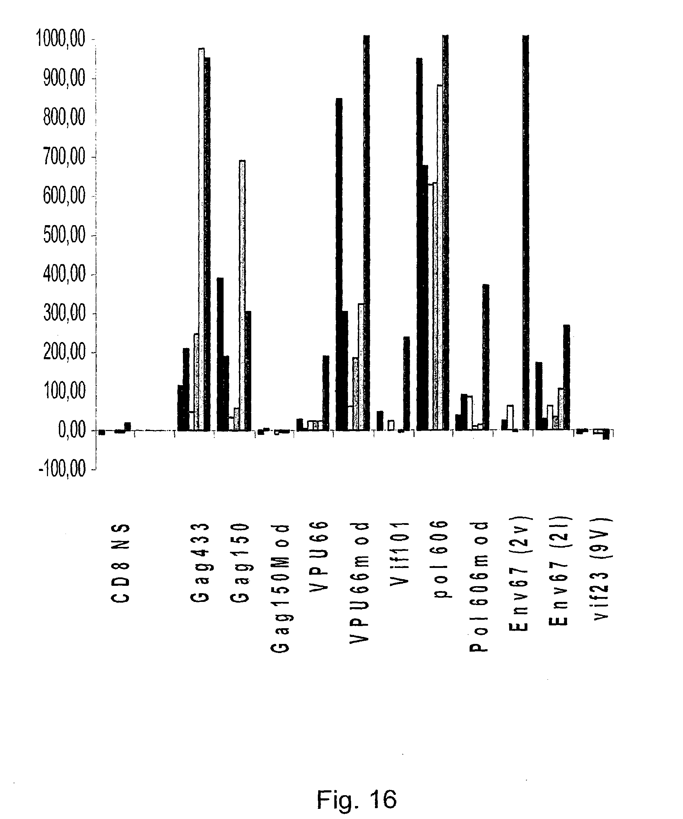

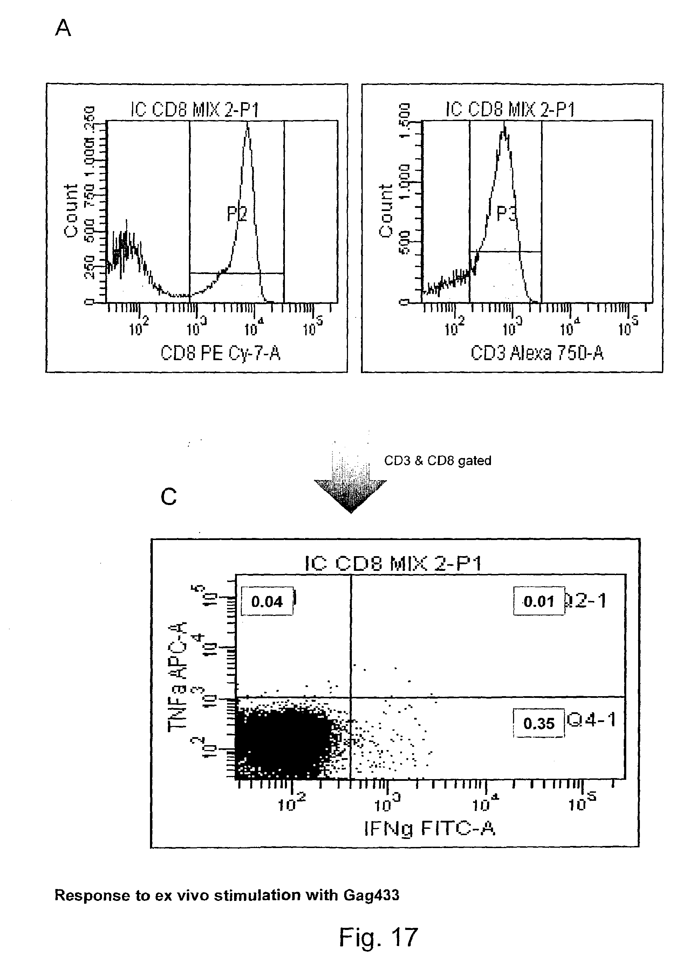

**Figure 16** shows substantiation of the Gag433 findings in Figure 15. Figure 16A-C shows intracellular evaluation of ex vivo Gag433 stimulated CD8 cells ability to produce TNF and IFN-g. Figure 16A and B is representative histograms showing the CD3 and CD8 marker expression on mononuclear cells in the culture. Signal from CD3 and CD8 dual positive cells are subsequently gated to a dual histogram evaluating IFN and TNF. Figure 16C shows a significant response in terms of interferon-g producing CD3+/CD8+ cells responding to the presence of Gag433 in the culture, with an additional small population producing TNF. By and large, this confirmed the findings presented in Figure 15 for Gag433, and shows that precisely measuring and quantitating the immunological responses to the DC based therapeutic vaccine is possible using these assays.

### EXAMPLES

### Example 1

### Sequence conservation of HLA-A2 binding CTL epitopes in HIV-1 clinical isolates

### Materials and methods

### Patients and viral isolates

Blood samples were collected from HIV-1 positive patients at the University Hospital of Copenhagen and Hvidovre according to the following criteria: CD4⁺ cell count > 300/µl and measurable viral load (>1,000 RNA copies/ml) without receiving highly active antiretroviral therapy (HAART). HLA-A and HLA-B genotyping of the patients was performed on DNA purified from peripheral blood mononuclear cells (PBMC) using either HLA-A or HLA-B Sequencing-Based Typing Kit (PE Biosystems) according to instructions by the manufacturer. MatchTool/MatchMaker and MT Navigator software (PE Biosystems) were used for allele identification. The patients gave informed consent to the study that was approved by the Ethical committee of the Copenhagen hospitals.

### Sequencing of full-length HIV-1 patient isolates

QIAamp™ DNA Blood Midi Kit (QIAgen Inc., Germany) was used to purify high molecular weight genomic DNA from 2 ml fresh whole blood stored in anticoagulant EDTA buffer or from PBMC isolated by Ficoll-Hypaque according to the manufacturer's protocol. For primary long PCR, the primers pcrpbs (5'atctctagcagtggcgcccgaacag 3') and pcru3LTRas (5' agcagctgcttatatgcatctgag 3') were designed in conserved regions of HIV-1 LTR to amplify the near full-length genome of HIV-1 (8811 bp). Semi-nested PCRs using pcrpbs and pcrpol4as (5' ttactactgccccttcacctttcca 3') were performed and yielded overlapping amplicons of approximately 5 Kb from the 5' prime end and the 3' prime end, respectively. PCR mix contained 200 ng genomic DNA, 10 µM of each primer, 10 nM dNTP and 2.5 U of the DNA polymerases, Taq and proof-reading Pwo (Expand™ Long Template System, Roche) (Salminen *et al.,* 1995). The 10 cycles were 10 sec. at 94 °C, 30 sec. at 65 °C, and 5 min. at 68 °C followed by 20 cycles with 15 sec. increment in elongation per cycle. The amplified PCR product of the correct length was purified using QIAquick™ PCR purification columns kit (Qiagen Inc., Germany). Nucleotide sequences were obtained by direct sequencing of the amplified PCR product, using sets of cycle sequencing primers covering the entire genome in the sense and antisense directions, respectively. Sequencing was carried out using Big Dye fluorescent dye-labelled terminator kit vs. 3.0 and run on the ABI3100 capillary sequencer (Applied Biosystems Inc., Foster city, California). Sequence analysis was preformed using Sequence Navigator, Autoassembler, and DNASTART™ software packages.

### HLA-A*0201 tg mice vaccine models

The cross-reactivity of selected peptide epitopes was determined in individual C57-A2K^{b} transgenic mice (Epstein *et al.,* 1989) as described earlier (Corbet *et al.,* 2003). In short, these C57-A2K^{b} tg mice express a chimeric heavy chain of the MHC I molecule (HLA-A201 α1 and α2 and H-2K^{b} α3, transmembrane and intracytoplasmic domains) and were kindly provided by N. Holmes. The 6-12 weeks old C57-A2K^{b} tg mice were immunised s.c. with 100 µg test peptide plus 120 µg of a T-helper peptide (TPPAYRPPNAPIL) SEQ ID NO: 58 in 100 µl incomplete Freunds adjuvants (Corbet *et al.,* 2003). Splenocytes were harvested on day 10 and restimulated with peptide-loaded mitomycin C-treated C57-A2K^{b}tg blast cells and a standard 5 hour ⁵¹Cr-release assay was performed on peptide pulsed or unpulsed HHD-EL4S3-Rob target cells at different E:T cell ratios. The threshold for being considered positive was >10 % specific lysis at E:T ratio 50:1 and significantly different (α = 0.05) from the background activity. The unpulsed negative controls had approximately 0 % lysis.

For ELISPOT assay mouse spleen cells were plated on 96-well nitrocellulose microtiter plates (MAHA S45 10, Millipore, Bedford, MA) that where coated overnight at 4 °C with 50 µl/well of 0.5 µg/ml anti-IFN-y Mab (Pharmingen) in 0.1 M sodium bicarbonate buffer, pH 9.6. Serial 2-fold dilution of cells (500,000-62,500 cells /wells) were performed in duplicates and peptides were added to the wells at a final concentration of 10 µg/ml in complete RPMI-1640 medium supplemented with 5 IU/ml of rIL-2. The plates were incubated at 37 °C, 5% CO₂ for 16 hours and then processed as previously described (Corbet *et al.,* 2003). The number of specific IFN-γ secreting T cells is expressed as number of sfu per10⁶ cells. The cut-off for positivity was 10 sfu/10⁶ input cells and 2 time the negative control.

To study cross-reactivity (TCR promiscuity) to several variant peptide epitopes of the naturally anchor-optimized Vif101(9L) variant epitope-specific CTLs, HLA-A2 tg HHD mice kindly provided by F.A. Lemonnier were used (Pascolo *et al.,* 1997). HHD mice are H-2D^{b-/-} and β2m^{-/-} double knockout mice. Isolated bone marrow cells from HHD transgenic mice were kept overnight in culture medium (RPMI 1640 with 10 % FCS, penicillin, streptomycin, 2-mercapta-ethanol). The next day, the non-adhering cells were isolated and cultured with GM-CSF, 10 ng/ml, and IL-4, 20 ng/ml. At day 3, fresh culture medium with GM-CSF and IL-4 were supplied. At day 6, the cells were harvested and cultured overnight with GM-CSF, IL-4 and LPS, 1 µg/ml. At day 7, mature dendritic cells were harvested and used for vaccination. HHD transgenic mice, 8-12 weeks old, were injected s.c. with syngenic mature dendritic cells pulsed with the test peptide and the T-helper peptide. At day 11 after vaccination, the mice were sacrificed and the primed splenocytes were pooled and re-stimulated with the vaccination-peptide. Briefly, the splenocytes (100 x 10⁶/ml) were loaded with the vaccination-peptide, 100 µg/ml, for 3 hours at 37 °C and at day 1 after re-stimulation, IL-2, 100 IU/ml, was added. After 5 days of re-stimulation, the CTLs were harvested and used for the Cr-release assay, or kept another 6 days in culture media with IL-2 and tested in the ELISPOT assay. Same procedure was used when demonstrating the cross-reaction induced by other epitope immunogens, e.g. Env67(2i) that induced cross-reaction to Env67(2V).

Animal experiments were approved and done in accordance with the legal requirements in Denmark.

### Results

### Selection of HLA-A2-binding HIV-1 peptides

The epitopes studied were selected from previously identified A2-binding putative HIV-1 CTL epitopes (Corbet *et al.,* 2003). The epitope peptides selected for further study induced cellular immunity as measured by cytotoxicity and/or IFNγ induction in splenocytes in groups of immunised HLA-A2tg mice and/or were recognised by CD8⁺ T cells in HIV-1 infected HLA-A2⁺ and HLA-A2⁻ patients as measured by IC-FACS for specific IFNγ production from CD8⁺ T cells. A total of 28 naturally occurring epitopes and 5 variant epitopes fulfilled these criteria (Table 3). Of these novel epitopes, 22 induced immune responses in both HIV-1-infected patients and A2tg mice. In addition, the recognition of the A2-binding epitopes by CD8⁺ T cells was studied in non-A2 but HIV-1 infected patients (Table 3). Thus, 12 of the 28 epitopes were recognized also by non-A2 patients suggesting processing and immunogenicity *in vivo* and ability to bind other alleles than HLA-A2.

**Table 3. Sequence and immunogenicity of new HIV-1 CTL epitopes in groups of HLA-A201 tg mice and their immune recognition in 17 HLA-A2⁺ and 16 HLA-A2⁻ HIV-infected patients (left). The presence of identical epitope sequence in patient HIV-1 isolates and the immune recognition of the corresponding epitope (IC-FACS) in 11 of the 17 HLA-A2+ patients (right)**

| | | **A2 tg mice** **(n = 6)** | | **Patients** **(IC-IFNγ)** | |
|---|---|---|---|---|---|
| | | | | **A2** **(n=17)** | **Non-A2** **(n=16)** |
| **Epitopes** | **Sequence** **(SEQ ID NO)** | **CTL** | **IFNg** | | |
| **Gag 80** | NTVATLYCV (20) | 4 | 4 | 1 | 0 |
| **Gag 150** | RTLNAWVKV (22) | 2 | 0 | 1 | 0 |
| **Gag 242** | TLQEQIGWM (33) | 0 | 0 | 2 | 1 |
| **Gag 341** | ATLEEMMTA (34) | 0 | 0 | 2 | 0 |
| **Gag 345** | EMMTACQGV (35) | 1 | nd | 1 | 0 |
| **Gag 362(9V)** | VLAEAMSQV (36) | 3 | 4 | 1 | 1 |
| ***Gag 362(9A)^{var}*** | *VLAEAMSQA* (37) | *2* | *0* | *2* | *1* |
| **Gag 433** | FLGKIWPS (27) | 2 | 1 | 7 | 0 |
| **Pol 59** | ITLWQRPLV (38) | 2 | 2 | 0 | 2 |
| **Pol 571** | FVNTPPLV (39) | 1 | 0 | 0 | 1 |
| **Pol 606** | KLGKAGYVT (28) | 0 | 0 | 2 | 1 |
| **Pol 687** | YLAWVPAHK (40) | 0 | 1 | 1 | 1 |
| **Env 35** | VTVYYGVPV (41) | 0 | 1 | 0 | 0 |
| **Env 67(2V)** | NVWATHACV (29) | 1 | 1 | 0 | 0 |
| ***Env 67(2I)^{var}*** | *NIWATHACV* (18) | *2* | *2* | *1* | *0* |
| **Env 529** | TMGAASITL (42) | 0 | 0 | 5 | 0 |
| **Env 565** | LLQLTVWGI (43) | 0 | 2 | 0 | 0 |
| **Env 681** | YIKIFIMIV (44) | 2 | 1 | 2 | 1 |
| **Rev 73** | LQLPPIERL (45) | 4 | 0 | 0 | 1 |
| **Rev 96** | GMGSPQILV (46) | 0 | 1 | 1 | 0 |
| **Rev 102** | ILVESPAVL (47) | 0 | 0 | 2 | 0 |
| **Vif 23(91)** | *SLVKHHMYI* (24) | *1* | *0* | *1* | *0* |
| ***Vif 23(9V)^{var}*** | SLVKHHMYV (13) | 4 | 4 | 1 | 1 |
| **Vif 101 (9L)** | GLADQLIHL (12) | 2 | 2 | 3 | 3 |
| ***Vif 101(9M)^{var}*** | *GLADQLIHM* (23) | *1* | *1* | *1* | *1* |
| **Vif 149** | ALAALITPK (48) | 1 | 1 | 1 | 0 |
| **Vif 158(21)** | KIKPPLPSV (49) | 0 | 2 | 1 | 1 |
| ***Vif 158(2T)^{var}*** | *KTKPPLPSV* (50) | *0* | *0* | *0* | *0* |
| **Vpr 34** | FPRPWLHGL (51) | 0 | 1 | 3 | 2 |
| **Vpr 41** | SLGQHIYET (22) | 2 | 2 | 0 | 2 |
| **Vpu 13** | VVAAIIAIV (52) | 0 | 0 | 2 | 0 |
| **Vpu 66** | ALVEMGHHA (25) | 0 | 0 | 0 | 1 |
| **Nef 141** | WCFKLVPV (53) | 0 | 1 | 2 | 2 |
| **Control Gag 77** | SLYNTVATL (54) | 4 | 2 | 10 | 1 |
| **Control Pol 334** | VIYQYMDDL (55) | nd | | 1 | 1 |
| **Control Pol 476** | ILKEPVHGV (56) | nd | | 9 | 4 |
| **Control Env 121** | KLTPLCVTL (57) | nd | | 3 | 2 |

| | | | | | |
|---|---|---|---|---|---|
| nd: not determined *Where nothing is noted the -/- combination was found | | | | | |

| | | | **A2 tg mice** **(n=6)** | | | **Patients** **(IC-IFNγ)** | | | **Epitope sequence identity / IC-IFNγ response of patient** **CD8+ T cells*** | | | |
|---|---|---|---|---|---|---|---|---|---|---|---|---|
| | | | | | | **A2** **(n=17)** | **Non-A2** **(n=16)** | | | | | |
| **Epitopes** | | | | | | | | | | | | **Tot** |
| | **P15** | **P16** | **P17** | **P18** | **P21** | **P23** | **P30** | **P33** | **P35** | **P41** | **P43** | |
| **Gag 80** | +/+ | +/- | | +/- | | | +/- | +/- | +/- | | | 6/1 |
| **Gag 150** | +/+ | +/- | +/- | +/- | +/- | +/- | +/- | +/- | +/- | +/- | +/- | 11/1 |
| **Gag 242** | | +/- | +/- | +/- | +/- | +/- | | | +/+ | +/+ | +/- | 8/2 |
| **Gag 341** | +/- | +/- | +/- | +/+ | +/- | +/+ | +/- | +/- | +/- | +/- | +/- | 11/2 |
| **Gag 345** | +/- | +/- | +/- | +/- | +/- | +/- | +/- | +/- | +/- | +/- | +/- | 11/0 |
| **Gag 362(9V)** | | +/- | | +/- | +/- | +/+ | +/- | | +/- | +/- | +/- | 8/1 |
| ***Gag 362(9 A)^{var}*** | +/- | | | -/+ | | -/+ | | +/- | | | | 2/2 |
| **Gag 433** | +/- | -/+ | +/- | +/- | +/- | +/- | +/+ | +/+ | +/- | +/+ | -/+ | 9/4 |
| **Pol 59** | +/- | +/- | +/- | +/- | | +/- | +/- | +/- | +/- | | +/- | 9/0 |
| **Pol 571** | +/- | +/- | +/- | | +/- | +/- | +/- | +/- | +/- | +/- | +/- | 10/0 |
| **Pol 606** | | | | +/- | +/- | +/- | +/+ | +/- | -/+ | +/- | +/- | 7/2 |
| **Pol 687** | | +/+ | +/- | +/- | +/- | +/- | +/- | +/- | +/- | +/- | +/- | 10/1 |
| **Env 35** | +/- | +/- | +/- | +/- | +/- | +/- | +/- | +/- | +/- | +/- | +/- | 11/0 |
| **Env 67(2V )** | +/- | +/- | +/- | +/- | +/- | +/- | | +/- | +/- | +/- | +/- | 10/0 |
| ***Env 67(2I )^{var}*** | | | | | | -/+ | | | | | | 0/1 |
| **Env 529** | +/+ | -/+ | | | -/+ | -/+ | +/- | +/- | | -/+ | | 3/5 |
| **Env 565** | | +/- | +/- | | +/- | | | +/- | +/- | | +/- | 6/0 |
| **Env 681** | +/- | +/+ | | +/- | +/- | -/+ | +/- | +/- | | | +/- | 7/2 |
| **Rev 73** | | | | | | | | | | | | 0/0 |
| **Rev 96** | | | | | | | -/+ | | | | | 0/1 |
| **Rev 102** | | | | | | -/+ | +/- | | | | -/+ | 1/2 |
| **Vif 23(9I )** | | | +/- | | | +/- | | | | | +/+ | *3*/*1* |
| ***Vif 23(9V* )^{var}** | | | | +/- | | | | | | | -/+ | 1/1 |
| **Vif 101 (9L)** | | -/+ | | | | -/+ | | | | | | 0/2 |
| ***Vif 101(9 M)^{var}*** | | | | | | | | | | -/+ | +/- | 1/1 |
| **Vif 149** | | +/- | | | | | | -/+ | | | | 1/1 |
| **Vif 158(2 I)** | | | | | | | -/+ | | +/- | +/- | | 2/1 |
| ***Vif 158(2 T)^{var}*** | | +/- | | | | +/- | | +/- | | | +/- | 4/0 |
| **Vpr 34** | -/+ | | | | | -/+ | | | | | | 0/2 |
| **Vpr 41** | | | | | -/nd | +/- | | | | | | 1/0 |
| **Vpu 13** | | /n.d. | | | | +/- | -/nd. | | -/+ | | | 2/1 |
| **Vpu 66** | | | | +/- | +/- | | +/- | +/- | | | +/- | 5/0 |
| **Nef 141** | -/+ | | +/- | +/- | +/+ | +/- | +/- | +/- | +/- | +/- | +/- | 9/2 |
| | -/+ | +/+ | -/- | -/+ | | -/+ | | | +/- | -/+ | -/+ | 2/6 |
| **Contr ol Gag 77** | | | | | | | | | | | | |
| | +/+ | +/- | +/- | +/- | +/- | +/- | +/- | +/- | +/- | +/- | +/- | 11/1 |
| **Contr ol Pol 334** | | | | | | | | | | | | |
| | +/- | +/- | +/- | | +/- | +/+ | +/+ | +/+ | +/- | -/- | +/+ | 9/4 |
| **Contr ol Pol 476** | | | | | | | | | | | | |
| | +/+ | +/- | +/- | +/- | +/- | +/- | | +/+ | +/+ | | | 8/3 |
| **Contr ol Env 121** | | | | | | | | | | | | |

| | | | | | | | | | | | | |
|---|---|---|---|---|---|---|---|---|---|---|---|---|
| nd: not determined *Where nothing is noted the -/- combination was found | | | | | | | | | | | | |

### Conservation of epitopes in HIV-1 isolates from untreated Danish patients

To investigate the presence of our newly identified HIV-1 epitopes in Danish HIV-1 patient isolates, we full-length sequenced the patients' viral isolates from PBMCs of 11 of the HLA-A2 positive HIV-1 infected individuals. Table 4 shows the amino acid sequence conservation of the 28 new HLA-A2-binding HIV-1 epitopes among the 11 Danish patient isolates as well as among the HIV-1 subtype B and globally (subtype A-J) and in the 2005 www.lanl.hiv.gov database. The conservation of 10 variant epitopes that was synthesised and tested for immunogenicity is also shown. In general, the epitopes identified in the structural proteins Gag, Pol, and Env plus Nef were more conserved in the clinical isolates than the epitopes in the shorter and more variable accessory and regulatory proteins Vpu, Vif, Vpr, and Rev (Table 4). More than half of the identified epitopes (16/28) were present in 6 or more of the 11 patient isolates and thus relatively well conserved among the sequenced Danish HIV-1 isolates. In general, the conservations of the epitopes in Danish isolates followed the conservation among other subtype B isolates in the 2005 database, which is not surprising since all the 11 Danish isolates, but one subtype C in patient 15, were of subtype B. Although the conservation among the different subtypes varied, the most conserved epitopes were also conserved globally (subtype A-J, Table 4). Thus, many of the identified epitopes are not subtype-specific.

### Sequence conservation of CTL epitopes compared with the CD8⁺ T cell response during HIV-1 infection

In order to design tailor-made therapeutic vaccine components for Danish patients, the sequence of the epitopes in the viral genome isolates from 11 A2⁺ HIV-1 subtype B-and -C infected Danish patients were compared to the patients' CD8⁺ T cell reactions *ex vivo* (Table 3). Four combinations of epitope sequence and CD8⁺ T cell reaction were observed. In some cases the epitope sequence in the patient isolate was identical to the test peptide and a CD8⁺ T cell reaction was readily observed to the test peptide (e.g. Gag₈₀ and patient 15 (+/+), Table 3). However, the presence of an epitope sequence 100 % identical to the test epitope peptide was not necessarily associated with a positive CTL recall response. Examples of this was seen even-though the epitope proved to be immunogenic in A2tg mice and recognised by other A2⁺ patients and thus assumed to be processed (e.g. Gag₃₆₂ (9V) and patients 16, 18, 21, 30, 35, 41, 43 (+/-), Table 1). This may represent the phenomenon described as subdominance (Yewdell & Del Val, 2004). A lack of an identical epitope in a patient HIV-1 isolate can explain a lack of CD8⁺ T cell recognition in the patient (e.g. Vif₁₄₉ and patients 15, 17, 18, 21, 23, 30, 35, 41, 43 (-/-), Table 3). However, in other cases a CD8⁺ T cell reaction was observed towards the test peptide even though the epitope sequence in the patient HIV-1 isolate was not 100 % identical (e.g. Gag₄₃₃ and patient 16 and 43 (-/+), Table 3).

**Table 4: Conservation of HIV-1 HLA-A2-binding target epitopes (28 epitopes and 10 variants).**

| | | % Conservation | | |
|---|---|---|---|---|
| | | In Danish isolates* | Intra-subtype# | |
| Epitope | | | B | A-J |
| Gag 150 | | 100 (11) | 100 | 95 |
| Gag 345 | | 100 (11) | 93 | 93 |
| Gag 341 | | 100 (11) | 93 | 69 |
| Gag 433 | | 82 (9) | 90 | 82 |
| Gag 242 | | 73 (8) | 76 | 38 |
| Gag 362(9V) | | 73 (8) | 63 | 31 |
| Gag 80 | | 55 (6) | 52 | 58 |
| | *Gag 362(9A)var* | 18 (2) | 13 | 33 |
| Env 35 | | 100 (11) | 98 | 88 |
| Env 67(2V) | | 91 (10) | 90 | 75 |
| | *Env 67(2I)var* | 0 (0) | 4 | 12 |
| Env 681 | | 64 (7) | 68 | 59 |
| Env 565 | | 55 (6) | 82 | 47 |
| Env 529 | | 27 (3) | 23 | 52 |
| Pol 687 | | 91 (10) | 100 | 20 |
| Pol 571 | | 91 (10) | 87 | 92 |
| Pol 59 | | 82 (9) | 82 | 80 |
| Pol 606 | | 64 (7) | 76 | 54 |
| Nef 141 | | 82 (9) | 86 | 69 |
| Vpu 66 | | 45 (5) | 0 | 7 |
| Vpu 13 | | 9 (1) | 20 | 7 |
| | *Vif 158(2T)var* | 36 (4) | 10 | 13 |
| Vif 158(2I) | | 18 (2) | 50 | 31 |
| Vif 23(9I) | | 27 (3) | 48 | 33 |
| | *Vif 23(9V)var* | 9 (1) | 13 | 29 |
| Vif 149 | | 9 (1) | 24 | 9 |
| Vif 101 | | 0 (0) | 17 | 8 |
| | *Vif 101 (9M)var* | *9(1)* | *4* | 9 |
| | *Vif 101(9I)var* | *9 (1)* | *1* | 9 |
| | *Vif 101(1S)var* | *9 (1)* | *1* | 0 |
| | *Vif 101(1D)var* | *27 (3)* | *31* | 24 |
| | *Vif 101(1N)var* | *18 (2)* | *17* | 44 |
| | *Vif 101(1E)var* | *9 (1)* | *2* | 1 |
| Vpr 41 | | 9 (1) | 20 | 10 |
| Vpr 34 | | 0 (0) | 4 | 26 |
| Rev 102 | | 9 (1) | 25 | 5 |
| Rev 96 | | 0 (0) | 5 | 1 |
| Rev 73 | | 0 (0) | 3 | 17 |

| | | | | |
|---|---|---|---|---|
| * The epitopes are ranked in order of decreasing conservation in % and numbers of the 11 Danish subtype B HIV-1 isolates from HLA-A2⁺ patients. # Data compiled from HIV-1 sequences in the www.lanl.hiv.gov database (2005). | | | | |

### Sequence alignment of HIV-1 CTL epitopes from patient HIV-1 isolates

To study the sequence variation of the CTL epitopes in the Danish clinical HIV-1 isolates in more details, an alignment was made of the epitope amino acid sequences with the sequences found in the patient HIV-1 isolates (Figure 1). A CTL recall response *ex vivo* is indicated (shaded). The most frequently targeted epitopes were Gag₇₇, Gag₄₃₃, Pol₄₇₆, Env₁₂₁ and Env₅₂₉ of which Gag₇₇, Pol₄₇₆ and Env₁₂₁ are well described immunodominant epitopes (www.lanl.hiv.gov). We found considerable sequence variation in the TCR binding amino acids (positions 3-8) of the most targeted epitopes Gag₇₇, Env₁₂₁ and Env₅₂₉. The majority of our newly identified epitopes were infrequently targeted. In the cases where a patient responded to a test epitope, but a variant of the epitope was found in the patient's virus isolate, the amino acid substitutions involved in some cases the primary anchor-binding positions 2 and 9 (e.g. Gag₃₆₂ (9A)^{var} and (9V) in patient 23, Vif₂₃ 9I and 9V in patient 43, Env₆₇ 2I and 2V in patient 23, respectively). However, several alternative amino acids are known to serve as anchor binding residues for HLA-A2 such as L, M, I or Q in position 2 and L, I, A or V in the C-terminal position, respectively (Berzofsky *et al.,* 1999). Thus, these amino acid substitutions may not necessarily impair epitope HLA-A2 binding negatively. Hence, T-cell cross-reaction may still be possible. When testing the patient CD8⁺ T-cell reaction against both the parent and the variant epitope peptides, a positive reaction could in some cases be measured to both epitopes (e.g. patient 23 and Gag₃₆₂ (9V) and (9A)^{var}, and patient 43 and Vif₂₃ (9V) and (9I), Figure 1). Reaction to both variants was not readily detected in all cases (e.g. Vif₁₅₈ 2I and 2T for patient 30, and Env₆₇ 2I and 2V for patient 23, respectively (Figure 1).

### Cross-reacting variant epitopes in patients and HLA-A2tg mice

The A2tg mouse model was used to test possible cross-reactions to the observed CTL epitope variants. Cross-reaction between Gag₃₆₂ (9V) and (9A)^{var} and between Vif₂₃ (9V) and (9I) have previously been found in the A2tg mouse model (Corbet et al 2003). This confirms the suggested cross-recognition observed in patient 23 to these epitopes (Figure 1, B). None of the patients that reacted with the vaccine immunogen Vif₁₀₁ (9L) contained detectable HIV-1 isolates with an identical epitope sequence (Figure 1). However, immunising A2tg mice with the Vif₁₀₁(9L) induced CTL to the Vif₁₀₁(9L) that cross-reacted with the variants Vif₁₀₁ 9M, 9I, 1D, 1S, and 1N as measured by ELISPOT and cytotoxicity assays whereas 1E only responded weakly in IFNγ ELISPOT and (1H,9Q) did not cross-react, respectively (Table 5 and Figure 3). The same cross-reactions were observed for Vif₁₀₁(9L) and (1N) in patient 16, and Vif₁₀₁(9L) and (1D) in patient 23, respectively (Figure 1). This demonstrate that cross-reacting epitope variants explain some of the -/+ situations observed in the patients, e.g. in Gag₃₆₂(9A) for patient 23 and 18, in Vif₁₀₁(9L) for patient 16, 23, and in Vif₁₀₁(9M) for patient 41, in Vif₂₃(9V) for patient 43, and in Env₆₇(2I) for patient 23, respectively (Table 3). This cross-reaction induced by Vif₁₀₁, (9L) is relevant for design of a tailor-made vaccine for Danish patients since none of the 11 patients contained the sequence of the Vif₁₀₁(9L) vaccine immunogen in their isolate sequences. However, 8 of the 11 patient isolates had one of these cross-reactive Vif₁₀₁ target antigens (Table 4). We also demonstrated a cross-reaction induced by Vif101(9L) to Vif₁₀₁(9M) that none of the Danish clade B isolates contained; however, Vif₁₀₁(9M) is common among clade C (70%), clade D (35%) and clade H (33%) in the databases of strains outside Denmark, respectively (Corbet *et al.,* 2003). Vif₁₅₈ (2I) was immunogenic in A2tg mice and recognised by patient Pt30 whose virus had the Vif₁₅₈ (2T) variant. However, Vif₁₅₈ (2T) was not immunogenic in the A2tg mice (Table 3) and was not targeted by any of the patients studied (Table 3) and may represent a non-immunogenic or non-binding escape mutant. Env₆₇ (2I)^{var} and (2V) on the other hand were both immunogenic in A2tg mice and immunization of A2tg mice with Env67 (2V) was previously shown to cross-react to Env67(2i) (Corbet *et al.,* 2003). In the example in Figure 4 and 5 we show that the reverse is also true. Immunizing A2tg mice with the optimized Env67(2I) immunogen induces cross-reaction to the natural and more conserved Env67(2V). In other cases the amino acid substitutions were located in positions important for TCR recognition (e.g. position 3-8). In some cases, up to 3 mutations were found in the variant epitope in the patient HIV-1 isolate (e.g. Rev₁₀₂, Vpr₃₄, Vpu₁₃, Vif101(1H,9Q) in patient 41, Figure 1). Thus, the CD8⁺ T cell recall response observed in patients to the parent test epitope could not be induced by these epitopes that may instead represent escape mutated epitopes.

The infrequently targeted novel epitopes present in Danish patient HIV-1 isolates may not necessarily serve as optimal immunogens. Thus, for vaccine purposes it is of special importance to optimize these vaccine immunogens to raise immunity to the poorly immunogenic but relatively well conserved or cross-reacting target epitopes. To increase the affinity for HLA-A2, variant epitopes may be chosen with more favourable amino acids in the second and the C-terminal position. By this procedure, optimized immunogens were identified that generated CD8⁺ T cells in A2tg mice cross-reacting to the corresponding subdominant target epitope. Thus, immunizing A2tg mice with the very immunogenic but rare Vif₁₀₁ (9L) variant (Table 4) induced CTL against homologous Vif₁₀₁ (9L) that cross-reacted to Vif₁₀₁ (9M), (91), (1D), (1S), (1N) (Table 3). The same cross-reactive CTL immune induction was observed for other more immunogenic but less conserved variant epitopes, e.g. Gag₃₆₂(9V)^{var}, Vif₂₃(9V)^{var}, and Env₆₇(2I)^{var} (Figure 4 and 5).

**Table 5. Cross-reactivity in HLA-A2 tg mice of CTL raised by an epitope immunogen toward variant epitopes**

| | Immune response to target | | |
|---|---|---|---|
| | | CTL^{a} | IFN-γ ELISPOT |
| *Immunogen* | Target epitope | % lysis^{b} | sfu/10⁶cells^{b} |
| | | | |
| Vif₁₀₁ (9L) | Vif₁₀₁ (9L) | 33 ± 2 | 9,338 ± 1,422 |
| | Vif₁₀₁(9M)^{var} | 41 ± 1 | 10,458 ± 1,283 |
| | Vif₁₀₁(9I)^{var} | 40 ± 1 | 12,164 ± 541 |
| | Vif₁₀₁(1D)^{var} | 26 ± 1 | 1,409 ± 146 |
| | Vif₁₀₁(1S)^{var} | 30 ± 1 | 10,191 ± 647 |
| | Vif₁₀₁(1N)^{var} | 22 ± 0 | 5,595 ± 362 |
| | Vif₁₀₁(1E)^{var} | - | 3,013 ± 239 |

| | | | |
|---|---|---|---|
| a) lysis at effector to target cell ratio (E:T) of 50:1 b) mean ± standard deviation (triplicate values) of splenocyte pools from 3 mice. - : response below the threshold of positivity (<10 % lysis at E:T ratio 50:1) | | | |

### Discussion

In this study we have identified and characterized the sequence conservation and corresponding CTL immune recognition of newly described HLA-A2-binding HIV-1 CTL epitopes in Danish patients. We have shown that variants of new epitopes are targeted somewhat promiscuously. Using this information it may be possible to include new target epitopes to be covered by a vaccine and, importantly, select optimal epitope immunogens for therapeutic vaccines against several HIV-1 proteins. The 28 epitopes selected for this study were immunogenic in A2tg mice and/or had induced CD8⁺ T cell response during a natural HIV-1 infection (Corbet *et al.,* 2003). Of the 28 epitopes 18 were immunogenic in both A2tg mice and had induced a CD8⁺ T cell response in humans. Epitopes that induced CD8⁺ T cell responses in patients are considered processed, immunogenic and antigenic. In some cases, the tested epitope had raised a CD8⁺ T-cell recall response in A2⁺ HIV-1-infected individuals but a CTL response in the HLA-A2tg mouse model could not be measured (Gag₂₄₂, Gag₃₄₁, Pol₆₀₆, Env₅₂₉, Rev₁₀₂, Vpu₁₃). This phenomenon may demonstrate limitations in the mouse model (Wentworth *et al.,* 1996). Moreover, HLA-A allotyping found that 13 of the 17 HLA-A2 positive patients initially studied were HLA-A02011 of whom 6 were homozygote for HLA-A02011 (Corbet *et al.,* 2003). The remaining 4 patients carried one allele of the HLA-A0236, 0234 or 02201, respectively. Thus, none of the HLA-A2 positive patients shared genetic allotype with the humanized MHC-I molecule HLA-A0201 used in the A2tg mice which may also influence the results. Finally, the mouse model may not be sufficiently sensitive for detection of the poorly immunogenic epitope immunogens inducing responses in only a fraction of the mice.

Interestingly, for some of the epitopes *ex vivo* recall responses were induced also in non-A2 patients (e.g. Gag₂₄₂ and Pol₆₀₆, Table 3 and example 3). This could indicate that the epitopes are presented by non-A2 HLA alleles shared between the A2 and non-A2 patients or that the epitopes promiscuously bind other HLA alleles beside HLA-A2. In A2 and non-A2 patients responding to the Gag₂₄₂ and Pol₆₀₆ epitopes there were no shared HLA-A or HLA-B alleles (the HLA-C allele was not sequenced) or shared alleles belonging to the same MHC-I supertype family. Therefore it seems likely that Gag₂₄₂ and Pol₆₀₆ promiscuously bind other HLA alleles in addition to HLA-A2. This will broaden the use of the proposed HIV-1 vaccine to include also non-A2 patients. In addition a few epitopes were positive in A2tg mice and non-A2 patients but not in any of the A2 patients included (e.g. Vpu₆₆, Vpr₄₁, Table 3). Of these the Vpu₆₆ epitope was relatively well conserved (in 5/11 Danish patient isolates) compared to HIV-1 sequences in the Los Alamos database (www.lanl.hiv.gov). The numbers of patients included are however limited and may be biased since only HLA-A2 patient isolates were sequenced. Likewise, the HIV database may be biased by the different project sequences entered which may not precisely represent circulating clinical isolates. The Vpu₆₆ epitope was very low immunogenic (Table 3); however, it could serve as an A2-binding target epitope since immunisation with an anchor-optimized modified epitope Vpu₆₆(9V)^{mod} have been shown to induce a very strong immune response cross-reacting to the Vpu₆₆ (Corbet *et al.,* 2003). In contrast, the Vpr₄₁ was probably too rare (only 1 of 11 patient isolates, Figure 1 and Table 4) to find a positive response with the 11 A2 patients studied. Vpr41 was, however, immunogenic in A2tg mice immunised with peptide in Freunds adjuvants and binds to HLA-A2 MHC-I in vitro and is thus considered a CD8+ T cell epitope.

In general, the conservations among the Danish patients followed that of the subtype B isolates, which is not surprising since all the Danish isolates were of subtype B. Although the conservation among the different subtypes varied, the most conserved epitopes were also conserved globally. Thus, many of the identified epitopes seem not restricted to a single HIV-1 subtype, which broadens the geographic area where a vaccine targeting such epitopes could be useful. In fact, the majority (16/28) of these HIV-1 CTL epitopes were present in more than half of the Danish patient isolates and were also highly conserved globally (>45 %, Table 4) or within certain non-B subtypes (Corbet *et al.,* 2003). This may be a consequence of the method of the initial epitope identification and selection by "reverse immunology" via Artificial Neural Network prediction and selection for intermediate *in vitro* MHC-I bindings, respectively. This method identifies primarily immunological subdominant epitopes (Corbet *et al.,* 2003, Sette *et al.,* 1994, Buus *et al.,* 2003) that, however, seem more conserved perhaps as a consequence of their relative immune silence. A few target epitopes in variable HIV-1 proteins were less conserved in the 11 patient isolates and several variants were identified e.g. Vif₁₀₁(9L, 9M, 9 I, 1S, 1D, 1N) and Env₆₇ (2V, 2I). However, as a consequence of the demonstrated CTL cross-reaction to several epitope variants induced by the very immunogenic but infrequent Vif₁₀₁(9L) and Env₆₇(2I) the coverage was in fact as high as 8 and 10 of the 11 HIV-1 isolates, respectively (Table 4).

In some cases the sequence of the epitope in one of the patient isolates was identical to the epitope antigen and a CD8⁺ T-cell reaction was observed (e.g. Gag₈₀ and patient 15, Table 3). This confirms that the epitope is present in the patient isolate, is processed, antigenic and immunogenic *in vivo* and thus indicates a suitable vaccine target for other HIV-1 infected individuals. It is even possible that boosting of a pre-existing but weak CTL response may be beneficial. If a patient's HIV-1 isolate did not contain an epitope identical to the test epitope peptide a lack of CD8⁺ T cell recognition could be expected (e.g. Vif₁₄₉ and patients 17, 18, 15, 21, 23, 30, 35 or Env₈₁₃ and all patients, Table 3). Such epitopes seem not to be a suitable vaccine targets. The presence of an epitope sequence 100 % identical to the test epitope antigen was not always associated with a positive CTL recall response in the patients, even though it was immunogenic in A2tg mice and recognised by other A2 patients and thus assumed to be processed (e.g. Pol₆₀₆ and patients 18, 21, 41, 43, 33). Interestingly, each patient reacted to only a few of the potentially immunogenic target epitopes present and each patient showed a different pattern. This phenomenon may be explained as subdominance (Yewdell *et al.,* 1999, Yewdell & Del Val, 2004). Chronically infected HIV-1 patients on antiretroviral therapy have been reported to react to an average of 4 epitopes and in some patients a single epitope may account for >90 % of responses (Goulder *et al.,* 2001). However, in animal models of acute infections as many as 70 epitopes may be targeted (Yewdell & Del Val, 2004). Cell mediated responses to a few immunodominant epitopes have been reported in HIV-1 patients and have led to the inclusion of these epitopes in experimental vaccines as vaccine candidates (Hanke & McMichael, 2000). However, immune escape may eliminate such epitopes and the protective role of such epitopes in infection and in vaccines remains to be seen. For unknown reasons CD8⁺ T cell responses to some immunodominant determinants exert poor antiviral activity and compromise the effectiveness of the CD8⁺ T-cell response. CD8⁺ T cells responding to different epitopes show gross differences in their capacity to reduce viral replication and new effective HIV-1 targets need to be identified and targeted (Yewdell *et al.,* 2004). In this respect it is interesting that subdominant epitopes have shown to be as potent as immune dominant epitopes in inducing protective anti-viral immunity (Gegin & Lehmann-Grube 1992; Rodriquez *et al.,* 2001; van der Most *et al.,* 1997) and may better control HIV replication (Frahm et al 2006).

The demonstrated cross-reactions and TCR promiscuity could explain several of the situations where the epitope sequence in the clinical HIV-1 isolate differed from epitope antigen but where a positive CTL reaction to the epitope antigen was measured. The TCR is known to be somewhat promiscuous with respect to recognition of epitope peptides bound to HLA (McKinney *et al.,* 2004). TCR binding to variant epitopes are relatively unaffected by amino acid substitutions at TCR contact residues or for epitopes with conservative amino acid substitutions provided that binding to MHC-I is not negatively influenced. A hierarchy exists for amino acids within an epitope known as primary and secondary TCR contact residues (Evavold *et al.,* 1993). Promiscuity of TCR has been reported to be very high and estimates of up to 10⁶ different peptides binding to the same TCR have been reported (Gavin *et al.,* 1994; Jacobsen *et al.,* 2001; Mason *et al.,* 1998). The flexibility of HIV-1 epitope recognition by TCR has been demonstrated as CTL recognition of related but slightly variable epitopes by cloned CTL lines developed from HIV-1 infected individuals (Coullin et al., 1995; Tomiyama *et al.,* 2000; Buseyne *et al*.,2001, Brander *et al*., 1998) or by using HLA-A2tg and HLA-A11tg mice (McKinney *et al.,* 2004). This flexibility is useful for designing vaccines covering multiple HIV-1 subtypes or different clinical isolates within a cohort of patients. Thus, cross-reactive epitopes may be regarded as "conserved" epitope targets. However, cross-reaction could not be demonstrated in all such situations. This could be due to differences in test sensitivity mainly caused by differences in epitope antigen binding to the various HLA-A2 genotypes, or patients HLA-A2 heterozygocity to an epitope peptide in fact having also a non-A2 restriction. Finally, simple failure of the CTL to recognise the variant epitope as in escape mutations may in some cases explain the variant sequence found. For example, patient 30 reacted against the Vif₁₅₈ (2I) but the patient's isolate had the Vif₁₅₈ (2T) sequence as did 6/11 patients. This Vif₁₅₈ (2T) variant was unable to serve as an A2 epitope immunogen and induce CTL in A2tg mice (Table 3). Also several mutations within the TCR recognition amino acids may represent escape mutants e.g. Gag₇₇ (Iversen *et al.,* 2006).

As many HIV-1 proteins as possible should be targeted by vaccination to meet the high diversity of HIV-1 among patient isolates and prevent the emergence of viral escape mutants. An epitope based vaccine is anticipated in an attempt to force the immune system to respond to the selected epitopes. We have identified suitable poorly immunogenic but relatively conserved target epitopes and related variants in most HIV-1 proteins in Danish clinical isolates. To target determinants for a therapeutic vaccine optimal immunogens may be selected based on criteria of conservation, epitope antigenicity *in vivo* and immunogenicity as measured in A2tg mice. Thus, within Gag five epitope peptides were immunogenic in A2tg mice and antigenic in A2 patients. However, although Gag₃₄₅ was 100 % conserved among the 11 DK patients, none had a CD8⁺ T-cell recall response to it. This could be due to lack of processing and/or presentation and it would not serve as a suitable target. The most conserved were Gag₁₅₀ and Gag₄₃₃, respectively. Moreover these two epitopes can be anchor-optimized (Gag₁₅₀(2L)^{mod} and Gag₄₃₃(9V)^{mod}) resulting in very strong immunogens that induced cross-reaction to the naturally occurring epitopes (Corbet *et al.,* 2003). Within Pol most epitopes were all relatively well conserved but only Pol₆₀₆ and Pol₆₈₇ had been targeted by A2 patients and thus had demonstrated processing *in vivo* (Table 3). Both were poorly immunogenic in A2 mice but Pol₆₀₆ has been anchor-optimized resulting in high immunogenicity with induction of cross-reaction to the natural epitope (Corbet *et al.,* 2003). Within Env the most conserved epitopes that were immunogenic in A2tg mice and antigenic in man were Env₆₇ and Env₆₈₁ (10/11 and 7/11, respectively). The most conserved Env₆₇(2V) was also the less targeted *in vivo* with low immunogenicity, which was, however, improved by the rare but more immunogenic epitope variant Env₆₇ (2I)^{var} , which had induced immunity to both variants (Corbet *et al.,* 2003). Within Rev none of the epitopes were sufficiently conserved among the patients to serve as vaccine targets. Rev₇₃ and Rev₉₆ were both absent and Rev₁₀₂ was only present in 1 of 11 HIV-1 strains and was not immunogenic in A2tg mice. Within Vif the epitopes were all present in the patient strains but with several variants. In case of the most immunogenic Vif₂₃ (2V)^{var} (Table 3), a cross-reaction could be observed to cover the two variants examined Vif₂₃ (2V) and Vif₂₃ (9I). The most immunogenic but less conserved Vif₁₀₁ (9 L) variant induced cross-reaction to 6 low immunogenic Vif₁₀₁ variants and in this way it was able to cover 8 of the 11 patient strains. The same reaction to variants could not be obtained against the most frequent Vif₁₅₈(2T) variant that may represent a non-immunogenic escape epitope. Cross-reaction with the low conserved Vif₁₄₉ was not examined. Within Vpu the Vpu₆₆ was the most conserved among the two Vpu epitopes identified and its poor immunogenicity has previously been improved by the anchor-optimization as Vpu₆₆ (9V)^{mod} (Corbet *et al.,* 2003). Within Nef the only epitope studied (Nef₁₄₁) was very conserved (9/11), targeted *in vivo,* and immunogenic.

In conclusion, sequence conservation of the identified HLA-A2-binding HIV-1 CTL epitopes was high among Danish patient isolates as in HIV-1 sequences from the HIV Databases. In addition, cross-reacting variants resulted in a broad coverage of the target antigens. Optimal target epitopes could be identified in Gag, Pol, Env, Vif, Vpr, Vpu, and Nef based on this sequence conservation and measured patient CTL immune recognition. To target these epitopes it is proposed to test the corresponding optimal immunogens either identified among natural rare epitopes shown here or modified artificially anchor-optimized epitopes for tailor-made therapeutic vaccinations of a defined group of patients.

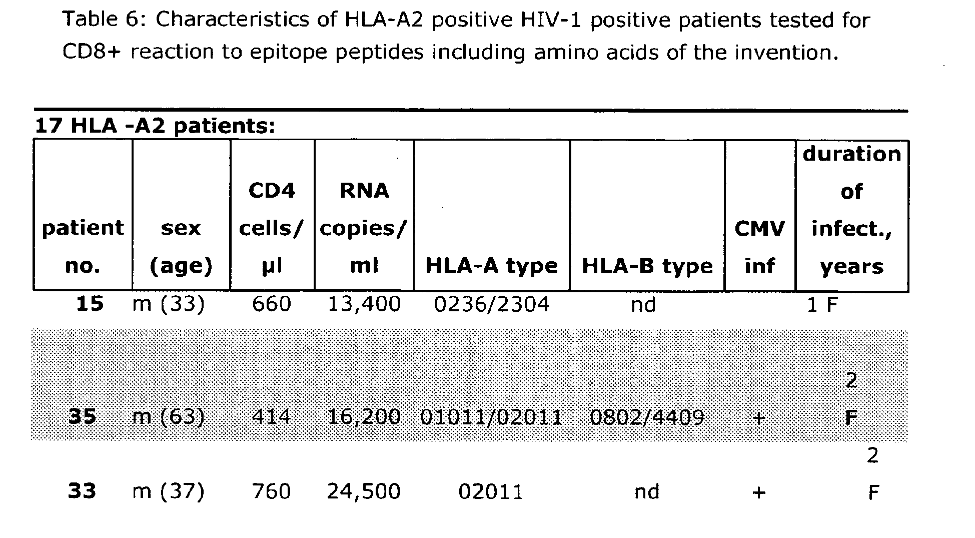

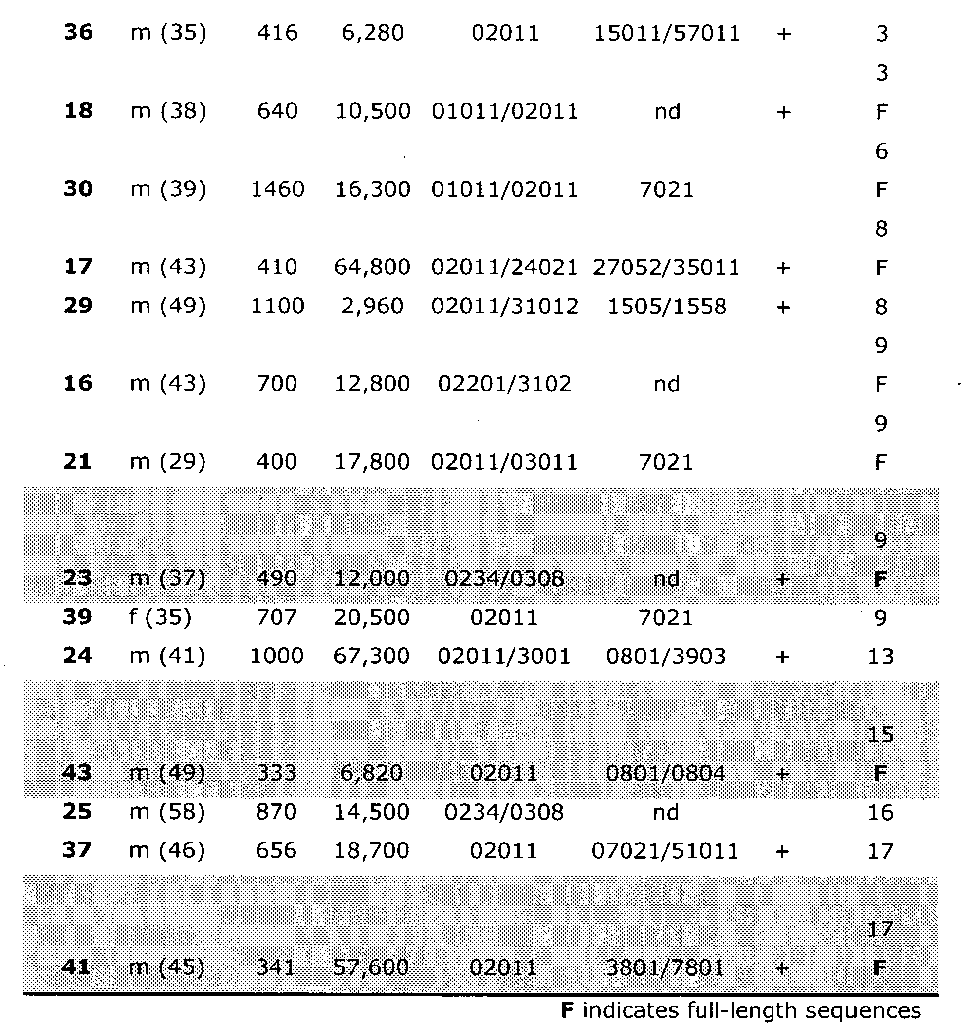

### HIV-1 strains

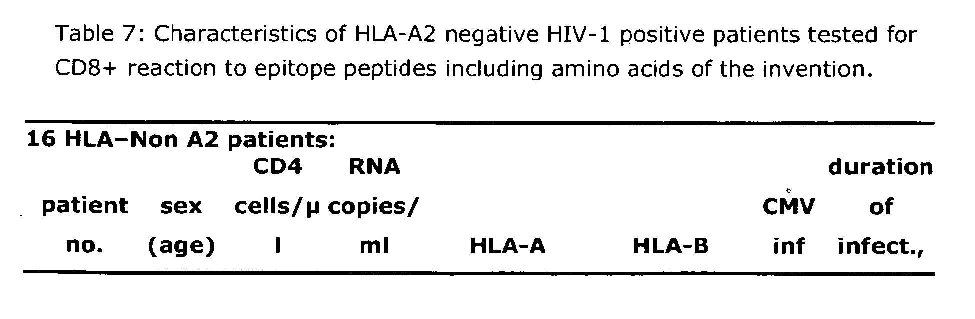

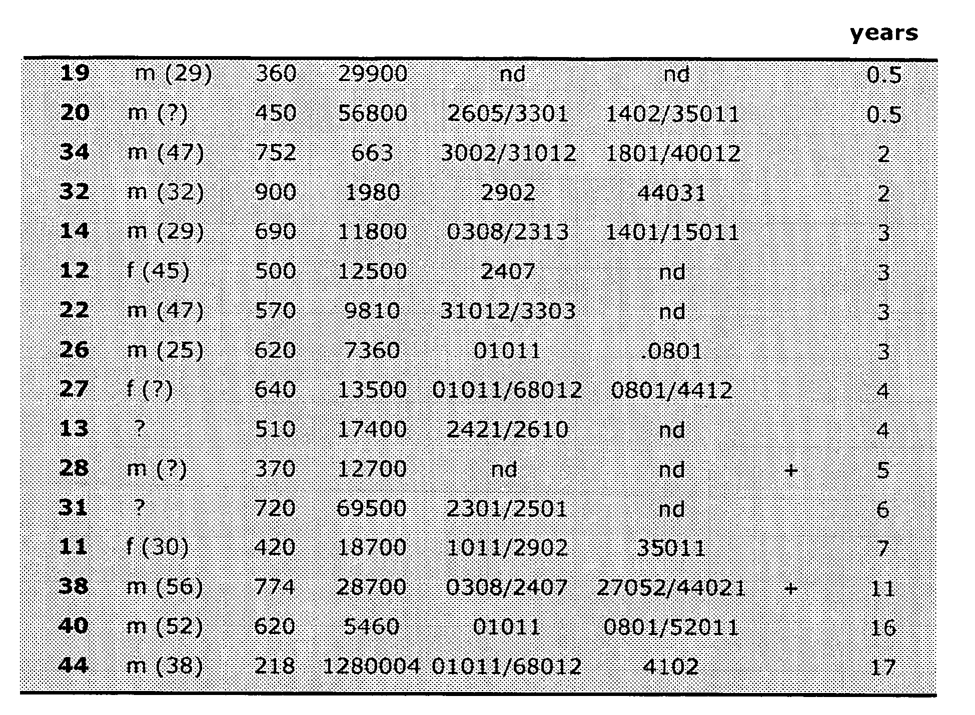

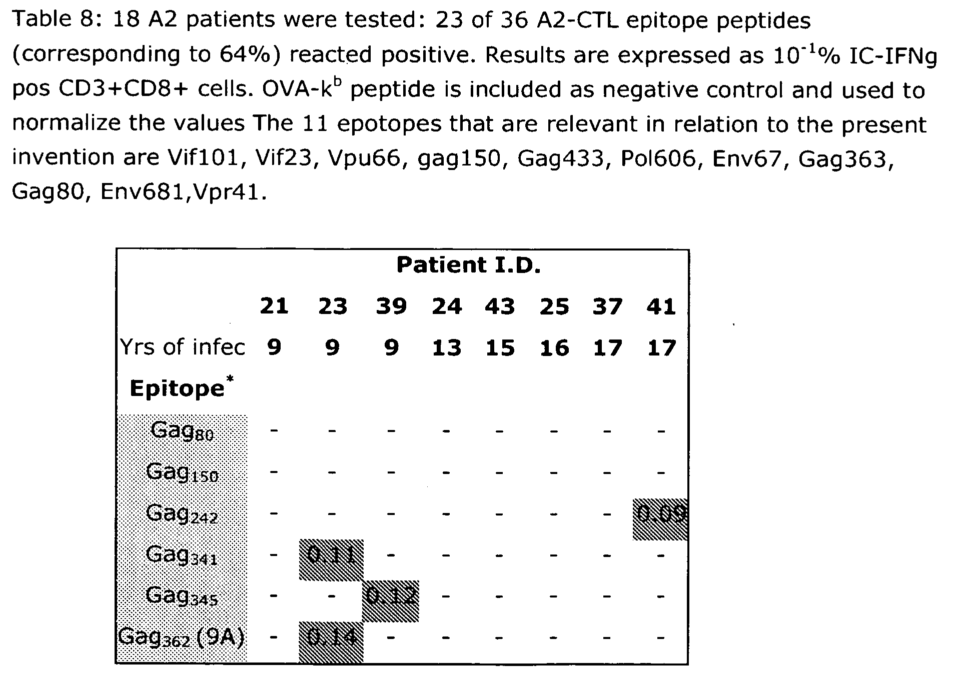

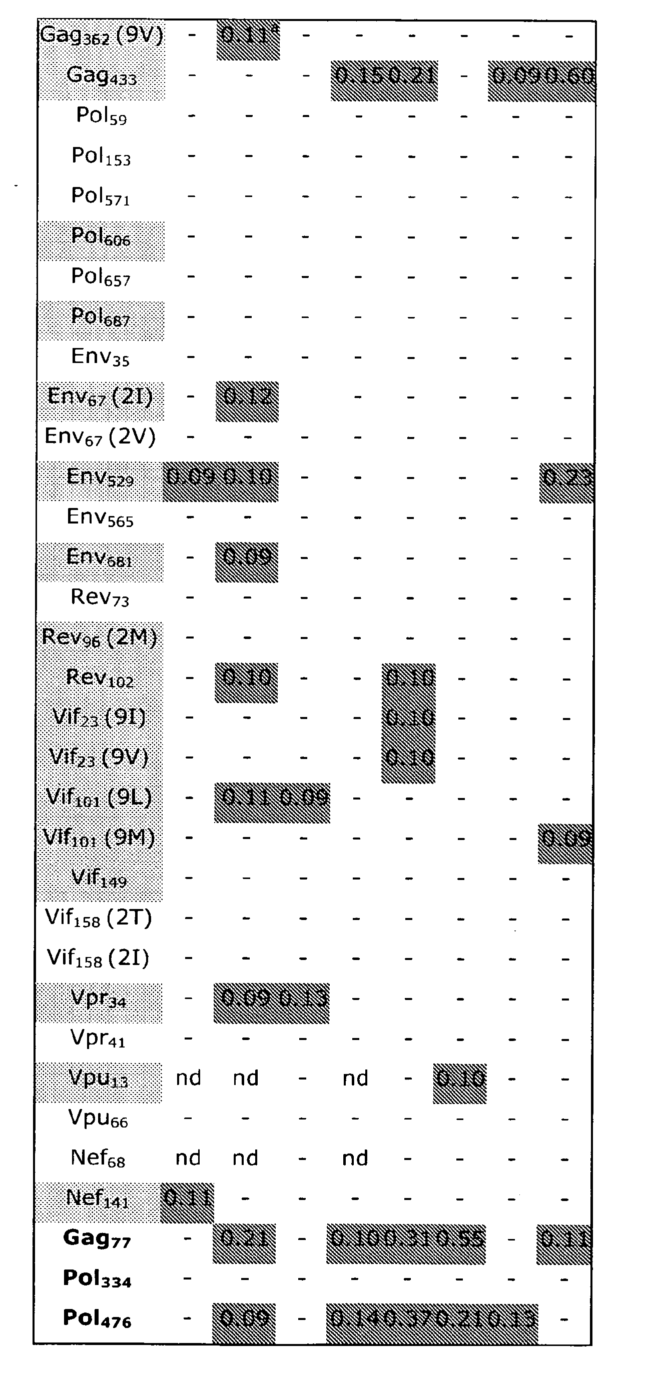

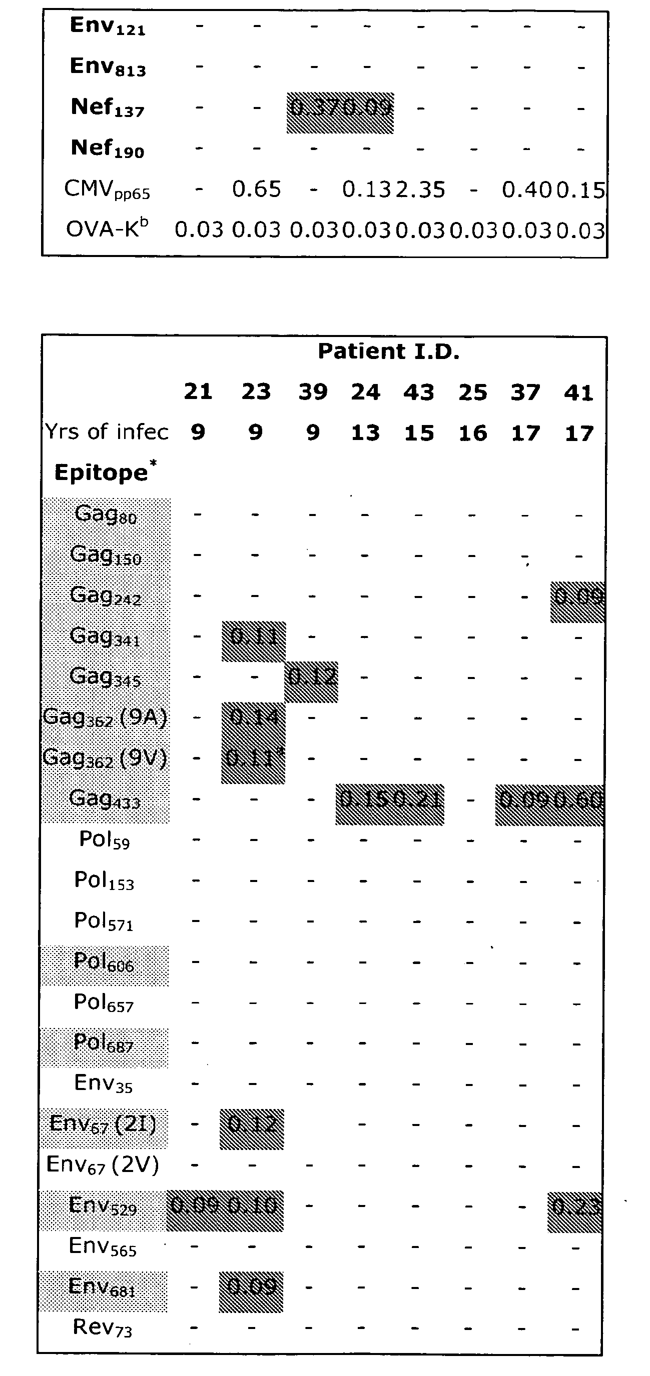

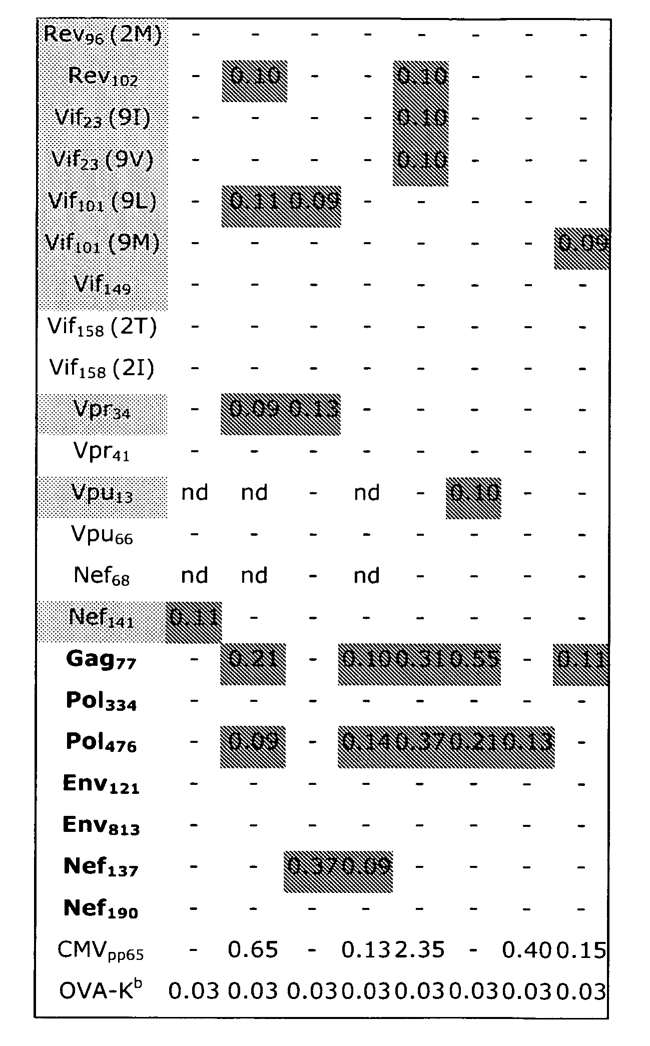

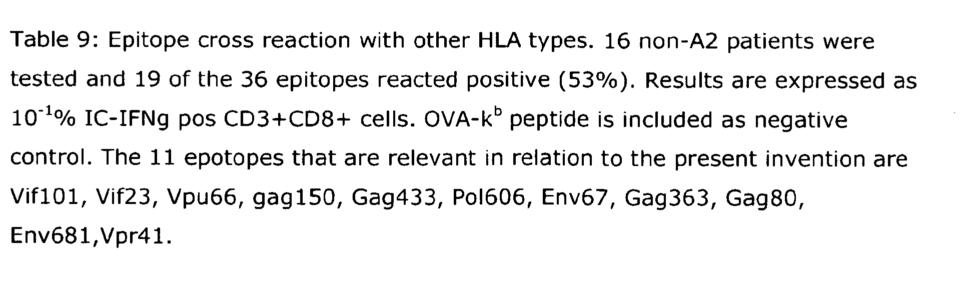

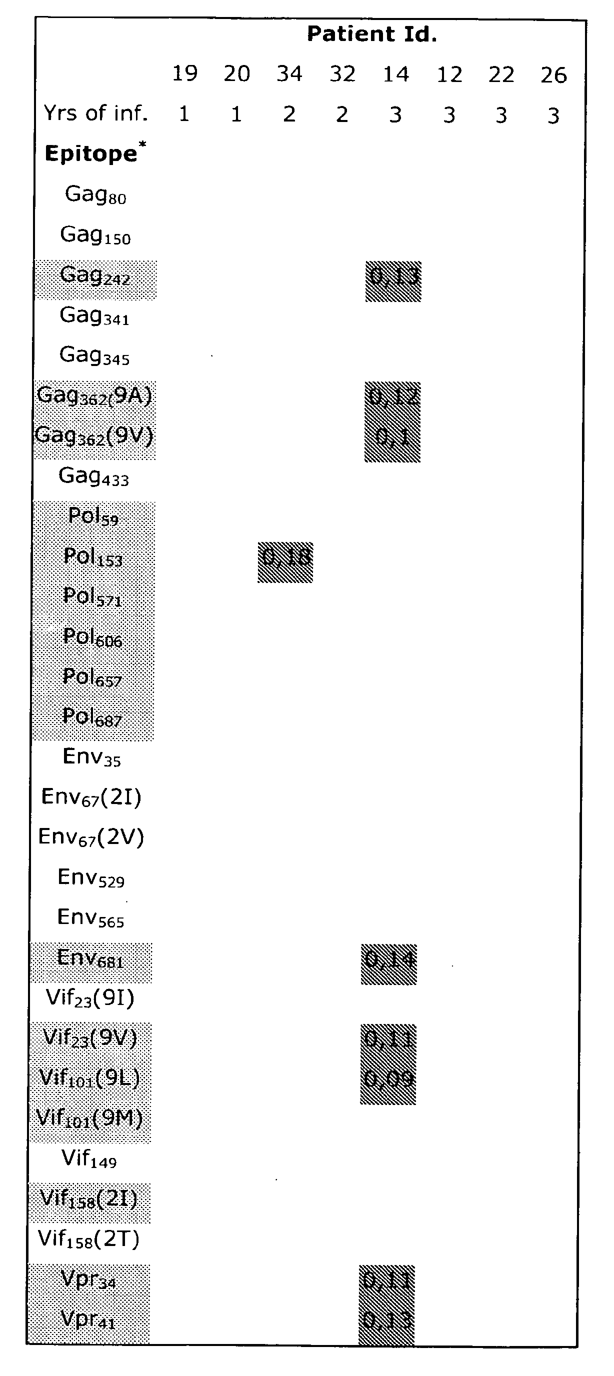

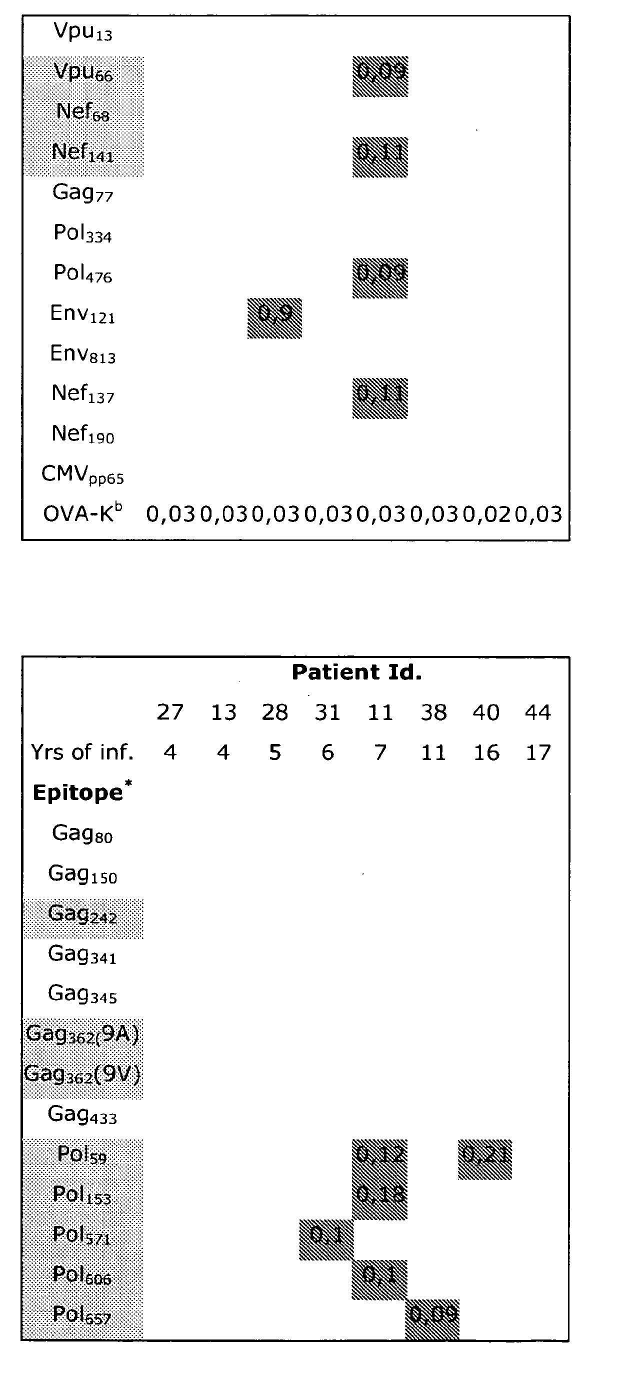

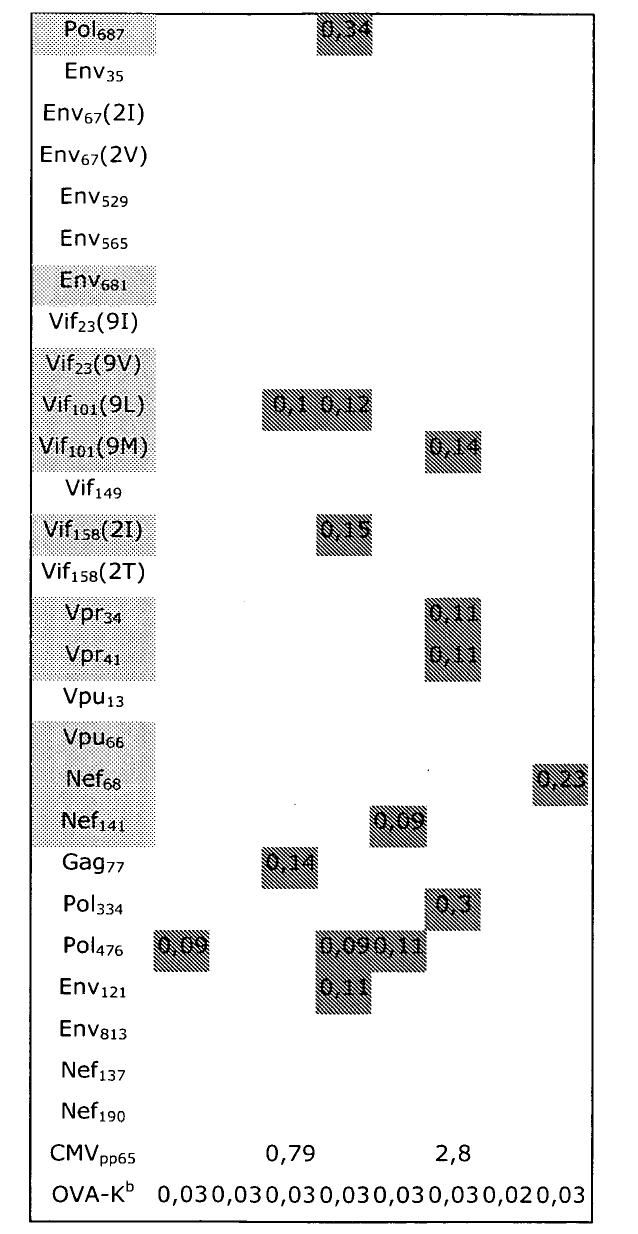

### Example 2

CD8⁺ T-lymphocyte reactivity to novel epitopes in HIV-positive patients.

### Materials and methods

### Patients and viral isolates

Blood samples were collected from HIV-1 positive patients at the University Hospital of Copenhagen and Hvidovre according to the following criteria: CD4⁺ cell count > 300/µl and measurable viral load (>1,000 RNA copies/ml) without receiving highly active antiretroviral therapy (HAART). HLA-A and HLA-B genotyping of the patients was performed on DNA purified from peripheral blood mononuclear cells (PBMC) using either HLA-A or HLA-B Sequencing-Based Typing Kit (PE Biosystems) according to instructions by the manufacturer. MatchTool/MatchMaker and MT Navigator software (PE Biosystems) were used for allele identification. Intracellular interferon gamma reaction in CD8⁺CD3⁺ T-lymphocytes (IC-IFNγ) to 43 peptides was measured by flowcytometry. Cells were stimulated with 2 µg peptide, as described (Kern *et al.,* 1998), except that 400 µl of full heparinized blood was used instead of purified PBMCs. Blood was treated with red blood cell lysing solution and lymphocyte permeabilization solution (Becton Dickinson Immunocytometry System) according to the manufacturer's instructions. Cells were stained with appropriate antibodies for 30 minutes at room temperature and fixed in 2 % paraformaldehyde. Mouse anti-human CD28, anti-human CD49d, FITC-conjugated anti-human IFN-γ, PE-conjugated anti-human CD3, PerCP-conjugated anti-human CD8 and mouse immunoglobulin G1 and G2a isotype controls were obtained from Becton Dickinson. Five-parameter flowcytometric analysis was performed on a FASCAN (Becton Dickinson) using 150,000 to 400,000 events per analysis. CD3⁺ CD8⁺ cells were examined for IC-IFN-γ and data analysed using CellQuest software. Mouse OVA-K^{b} epitope peptide (SIINFEKL) stimulated human cells were used to set the cell populations boundaries for exclusion of 99.97 % of the control lymphocytes. Positive reactions were recorded if the percentage of positive cells were > 3 times the value of the negative OVA-K^{b} control. HLA-A and B of patients were typed using a genotypic sequencing kit (Applied Biosystems) according to the manufacture's protocol. The patients gave informed consent to the study that was approved by the Ethical committee of the Copenhagen hospitals.

### Results

We examined specific CD8⁺ T-cell reactivity to the 36 new natural HLA-A2 HIV-1 epitope peptides with confirmed MHC-I binding (IC₅₀ < 500 nM) in immune competent HIV-1 infected patients controlling their virus without the need for anti-viral therapy. Five of the patients (nos. 24, 25, 37, 41, and 43) were infected for more than 13 years and.may be classified as long-term non-progressors. Among the 17 HLA-A2+ patients, specific CD8⁺ T-cell reactivity could be demonstrated against 21 of the 36 new natural epitopes. For comparison, specific CD8⁺ T-cell reactivity could be demonstrated against 5 of 7 known natural epitopes. Each HLA-A2 positive patient recognised at least one of the new HIV-1 epitope. An average of 4 HIV-1 epitopes were recognised per patient. One patient (no. 21), who did not react to any of the 7 known epitopes, reacted to two of the new CTL epitopes. 18 of the 28 epitopes that were immunogenic in mice were also immunogenic in natural infections, as evidenced by the 17 HLA-A2 and 16 non-HLA-A2 patients examined. Thus, a total of 28 new HIV-1 CTL epitopes were immunogenic in HLA-A2 mice or humans.

In conclusion: Several of the examined putative HLA-A2 binding CTL epitopes were recognised by HIV infected patients during their HIV infection. In particular all the epitopes relevant for the present application were recognised by recall CD8 T cell responses as measured by IC-FACS in HLA-A2 and/or non-A2 patients. This demonstrates their antigenicity and processing of these target epitopes. Also the target epitopes are immunogenic in HLA-A2tg mice. Some of these could be immunogically optimized and induce higher immune reaction and cross-reaction with these new target epitopes.

### Example 3

### Studies on variant peptide epitopes Vif₁₀₁ and Env₆₇(2I)

### Methods

To study cross-reactivity (TCR promiscuity) to several variant peptide epitopes of the naturally anchor-optimized Vif₁₀₁(9L) variant epitope-specific CTLs and the Env₆₇(2I) variant epitope-specific CTLs, HLA-A2 tg HHD mice kindly provided by F.A. Lemonnier were used (Pascolo *et al.,* 1997). HHD mice are H-2D^{b-/-} and β2m^{-/-} double knockout mice. Isolated bone marrow cells from HHD transgenic mice were kept overnight in culture medium (RPMI 1640 with 10 % FCS, penicillin, streptomycin, 2-mercapta-ethanol). The next day, the non-adhering cells were isolated and cultured with GM-CSF, 10 ng/ml, and IL-4, 20 ng/ml. At day 3, fresh culture medium with GM-CSF and IL-4 were supplied. At day 6, the cells were harvested and cultured overnight with GM-CSF, IL-4 and LPS, 1 µg/ml. At day 7, mature dendritic cells were harvested and used for vaccination. HHD transgenic mice, 8-12 weeks old, were injected s.c. with syngenic mature dendritic cells pulsed with the test peptide and the T-helper peptide. At day 11 after vaccination, the mice were sacrificed and the primed splenocytes were pooled and re-stimulated with the vaccination-peptide. Briefly, the splenocytes (100 x 10⁶/ml) were loaded with the vaccination-peptide, 100 µg/ml, for 2 hours at 37 °C, 5 % CO₂ and at day 1 after re-stimulation, IL-2, 50 IU/ml, was added. After 5 days of re-stimulation, the CTLs were harvested and used for the Cr-release assay, or kept another 6 days in culture media with IL-2 and tested in the IFN-γ ELISPOT assay. Alternatively at day 5, the splenocytes were depleted for NK (CD49bDX5) and B (CD19) cells prior to use in Cr-release assay and IFN-γ ELISPOT assay.

Animal experiments were approved and done in accordance with the legal requirements in Denmark.

### Conclusion 1

As shown in figure 2, Vif101 specific splenocytes were able to recognize variants of the parental epitope. By immunizing HLA-A*0201 transgenic mice with Vif₁₀₁ pulsed dendritic cells it is possible to generate specific CTLs that were able to recognize the parent vaccine peptide Vif₁₀₁ and several of its natural variants. The CTLs generated did produce high levels of IFN-γ and were able to mediate lytic activity against the parent peptide and the following variants L9I, G1N, G1S and L9M which corresponds well with the *in vitro* binding values IC₅₀ = 219, 30, 77, 43, and 68 nM respectively. The variant G1D had a higher lytic activity compared to its IFN-γ production, but both where above the background. The variant G1E only produced IFN-γ and had no lytic activity above background. The variant G1HL9Q were low in IFN-γ production and lytic activity. These last 3 variants showed less or no recognition, which also corresponds with their high *in vitro* binding values IC₅₀ = 1946, 6594 and 1161 nM respectively.

### Conclusion 2

By immunizing HLA-A*0201 transgenic mice with Env67(2I) pulsed dendritic cells it was possible to generate specific CTLs that were able to recognize the parent vaccine peptide Env67(2I) and its natural variant I2V. The parent peptide gave rise to the highest level of IFN-γ production in the ELISPOT assay compared to the I2V variant. These data corresponded to their different *in vitro* binding IC₅₀ values with the parent peptide having the highest affinity for the HLA-A*0201 molecule. In the cytotoxic assay the CTLs were able to lyse target cells pulsed with the parent peptide and the 12V variant with equal percent specific lysis.

### Example 4

### Immunization of A2tq mice with peptidemix in lipovac adjuvants

### Mouse Immunisation

HHD-A2 mice (Pascolo et al., 1997), were breed at PANUM Institute, CPH, animal facility. Mice were maintained with food and water ad libitum and artificially lighted 12 hours per day.

### Peptide immunisation.

A group of five female 8-12 weeks-old HHD-A2 mice were immunised with all 11 peptides (11 HIV epitopes + PADRE) solubilized in Tris-HCL pH 7.5 and 50 µl Lipovac adjuvant. Mice were given two intra dermal (i.d.) immunisation (total 33 µg of each peptide) in the belly skin with 50 µl injections and compared with non-immunised animals. Mice were vaccinated only once and spleens were taken 10 days after the immunisation.

### ELISPOT assay

For ELISPOT assays, spleen cells, either directly or pre-stimulated with peptide for 5 days *in vitro,* were plated on 96-well multiscreen filter plates (Multiscreen MAIP S45 10, Millipore, MA, USA). For pre-stimulation cultures, spleen cells (2.5×10⁶ cells/ml) were mixed with 1/10 mitomycin-C treated peptide-pulsed or unpulsed stimulator cells (HHD-EL4S3⁻Rob; HLA-A2*0201/hβ2m-transfected EL4 target cells) (Pascolo et al., 1997). Cultures were grown at 37°C and 5% CO₂ in 24-well plates for 5 days in growth medium (RPMI₁₆₄₀; 10% FBS; antibiotics). Multiscreen filter plates were precoated overnight at 4°C with 10 µg/ml rat anti-mouse IFN-γ (clone R4-6A2; 551216; BD Pharmingen, CA, USA) in 0.1 M sodium bicarbonate buffer, pH 9.6, washed 3 times wash in wash-buffer (PBS pH 7.4 with 0.05% Tween-20) and blocked for >0.5 h with 150 µl PBS pH 7.4 with 5% BSA per well. Splenocytes were resuspended in growth medium with 5 IU/ml recombinant IL-2, placed in duplicates, and serially diluted (maximum of 500.000 cells per well). Cells were incubated overnight at 37°C and 5% CO₂ with or without 10 µg/ml of peptide. After culture, the plate was washed ten times in wash buffer and once in demineralised water and then incubated 2 h at room temperature or overnight at 4°C with 5 µg/ml biotinylated rat anti-mouse IFN-γ detection antibody (clone XMG1.2; 554410; BD Pharmingen, CA, USA) in 50 µl PBS pH 7.4 with 1% bovine serum albumin (BSA). After additional six washings the plates were incubated 2 h at room temperature with 50 µl alkaline phosphatase-conjugated avidin (D 0396, DAKO Cytomation, Denmark) diluted 1:1000 in PBS pH 7.4 with 1% BSA. Colored spots were developed by adding 50 µl of freshly made BCIP (5-bromo-4-chloro-3-indolylphosphate)-nitroblue tetrazolium solution (Bio-Rad, CA, USA) per well. Color development was stopped after approx. 15 minutes using demineralized water, plates was air-dried and spots were counted electronically using an AID ELISPOT reader.

The number of specific IFN-γ-secreting lymphocytes was calculated by subtracting the value of the negative control (no peptide) from the value of a specific peptide, and expressed as the number of spot-forming units (SFU) per 10⁶ input cells. Negative reactions of non-immunised animals to all HIV-epitopes served the cut-off values.

### Conclusion

By immunising groups of 5 HLA-A2tg mice with a mixture of 11 HIV-1 epitope immunogens plus PADRE in LipoVac adjuvants it was possible to generate CTLs specific for 10 of the 11 epitopes. The numbers of epitopes recognised varied for individual mice ranging from 0 to 8 of the 11 epitopes.

### Example 5

### Methods

To test whether the epitope peptides were immunogenic when administered using the Dendritic cell immunization technique a group of 3 mice were immunized with 10 mill. syngeneic dendritic cells each pulsed with a test peptide (10 µg/ml) with PADRE (5 µg/ml). 10 days post immunization mice were sacrificed and the pooled splenocytes were pulsed with the vaccination peptide (100 µg/ml) at 100 mill/ml and expanded *in vitro* for 5 days receiving 50 iU/ml IL-2 at day 1. At day 5 splenocytes were tested in ELISPOT wells for IFN-γ production as measured as spot forming units (sfu) per 10⁶ splenocytes against the test peptide or an irrelevant mock peptide (the mock peptide Gag77 (SLYNTVATL) is not shown, but subtracted 44 sfu/10⁶ splenocytes).

At day 5 splenocytes were also tested in a cytotoxicity assay (% specific lysis) against EL-4s3 cells pulsed with the immunization peptide or an irrelevant mock peptide Gag77 (the mock peptide value is not shown, but subtracted 10.8 % lysis). Different effector target ratios are shown.

The epitopes tested were immunogenic also when tested using Dendritic cell immunization as measured by IFN ELISPOT assay against Gag433, Env67, Gag150mod, Vif23, and the positive control peptide Gag77 (Figure 7), as well as when measuring specific cell lyse using : Gag433, Vif101(9L), and Env67(2i) (Figure 8), Env67(2i), the control peptide gag77 with no significant respons from unvaccinated mouse splenocytes (Figure 9), and Gag150mod, Pol606mod, and Vif23(9V) (Figure 10), with no unspecific responses from peptide unpulsed splenocytes (Figure 11).

### Conclusion

5 of the vaccine epitope peptides that were immunogenic when immunizing A2tg mice with peptide in Freunds adjuvants were also immunogenic using the method of dendritic cell immunization. This is relevant for dendritic cell immunisation of patients.

### Example 6

### Vaccination with autologous dendritic cells pulsed with HIV antigens for treatment of patients with chronic HIV infection

### Experimental design

The experiment is designed as an open non-controlled, prospective phase I trial. A total of 12 patients with chronic HIV infection are being treated. The criteria for inclusion of the patients are as follows:
1. HIV-1 sero positive (>1 year) male with stable (over a period of at least 3 years) measurable viral load >10⁴ and CD4⁺ T cell count > 300, tissue type HLA-A2⁺, and
2. not in antiviral therapy
3. Age 18-50 years
4. Leucocytes ≥ normal range. Thrombocytes between 100,000/µl and 700,000/µl Hgb ≥ nomal range
5. Creatinin < 140 µmol/l.
6. The patient can be expected to follow the instructions
7. Written consent after having received appropriate information

Criteria for exclusion are as follows:
1. Having received other vaccines within a period of three months prior to receiving the first dendritic cell vaccination.
2. Treated with immune modulatory medicine within a period of three months prior to receiving the first dendritic cell vaccination.
3. Suffering from other chronic infectious diseases including HBV, HCV based on HBsAG, HCV antibody and, possibly, HCV RNA at the time of inclusion.
4. Significant medical disorder according to the researchers' evaluation; including for instance heavy asthma/cold, poorly managed cardiac disease, insulin dependent diabetes mellitus.
5. Serious allergy or previous anaphylactic reactions.
6. Concomitant treatment with other experimental agents.
7. Laboratory parameters which are outside the normal range and regarded as clinically significant.

### Preparation of vaccine and treatment with the vaccine

### HIV-1 peptides

### HLA-A2 peptides

| | Optimal vaccine immunogen NAME | Optimal vaccine immunogen SEQUENCE | | HIV-1 protein | SEQ ID NO: |
|---|---|---|---|---|---|
| 1 | Vif101(9L)var | GLADQLIHL | | Vif | 12 |
| 2 | Vif23(9V)var | SLVKHHMYV | | | 13 |
| 3 | Vpu66(9V)mod | ALVEMGHHV | | Vpu | 14 |
| 4 | Gag150(2L)mod | RLLNAWVKV | | Gag | 15 |
| 5 | Gag433(8S)mod | FLGKIWPS | | | 27 |
| 6 | Pol606(9V)mod | KLGKAGYVV | | Pol | 17 |
| 7 | Env67(2I)var | NIWATHACV | | Env | 18 |

### MHC class II T_{H} peptides

DR1,2,3,4,5,6,7,8,9,51,52,53:
Gag298: KRWIILGLNKIVRMY (SEQ ID NO: 59),

DR1,DR:
Gp41: VWGIKQLQARVLAVERYLKD (SEQ ID NO: 60)

Universal:
PADRE aK(X)VAAWTLKAAa (SEQ ID NO: 61), X=cyclohexylalanine a=D-alanine

The peptides are synthesised to a purity of >95%. Prior to use they are redissolved in sterile RPMI medium and sterile filtered through a 0.22 µm filter. Subsequently, endotoxin tests and culture tests are conducted.

### Preparation of vaccine

### Preparation of dendritic cells

Mononuclear cells (MNC) are isolated by leukapheresis of the patient. During leukapheresis the blood is routinely anti-coagulated with citrate. After leukapheresis the red blood cells are lysed and a further concentration of monocytes is performed by adherence in sterile Petri dishes. From the MNC of each patient autologous immature dendritic cells (DC) are differentiated *in vitro* over a period of 6 days. X-VIVO15 medium is used for the differentiation; the medium is supplemented with 1% autologous plasma and recombinant cytokines approved for clinical use: GM-CSF 1000 U/ml (Leukine), IL-4 250 U/ml (Cellgro). Further maturation of the DC occurs from day 6-8 with the use of IL-1β 25 ng/ml, TNF-α 50 ng/ml, IFN-α 3000U/ml, IFN-γ 1000 U/ml, Polyinosine:polycytidine 12.5 µg/ml. This cocktail provides an optimal maturation of DC and IL-12 induction and is used routinely for patients for the same type of DC immunisation.

### Peptide pulsing

At day 8 the prepared dendritic cells are split out for pulsing with the individual species of peptides. 40 µg/ml of each peptide species is added and the mixture is incubated for 2 hours. Subsequently, the cells are pooled prior to freezing.

### Cryopreservation

Dendritic cells are harvested by addition of 0.2% EDTA. Matured DC are pulsed with the individual species of peptides, are pooled and adjusted to a titre of 10⁸ in 1 ml and are cryopreserved in amounts of 1x10⁷ cell/freezing vial in 85% autologous serum, 5% glucose (Glucosteril 40%) and 10% DMSO. After thawing and wash DC are injected s.c. at 4 sites (0.25 ml injected at each site) for each treatment.

### Microbiological tests

Prior to use of the vaccines endotoxin tests and culturing tests of the cell preparations are conducted.

### Vaccination

Prior to injection the dendritic cells are washed twice in brine. A minimum of 10x106 dendritic cells are administered in 1 ml sterile RPMI at each vaccination. Subcutaneous injection is performed close to both trigonum femorale and at the inner side of each overarm. (0.25 ml at each site x 4 = 1 ml). Aldara^{®} lotion is applied to the skin covering the injection site in connection with the first vaccination and 1 day prior to the subsequent treatments. Aldara^{®} lotion improves the maturation and migration of the injected DC. Aldara^{®} lotion (5% Imiquimod) is registered in Denmark, used for several years and substantially free of adverse reactions. The patients are monitored for 30 minutes for acute toxicity comprising allergic reactions including anaphylactic shock. In case anaphylactic shock occurs it is treated according to general guidelines.

### Response evaluation

### Recording and analysis of data

All relevant data are recorded in data collection documents ("case report form", CRF) and are subsequently registered in a database (Excell or Access) with double recording in order to eliminate typographical errors.

The analysis includes recordings of
1. Toxicity: Clinical investigation, blood chemistry and haematology
2. Immunological response (CRF): IC-Facs and ELLISPOT against HIV peptides, neutralising antibodies against HIV isolates
3. Virus response: viral load, epitope sequences.

### Evaluation of the effect

### Parameters

Paraclinical evaluation: For a preliminary evaluation of the immunological effect of the vaccinations the patients are monitored in terms of the HIV viral load, CD4+ T-cell counts as well as continuous analysis of the HIV antigen specific T-cell reactivity and of the epitope sequences of the HIV isolates to record eventual immune escape mutations. The immunological response against HIV antigens before, under and after vaccinations will be compared.

### Statistics

Paired data before and at different time points after the immunizations are compared using the Wilcoxon test. The immunological response rate and its 95% confidence interval are calculated. Descriptive statistics will be used in order to summarise toxicity, duration of response and characteristics of the patients.

### Immunological and viral monitoring

### Blood samples for immunological and viral monitoring:

Blood samples of 100 ml heparinised blood and 10 ml dry glass for immunological monitoring will be collected prior to treatment, after the third and fourth vaccination and 3 and 6 months after cessation of treatment.

Peripheral blood mononuclear cells (PBMC) are isolated using Lymphoprep density gradient technology and a panel of relevant immunological assays for tests of antigen specific immune reactivity is applied, including measurements of the cytokine production (intracellular fluorescence staining detected by flow cytometry analysis (IC-FACS), and ELLISPOT) and T-cell surface markers for memory and effector cell populations, as well as the proliferative and cytotoxic potential are determined. HIV neutralising antibodies are measured against the patient's own virus and against a panel of other broadly representing HIV-1 prior to treatment and approximately 6 months after last vaccination.

HIV-1 from the patient are isolated from blood (PBMC) and PCR amplified and sequenced to determine if the target epitopes are present and to record eventual immune escape mutations. HIV sequences of the patient are determined prior to treatment, 10-14 days after third and fourth vaccination and 6 months after cessation of treatment as well as at any significant increase in viral load in order to test for possible resistance mutations.

Viral load and CD4+ T-cell counts are determined prior to treatment after third and fourth vaccination as well as 3 and 6 months after cessation of treatment.

### Example 7

### Vaccination with peptide vaccine comprising the preferred 11 HIV-1 epitope immunogens for treatment of patients with chronic HIV infection

### Experimental design

The experiment is designed as an open non-controlled, prospective phase I trial. A total of 20 patients in Africa and/or China with chronic HIV-1 infection are being treated.

### Inclusion criteria

1. HIV-1 seropositive (only HIV group 1).
2. Not in HIV Antiretroviral Therapy (>1 year).
3. Males above 18 years and females above 40 years.
4. Informed consent after oral and written information.

### Exclusion criteria

1. HIV-2 seropositive.
2. Active Tuberculosis (TB).
3. Significant medical disease as judged by the investigators, for example known severe allergy, severe asthma/COLD, badly regulated heart disease, insulin-dependent diabetes mellitus.

### Examination and treatment plan

3 tubes (a vacuum blood sampling tube with a draw volume of 10 ml) is taken from each patient for testing ELISPOT response to vaccine epitopes before and after immunization with a 1.1 solution of peptides in LipoVac adjuvant. In addition Viral load and CD4 cell counts are measures along with clinical status.

### Preparation of vaccine

The vaccine is prepared as a solution of the 11 peptides: GLADQLIHL (SEQ ID NO: 12), SLVKHHMYV (SEQ ID NO: 13), ALVEMGHHV (SEQ ID NO: 14), RLLNAWVKV (SEQ ID NO: 15), FLGKIWPV (SEQ ID NO: 16), KLGKAGYVV (SEQ ID NO: 17), NIWATHACV (SEQ ID NO: 18), VLAEAMSQV (SEQ ID NO: 19), NTVATLYCV (SEQ ID NO: 20), YIKIFIMIV (SEQ ID NO: 21), SLGQHIYET (SEQ ID NO: 22). The vaccine is prepared to be administered in doses containing from 75-100 µg of each peptide.

The patients are monitored for 30 minutes for acute toxicity comprising allergic reactions including anaphylactic shock. In case anaphylactic shock occurs it is treated according to general guidelines.

### Response evaluation

### Recording and analysis of data

All relevant data are recorded in data collection documents ("case report form", CRF) and are subsequently registered in a database (Excell or Access) with double recording in order to eliminate typographical errors.

The analysis includes recordings of
4. Toxicity: Clinical investigation, blood chemistry and haematology
5. Immunological response (CRF): ELISPOT against HIV peptides,
6. Virus response: viral load, epitope sequences.

### Evaluation of the effect Parameters

Paraclinical evaluation.: For a preliminary evaluation of the immunological effect of the vaccinations the patients are monitored in terms of the HIV viral load, CD4+ T-cell counts as well as continuous analysis of the HIV antigen specific T-cell reactivity and of the epitope sequences of the HIV isolates to record eventual immune escape mutations. The immunological response against HIV antigens before, under and after vaccinations will be compared.

### Statistics

Paired data before and at different time points after the immunizations are compared using the Wilcoxon test. The immunological response rate and its 95% confidence interval are calculated. Descriptive statistics will be used in order to summarise toxicity, duration of response and characteristics of the patients.

### Immunological and viral monitoring

### Blood samples for immunological and viral monitoring:

Peripheral blood mononuclear cells (PBMC) are isolated using Lymphoprep density gradient technology and a panel of relevant immunological assays for tests of antigen specific immune reactivity is applied, including measurements of the cytokine production ELLISPOT) prior to treatment and approximately 6 months after last vaccination.

HIV-1 from the patient is isolated from blood (PBMC) and PCR amplified and sequenced to determine if the target epitopes are present and to record eventual immune escape mutations. HIV sequences of the patient are determined prior to treatment, 10-14 days after last immunization and 6 months after cessation of treatment as well as at any significant increase in viral load in order to test for possible resistance mutations.

Viral load and CD4+ T-cell counts are determined prior to treatment, after the last vaccination as well as 6 months after cessation of treatment.

### Example 8

### Vaccination with autologous dendritic cells pulsed with HIV antigens for treatment of patients with chronic HIV infection

The present example presents data from a clinical therapeutic HIV vaccine phase I/II trial. The concept of the therapeutic vaccination protocol is the introduction of anti-HIV immunity in patients towards 7 of the novel identified infrequently targeted HIV epitopes including two epitopes in the Gag gene, one in the Pol gene, two in the Vif gene, one in the VPU gene, and lastly one epitope in the HIV Envelope gene. The introduction of immunity was specifically directed towards the patients' cytotoxic CD8 cells through an interaction with the patients' dendritic cells (DC). The introduction of novel immunity in patients against these selected infrequently targeted HIV epitopes was accomplished by use of autologous dendritic cells.

### Experimental design

The experiment was designed as an open non-controlled, prospective phase I trial. Target population was a total of 12 fully evaluable patients with chronic HIV infection. The number of enrolled patients was variable in order to obtain the required target population. The criteria for inclusion of the patients were as follows:
1. HIV-1 sero positive (>1 year) male with stable (over a period of at least 3 years) measurable viral load >10⁴ and CD4⁺ T cell count > 300, tissue type HLA-A2⁺, and
2. not in antiviral therapy
3. Age 18-50 years
4. Leucocytes in normal range. Thrombocytes between 100,000/µl and 700,000/µl Hgb in nomal range
5. Creatinin < 140 µmol/l.
6. The patient can be expected to follow the instructions (compliance)
7. Written consent after having received appropriate information

Criteria for exclusion were as follows:
1. Having received other vaccines within a period of three months prior to receiving the first dendritic cell vaccination.
2. Treated with immune modulatory medicine within a period of three months prior to receiving the first dendritic cell vaccination.
3. Suffering from other chronic infectious diseases including HBV, HCV based on HBsAG, HCV antibody and, possibly, HCV RNA at the time of inclusion.
4. Significant medical disorder according to the researchers' evaluation; including for instance heavy asthma/cold, poorly managed cardiac disease, insulin dependent diabetes mellitus.
5. Serious allergy or previous anaphylactic reactions.
6. Concomitant treatment with other experimental agents.
7. Laboratory parameters which are outside the normal range and regarded as clinically significant.

### Preparation of vaccine and treatment with the vaccine

### HIV-1 peptides

### HLA-A2 peptides

| | Vaccine immunogen NAME | vaccine immunogen SEQUENCE | | HIV-1 protein | SEQ ID NO: |
|---|---|---|---|---|---|
| 1 | Vif101 | GLADQLIHM | | Vif | 23 |
| 2 | Vif23(9V)var | SLVKHHMYV | | | 13 |
| 3 | Vpu66(9V)mod | ALVEMGHHV | | Vpu | 14 |
| 4 | Gag150(2L)mod | RLLNAWVKV | | Gag | 15 |
| 5 | Gag433 | FLGKIWPS | | | 27 |
| 6 | Pol606(9V)mod | KLGKAGYVV | | Pol | 17 |
| 7 | Env67(2I)var | NIWATHACV | | Env | 18 |

### MHC class II T_{H} peptides

DR1,2,3,4,5,6,7,8,9,51,52,53:
Gag298: KRWIILGLNKIVRMY (SEQ ID NO: 59),

DR1,DR:
Gp41: VWGIKQLQARVLAVERYLKD (SEQ ID NO: 60)

Universal:
PADRE aK(X)VAAWTLKAAa (SEQ ID NO: 61), X=cyclohexylalanine a=D-alanine

The peptides were synthesised to a purity of >95%. Prior to use they were redissolved in sterile RPMI medium and sterile filtered through a 0.22 µ filter. Subsequently, endotoxin tests and culture tests were conducted.

### Preparation of vaccine

### Preparation of dendritic cells

Mononuclear cells (MNC) were isolated by leukapheresis of the patient. During leukapheresis the blood was routinely anti-coagulated with citrate. After leukapheresis the red blood cells were lysed and a further concentration of monocytes was performed by adherence in sterile Petri dishes. From the MNC of each patient autologous immature dendritic cells (DC) were differentiated *in vitro* over a period of 6 days. X-VIVO15 medium is used for the differentiation; the medium was supplemented with 1% autologous plasma and recombinant cytokines approved for clinical use: GM-CSF 1000 U/ml (Leukine), IL-4 250 U/ml (Cellgro).

Further maturation of the DC occurs from day 6-8 with the use of IL-1β 25 ng/ml, TNF-α 50 ng/ml, IFN-α 3000U/ml, IFN-γ 1000 U/ml, Polyinosine:polycytidine 12.5 µg/ml. This cocktail provided an optimal maturation of DC and IL-12 induction and is used routinely for patients for the same type of DC immunisation.

### Peptide pulsing

At day 8 the prepared dendritic cells were split out for pulsing with the individual species of peptides. 40 g/ml of each peptide species was added and the mixture was incubated for 2 hours. Subsequently, the cells are pooled prior to freezing.

### Cryopreservation

Dendritic cells were harvested by addition of 0.2% EDTA. Matured DC are pulsed with the individual species of peptides, were pooled and adjusted to a titre of 10⁸ in 1 ml and were cryopreserved in amounts of 1x10⁷ cell/freezing vial in 85% autologous serum, 5% glucose (Glucosteril 40%) and 10% DMSO. After thawing and wash DC were injected s.c. at 4 sites (0.25 ml injected at each site) for each treatment.

### Microbiological tests

Prior to use of the vaccines endotoxin tests and culturing tests of the cell preparations were conducted.

### Vaccination

Prior to injection the dendritic cells were washed twice in X-Vivo15. A minimum of 10x10⁶ dendritic cells were administered in 0,5 ml sterile X-Vivo15 at each vaccination. Subcutaneous injection was performed at the inner side of each overarm. (0.5 ml at each site x 2 = 1 ml). Aldara^{®} lotion was applied to the skin covering the injection site in connection with the first vaccination and 1 day prior to the subsequent treatments. Aldara^{®} lotion improved the maturation and migration of the injected DC. Aldara^{®} lotion (5% Imiquimod) was registered in Denmark, used for several years and substantially free of adverse reactions. The patients were monitored for 30 minutes for acute toxicity comprising allergic reactions including anaphylactic shock. In case anaphylactic shock occurs it was treated according to general guidelines.

### Response evaluation

### Recording and analysis of data

All relevant data were recorded in data collection documents ("case report form", CRF) and are subsequently registered in a database (Excell or Access) with double recording in order to eliminate typographical errors.

The analysis included recordings of
1. Toxicity: Clinical investigation, blood chemistry and haematology
2. Immunological response (CRF):
   - Phenotypical delineation the CD4 and CD8 elements of the immunesystem
   - Intracellular Flow cytometry evaluating CD8 and CD4 response (TNF-a/INF-g) towards vaccine and wild type epitopes.
   - Dextramer analysis of antigen specific CD8 cells targeted against vaccine epitopes.
   - Cytometric Bead Array evaluating response towards vaccine and wildtype epitopes measured by expression of IL-2, IL-4, IL-5, IL-10, TNF-a and IFN-g.
   - Neutralising antibodies against HIV isolates
3. Virus response:
   - Viral load.
   - Epitope sequences.

### Evaluation of the effect

### Parameters

Paraclinical evaluation: For a preliminary evaluation of the immunological effect of the vaccinations the patients were monitored in terms of the HIV viral load, CD4+ T-cell counts as well as continuous analysis of the HIV antigen specific T-cell reactivity and of the epitope sequences of the HIV isolates to record eventual immune escape mutations. The immunological responses against HIV antigens before, under and after vaccinations were compared.

### Statistics

Paired data before and at different time points after the immunizations were compared using the Wilcoxon test. The immunological response rate and its 95% confidence interval were calculated. Descriptive statistics were be used in order to summarise toxicity, duration of response and characteristics of the patients.

### Immunological and viral monitoring

### Blood samples for immunological and viral monitoring:

Blood samples of 100 ml heparinised blood and 10 ml dry glass for immunological monitoring was collected prior to treatment, after the third and fourth vaccination and 3 and 6 months after cessation of treatment.

Peripheral blood mononuclear cells (PBMC) were isolated using Lymphoprep density gradient technology and a panel of relevant immunological assays for tests of antigen specific immune reactivity was applied, including measurements of the cytokine production (intracellular fluorescence staining detected by flow cytometry analysis, and T-cell surface markers for memory and effector cell populations, as well as the proliferative and cytotoxic potential were determined. HIV neutralising antibodies were measured against the patient's own virus and against a panel of other broadly representing HIV-1 prior to treatment and approximately 6 months after last vaccination.

HIV-1 from the patient were isolated from blood (PBMC) and PCR amplified and sequenced to determine if the target epitopes were present and to record eventual immune escape mutations. HIV sequences of the patient were determined prior to treatment, 10-14 days after third and fourth vaccination and 6 months after cessation of treatment as well as at any significant increase in viral load in order to test for possible resistance mutations.

Viral load and CD4+ T-cell counts were determined prior to treatment after third and fourth vaccination as well as 3 and 6 months after cessation of treatment.

### Results

The clinical protocol called for the enrollment of 12 fully evaluated patients undergoing the complete pre-evaluation, vaccination phase, and post vaccination phase. 10 patients were enrolled of whom 9 was engaged in the vaccination phase (see Figure 13, Study design). Of these, 4 patients completed the vaccination phase and the evaluation point is concluded. No major side effects of the vaccinations have been observed within the group of 4 completed patients. Biological induction of novel immunity has been observed in two of three completed patients, with one not responding to the vaccination.

### REFERENCES

1 WHO Global situation of HIV/AIDS pandemic, end 2001. Weekly epidemiological record 76:381-388, 2001.
2 Editorial. Looking forward to the back of HIV. Nat Med 4:867-868, 1998.
3 Addo MM, MG Yu, ES Rosenberg, BD Walker, M Altfeld. Cytotoxic T-lymphocyte (CTL) responses directed against regulatory and accessory proteins in HIV-1 infection. DNA Cell Biol 21:671-678, 2002.
4 Altfeld M, ES Rosenberg. The role of CD4+ T helper cells in the cytotoxic T lymphocyte response to HIV-1. Curr Opin Immunol 12:375-380, 2000
5 Moore JP, DR Burton. HIV-1 neutralizing antibodies: How full is the bottle? Nat Med 5:142-143, 1999
6 Robinson HL. New hope for an AIDS vaccine. Nat Rev Immunol 2:239-250,2002
7 Fomsgaard A. HIV-1 DNA vaccines. Immunol Lett 1999; 65:127-131
8 Vinner L, Nielsen HV, Bryder, Corbet S, Nielsen C & Fomsgaard A. Gene gun DNA vaccination with Rev independent synthetic HIV-1 gp160 envelope gene using mammalian codons. Vaccine 1999;17:2166-75
9 Fomsgaard A, Nielsen HV, Kirkby N, Bryder K, Corbet S, Nielsen C, Hinkula J, Buus S. Induction of cytotoxic T-cell responses by gene gun DNA vaccination with mini-genes encoding influenza A virus HA and NP CTL-epitopes. Vaccine 1999,18: 681-691
10 Nielsen HV, Lauenmøller SL, Christiansen , Buus S, Fomsgaard A, Petersen E. Complete protection against lethal Toxoplasma gondii infection in mice immunized with a plasmid encoding the SAG1 gene. Infection and Immunity1999; 67:6358-63
11 Corbet S, Nielsen HV, Vinner L, Laemoller S, Therrien D, Tang S, Kronborg G, Mathiesen L, Chaplin P, Brunak S, Buus S, Fomsgaard A. Optimization and immune recognition of multiple novel conserved HLA-A2, human immunodeficiency virut type-1-specific CTL epitopes. 2003. J General Virol 84: 2409-2421.
12 Buus S, Laumoller SL, Worning P, Kesmir C, Frimurer T, Corbet S, Fomsgaard A, Hilden J, Holm A, Brunak S. Sensitive quantitative predictions of peptide-MHC binding bu a "query by committee" artificial neural network approach. 2003. Tissue Antigens (in press)
13 Lauemoller SL, Kesmir C, Corbet S, Fomsgaard A, Holm A, Claesson MH, Brunak S, Buus S. Identifying cytotoxic T cell epitopes from genomic and protein information: "The human MHC project". Reviews in Immunogenetics 2001, 2(4):477-491
14 Patent PA 2000 21922 EP1 "HIV peptides and nucleic acids encoding them for diagnosis and control of HIV infection" 28 jan 2000. Inventor: A Fomsgaard, S Corbet, S Buus, S Brunak
15 Corbet S, Vinner L, Hougaard DM, Bryder K, Nielsen HV, Fomsgaard A. Construction, biological activity, and immunogenicity of synthetic envelope DNA vaccines based on a primary, CCRS-tropic, early HIV type 1 isolate (BX08) with human codons. 2000 AIDS Research and Human Retroviruses 16(18):1997-2008.
16 Vinner L, Wee EGT, Patel S, Corbet S, Gao GP, Nielsen C, Wilson JM, Ertl HCJ, Hanke T, Fomsgaard A. Immunogenicity in Mamu-A*01 Rhesus macaques of a CCRS-tropic HIV-1 envelope from the primary isolate (Bx08) after synthetic DNA prime and recombinant Adenovirus 5 boost. 2003. J Gen Virol 84:203-213.
17 Patent PA 1999 00427: Method for producing a nucleotide sequence construct with optimized codons for an HIV genetic vaccine based on a primary, early HIV isolate and synthetic envelope BX08 constructs" 29 marts 1999, Inventor: A Fomsgaard
18 Goulder PJ et al Late escape from an immunodominant cytotoxic T-lymphocyte response associated with progression to AIDS. Nat Med 3:212-217, 1997
19 Goulder PJ et al Patterns of immunodominance in HIV-1-specific cytotoxic T lymphocyte responses in two human histocompatibility leukocyte antigen (HLA)-identical siblings with HLA-A*0201 are influenced by epitope mutation. J Exp Med 185:1423-1433, 1997
21 Richmann DD et al Rapid evolution of the neutralizing antibody response following primary HIV-1 infection. Proc Natl Acad Sci USA. 2003
22 Walker BD, Korber BT. Immune control of HIV: The obstacles of HLA and viral diversity. Nat Immunol 2:473-475,2001
23 Sheila Tang (Dept Virol, SSI). Novel CTL epitopes in the full genome sequence of Danish HIV-1 clinical isolates. Master Thesis. June 2003.
24a MacGregor RR, JD Boyer, Ugen KE, et al. First human trial of a DNA-based vaccine for treatment of HIV-1 infection: Safety and host response. J Infect Dis 178:92-100,1998.
24b Ugen KE, Nyland SB, Boyer J, et al. DNA vaccination with HIV-1 expressing constructes elicits immune responses in humans. Vaccine 16:1818-1821, 1998
25a Calarota S, Bratt G, Nordlund S, Hinkula J, et al. Cellular cytotoxic responses induced by DNA vaccination in HIV-1 infected patients. Lancet 351:1320-1325,1998.
25bCalarota C, Leandersson AC, Bratt G, Hinkula J, et al. Immune responses in asymptomatic HIV-1-infected patients after HIV-DNA immunization followed by highly active antiretroviral treatment. J Immunol 163:2330-2338,1999.
26. Vinner L. Second-generation HIV-1 envelope DNA vaccines. PhD dissertation, Faculty of Health Sciences, University of Copenhagen. 2003.
27. Nyambi PN, Nadas A, Mbah HA, Burda S, Wiliams C, Gorny MK, S Zolla-Pazner. Immunereactivity of intact viruons of HIV-1 reveals the existence of fewer HIV-1 immunotypes than genotypes. J Virol 74:10670-10670, 2000.
28. Epstein, H et al. Expression and function of HLA-A2.1 in transgenic mice, Eur. J. Immunol. 19, 1575-1583, 1989.
29. Pascolo, S. et al., HLA restricted education and cytologic activity of CD8+ effector T lymphocytes from β2 microtubulin (β2m) HLA-A2.1 monochain transgenic H-2Db β2m double knockout mice, J. Exp. Med. 185, 2043-2051, 1997.
30. Lu, W. et al., Therapeutic dendritic-cell vaccine for chronic HIV-1 infection, Nature Medicine, 10, 1359-1365, 2004.
31. Frahm, N. et al., Control of human immunodeficiency virus replication by cytotoxic T lymphocytes targeting subdominant epitopes. Nature Immunol, 7(2), 173-178, 2006.

## Claims

1. A composition comprising at the most 20 amino acid sequences selected from the group consisting of:
X₁-X₂-ADQLIH-X₃ (SEQ ID NO: 1)
wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
wherein X₁ is selected from the group consisting of: V, I, T and R;
X₁-X₂-VEMGHH-X₃ (SEQ ID NO: 3)
wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
wherein X₁ is selected from the group consisting of: T and L;
FLG-X₁-IW-X₂-X₃ (SEQ ID NO: 5)
wherein X₁ is selected from the group consisting of K and R; X₂ is selected from the group consisting of: P and S; and X₃ is selected from the group consisting of: V and S;
K-X₁-GKAGYV-X₂ (SEQ ID NO: 6)
wherein X₁ is selected from the group consisting of: L, M, I and T; and X₂ is selected from the group consisting of: T and V;
N- X₁-WATHACV (SEQ ID NO: 7)
wherein X₁ is selected from the group consisting of: V and I;
VLAEAMSQ-X₁ (SEQ ID NO: 8)
wherein X₁ is selected from the group consisting of: V, M and A;
N-X₁-VATLYCV (SEQ ID NO: 9)
wherein X₁ is selected from the group consisting of: T and L;
Y-X₁-X₂-IFIMI- X₃ (SEQ ID NO: 10)
wherein X₁ is selected from the group consisting of: I and L; X₂ is selected from the group consisting of: K and R; and X₃ is selected from the group consisting of: V and I;
X₁-LGQHIY- X₂-T (SEQ ID NO: 11)
wherein X₁ is selected from the group consisting of: S, G and N; and X₂ is selected from the group consisting of: E and N;
and wherein said composition comprises the amino acid sequences:
X₁-X₂-ADQLIH-X₃ (SEQ ID NO: 1)
wherein X₁ is selected from the group consisting of: G, S, E, N, D and H; X₂ is selected from the group consisting of: L, M, I, T and V; and X₃ is selected from the group consisting of: L, Q and M;
SLVKHHMY- X₁ (SEQ ID NO: 2)
wherein X₁ is selected from the group consisting of: V, I, T and R;
X₁-X₂-VEMGHH-X₃ (SEQ ID NO: 3)
wherein X₁ is selected from the group consisting of: A and I; X₂ is selected from the group consisting of: L and M; and X₃ is selected from the group consisting of: A, V and R;
R-X₁-LNAWVKV (SEQ ID NO: 4)
wherein X₁ is selected from the group consisting of: T and L;
FLG-X₁-IW-X₂-X₃ (SEQ ID NO: 5)
wherein X₁ is selected from the group consisting of K and R; X₂ is selected from the group consisting of: P and S, and X₃ is selected from the group consisting of: V and S;
K-X₁-GKAGYV-X₂ (SEQ ID NO: 6)
wherein X₁ is selected from the group consisting of: L, M, I and T; and X₂ is selected from the group consisting of: T and V; and
N- X₁-WATHACV (SEQ ID NO: 7)
wherein X₁ is selected from the group consisting of: V and I.

2. A composition according to claim 1, wherein at least one of said amino acid sequences is selected from the group consisting of: GLADQLIHL (SEQ ID NO: 12), SLVKHHMYV (SEQ ID NO: 13), ALVEMGHHV (SEQ ID NO: 14), RLLNAWVKV (SEQ ID NO: 15), FLGKIWPS (SEQ ID NO: 16), KLGKAGYVV (SEQ ID NO: 17), NIWATHACV (SEQ ID NO: 18), VLAEAMSQV (SEQ ID NO: 19), NTVATLYCV (SEQ ID NO: 20), YIKIFIMIV (SEQ ID NO: 21), SLGQHIYET (SEQ ID NO: 22).

3. A composition according to any of the preceding claims, said composition further comprising a T-helper epitope.

4. A composition according to claim 3, wherein said T-helper epitope is selected from the group consisting of:TPPAYRPPNAPIL (SEQ ID NO: 58), KRWIILGLNKIVRMY (SEQ ID NO: 59), VWGIKQLQARVLAVERYLKD (SEQ ID NO: 60), aK(X)VAAWTLKAAa (SEQ ID NO: 61), X=cyclohexylalanine a=D-alanine (PADRE)

5. A composition according to any of the preceding claims, wherein at least one of said amino acid sequences as present in the composition, is:
a) capable of eliciting a CTL response in HLA-A2 transgenic mice as determined by > 10% lysis at Effector:Target (E:T) cell ratio 50:1 in a chromium release assay; and/or
b) capable of causing formation of > 10 spot forming units (s.f.u.) per 10⁶ splenocytes from HLA-A2 transgenic mice when assayed in IFN-γ ELISPOT assays; and/or
c) recognised by a CD8+ T cell recall response in at least one HIV positive HLA-A2+ and/or HLA-A2- patient in a group of 17 as determined in an intracellular-IFNγ FACS assay.

6. A composition according to any of the preceding claims, wherein each of said amino acid sequences are present in an amount of from 20-300 µg/dose, such as from 50-200 µg/dose, such as from 75-175 µg/dose, such as from 80-150 µg/dose, such as from 90-125 µg/dose, such as from 95-112.5 µg/dose.

7. A composition according to any of the preceding claims, said composition further comprising an adjuvant substance.

8. A composition according to claim 7, wherein the adjuvant substance is selected from the group consisting of: aluminium hydroxide or phosphate (alum), Freund's complete or incomplete adjuvants, QuilA and Quil A derivates, RIBI, Carbopol, physiologically acceptable oil vehicles including mannide mono-oleate (Aacel A), perfluorocarbon (Fluosol-DA), dimethyldioctadecylammonium bromide (DDA), endotoxins or lipopolysaccharide or Lipid A components of Gram-negative bacteria,

9. A composition according to any of the preceding claims, said composition further comprising monocyte-derived dendritic cells.

10. A nucleic acid molecule consisting of a sequence of nucleic acids encoding at least 7 and at the most 20 of the amino acid sequences as defined in any of claims 1-2.

11. A nucleic acid molecule according to claim 10, said nucleic acid molecule comprising one or more of the following nucleic acid sequences:
GGC CTG GCC GAC CAG CTG ATC CAC CTG (SEQ ID NO: 63);
AGC CTG GTG AAG CAC CAC ATG TAC GTG (SEQ ID NO: 64);
GCA CTA GTG GAG ATG GGA CAT CAC GTG (SEQ ID NO: 65);
CGC CTG CTG AAC GCC TGG GTG AAA GTG (SEQ ID NO: 66);
TTC CTG GGC AAG ATC TGG CCA TCT (SEQ ID NO: 67);
AAG CTT GGC AAG GCC GGC TAC GTG GTG (SEQ ID NO: 68);
AAC ATC TGG GCA ACA CAC GCC TGC GTC (SEQ ID NO: 69);
GTG CTG GCC GAA GCT ATG AGC CAG GTC (SEQ ID NO: 70);
AAC ACC GTG GCT ACT CTG TAC TGT GTC (SEQ ID NO: 71);
TAC ATC AAG ATT TTC ATC ATG ATC GTG (SEQ ID NO: 72);
AGC TTG GGA CAA CAT ATC TAC GAG ACC (SEQ ID NO: 73).

12. A composition comprising a nucleic acid molecule according to any of claims 10-11.

13. A composition according to any of claims 1-9 or 12 or a nucleic acid molecule according to any of claims 10-11 for use in medicine.

14. Use of a composition according to any of claims 1-9 or 12 or a nucleic acid sequence according to any of claims 10-11 in the manufacture of a medicament for the prevention and/or treatment of human immunodeficiency virus infections/acquired immunodeficiency syndrome.

## Patentansprüche

1. Zusammensetzung, umfassend höchstens 20 Aminosäuresequenzen, ausgewählt aus der Gruppe, bestehend aus:
X₁-X₂-ADQLIH-X₃ (SEQ ID Nr.: 1) wobei X₁ ausgewählt ist aus der Gruppe, bestehend aus: G, S, E, N, D und H;
X₂ ausgewählt ist aus der Gruppe, bestehend aus: L, M, I, T und V; und X₃ ausgewählt ist aus der Gruppe, bestehend aus: L, Q und M;
SLVKHHMY- X₁ (SEQ ID Nr.: 2)
wobei X₁ ausgewählt ist aus der Gruppe, bestehend aus: V, I, T und R;
X₁-X₂-VEMGHH-X₃ (SEQ ID Nr.: 3)
wobei X₁ ausgewählt ist aus der Gruppe, bestehend aus: A und I; X₂ ausgewählt ist aus der Gruppe, bestehend aus: L und M; und X₃ ausgewählt ist aus der Gruppe, bestehend aus: A, V und R;
R-X₁-LNAWVKV (SEQ ID Nr.: 4)
wobei X₁ ausgewählt ist aus der Gruppe, bestehend aus: T und L;
FLG-X₁-IW-X₂-X₃ (SEQ ID Nr.: 5)
wobei X₁ ausgewählt ist aus der Gruppe, bestehend aus K und R; X₂ ausgewählt ist aus der Gruppe, bestehend aus : P und S; und X₃ ausgewählt ist aus der Gruppe, bestehend aus: V und S;
K-X₁-GKAGYV-X₂ (SEQ ID Nr.: 6)
wobei X₁ ausgewählt ist aus der Gruppe, bestehend aus: L, M, I und T; und X₂ ausgewählt ist aus der Gruppe, bestehend aus: T und V;
N-X₁-WATHACV (SEQ ID Nr.: 7)
wobei X₁ ausgewählt ist aus der Gruppe, bestehend aus: V und I;
VLAEAMSQ-X₁ (SEQ ID Nr.: 8)
wobei X₁ ausgewählt ist aus der Gruppe, bestehend aus: V, M und A;
N-X₁-VATLYCV (SEQ ID Nr.: 9)
wobei X₁ ausgewählt ist aus der Gruppe, bestehend aus: T und L;
Y-X₁-X₂-IFIMI- X₃ (SEQ ID Nr.: 10)
wobei X₁ ausgewählt ist aus der Gruppe, bestehend aus: I und L; X₂ ausgewählt ist aus der Gruppe, bestehend aus: K und R; und X₃ ausgewählt ist aus der Gruppe, bestehend aus: V und I;
X₁-LGQHIY- X₂-T (SEQ ID Nr.: 11)
wobei X₁ ausgewählt ist aus der Gruppe, bestehend aus: S, G und N; und X₂ ausgewählt ist aus der Gruppe, bestehend aus: E und N;
und wobei die Zusammensetzung folgende Aminosäuresequenzen umfasst:
X₁-X₂-ADQLIH-X₃ (SEQ ID Nr.: 1)
wobei X₁ ausgewählt ist aus der Gruppe, bestehend aus: G, S, E, N, D und H; X₂ ausgewählt ist aus der Gruppe, bestehend aus: L, M, I, T und V; und X₃ ausgewählt ist aus der Gruppe, bestehend aus: L, Q und M;
SLVKHHMY- X₁ (SEQ ID Nr.: 2)
wobei X₁ ausgewählt ist aus der Gruppe, bestehend aus: V, I, T und R;
X₁-X₂-VEMGHH-X₃ (SEQ ID Nr.: 3)
wobei X₁ ausgewählt ist aus der Gruppe, bestehend aus: A und I; X₂ ausgewählt ist aus der Gruppe, bestehend aus: L und M; und X₃ ausgewählt ist aus der Gruppe, bestehend aus: A, V und R;
R-X₁-LNAWVKV (SEQ ID Nr.: 4)
wobei X₁ ausgewählt ist aus der Gruppe, bestehend aus: T und L;
FLG-X₁-IW-X₂-X₃ (SEQ ID Nr.: 5)
wobei X₁ ausgewählt ist aus der Gruppe, bestehend aus K und R; X₂ ausgewählt ist aus der Gruppe, bestehend aus : P und S, und X₃ ausgewählt ist aus der Gruppe, bestehend aus: V und S;
K-X₁-GKAGYV-X₂ (SEQ ID Nr.: 6)
wobei X₁ ausgewählt ist aus der Gruppe, bestehend aus: L, M, I uned T; und X₂ ausgewählt ist aus der Gruppe, bestehend aus: T und V; und
N- X₁-WATHACV (SEQ ID Nr.: 7)
wobei X₁ ausgewählt ist aus der Gruppe, bestehend aus: V und I.

2. Zusammensetzung Anspruch 1, wobei mindestens eine der Aminosäuresequenzen ausgewählt ist aus der Gruppe, bestehend aus :
GLADQLIHL (SEQ ID Nr.: 12), SLVKHHMYV (SEQ ID Nr.: 13), ALVEMGHHV (SEQ ID Nr.: 14), RLLNAWVKV (SEQ ID Nr.: 15), FLGKIWPS (SEQ ID Nr.: 16), KLGKAGYVV (SEQ ID Nr.: 17), NIWATHACV (SEQ ID Nr.: 18), VLAEAMSQV (SEQ ID Nr.: 19), NTVATLYCV (SEQ ID Nr.: 20), YIKIFIMIV (SEQ ID Nr.: 21), SLGQHIYET (SEQ ID Nr.: 22).

3. Zusammensetzung nach einem der vorhergehenden Ansprüche, wobei die Zusammensetzung weiterhin ein T-Helferepitop umfasst.

4. Zusammensetzung nach Anspruch 3, wobei das T-Helferepitop ausgewählt ist aus der Gruppe, bestehend aus :TPPAYRPPNAPIL (SEQ ID Nr.: 58), KRWIILGLNKIVRMY (SEQ ID Nr.: 59), VWGIKQLQARVLAVERYLKD (SEQ ID Nr.: 60), aK(X)VAAWTLKAAa (SEQ ID Nr.: 61), X=Cyclohexylalanin a=D-Alanin (PADRE)

5. Zusammensetzung nach einem der vorhergehenden Ansprüche, wobei mindestens eine der in der Zusammensetzung vorhandenen Aminosäuresequenzen:
a) in der Lage ist, bei HLA-A2 transgenen Mäusen eine CTL-Reaktion auszulösen, was durch > 10 % Lyse bei einem Zellverhältnis EffeKtor:Ziel (E:Z) von 50:1 in einem Chrom-Release-Assay bestimmt wird; und/oder
b) in der Lage ist, bei der Untersuchung mittels IFN-γ-ELISPOT-Assays die Bildung von > 10 Spot Forming Units (SFU) pro 10⁶ Splenozyten HLA-A2 transgener Mäuse einzuleiten; und/oder
c) von einer CD8+ Gedächtnis-T-Zellantwort bei mindestens einem HIVpositiven HLA-A2+- und/oder HLA-A2--Patienten in einer Gruppe von 17 erkannt wird, was durch ein intrazelluläres IFNγ-FACS-Assay bestimmt wird.

6. Zusammensetzung nach einem der vorherstehenden Ansprüche, wobei jede der Aminosäuresequenzen in einer Menge in Höhe von 20-300 µg/Dosis, wie 50-200 µg/Dosis, wie 75-175 µg/Dosis, wie 80-150 µg/Dosis, wie 90-125 µg/Dosis, wie 95-112,5 µg/Dosis, vorliegt.

7. Zusammensetzung nach einem der vorhergehenden Ansprüche, wobei die Zusammensetzung weiterhin eine Hilfsmittelsubstanz umfasst.

8. Zusammensetzung nach Anspruch 7, wobei die Hilfsmittelsubstanz ausgewählt ist aus der Gruppe, bestehend aus: Aluminiumhydroxid oder Aluminiumphosphat (Alaum), Freund's Complete oder Incomplete Adjuvants, Quil A und Quil-A-Derivate, RIBI, Carbopol, physiologisch verträgliche Ölträger, einschließlich Mannidmonooleat (Aacel A), Perfluorkohlenstoff (Fluosol-DA), Dimethyldioctadecylammoniumbromid (DDA), Endotoxine oder Lipopolysaccharide oder Lipid-A-Bestandteile gramnegativer Bakterien.

9. Zusammensetzung nach einem der vorhergehenden Ansprüche, wobei die Zusammensetzung weiterhin von Monozyten abstammende dendritischen Zellen umfasst.

10. Nukleinsäuremolekül, bestehend aus einer Sequenz Nukleinsäuren, die mindestens 7 und höchstens 20 der Aminosäuresequenzen nach einem der Ansprüche 1-2 kodieren.

11. Nukleinsäuremolekül nach Anspruch 10, wobei das Nukleinsäuremolekül eine oder mehrere der folgenden Nukleinsäuresequenzen umfasst:
GGC CTG GCC GAC CAG CTG ATC CAC CTG (SEQ ID Nr.: 63);
AGC CTG GTG AAG CAC CAC ATG TAC GTG (SEQ ID Nr.: 64);
GCA CTA GTG GAG ATG GGA CAT CAC GTG (SEQ ID Nr.: 65);
CGC CTG CTG AAC GCC TGG GTG AAA GTG (SEQ ID Nr.: 66);
TTC CTG GGC AAG ATC TGG CCA TCT (SEQ ID Nr.: 67);
AAG CTT GGC AAG GCC GGC TAC GTG GTG (SEQ ID Nr.: 68);
AAC ATC TGG GCA ACA CAC GCC TGC GTC (SEQ ID Nr.: 69);
GTG CTG GCC GAA GCT ATG AGC CAG GTC (SEQ ID Nr.: 70);
AAC ACC GTG GCT ACT CTG TAC TGT GTC (SEQ ID Nr.: 71);
TAC ATC AAG ATT TTC ATC ATG ATC GTG (SEQ ID Nr.: 72);
AGC TTG GGA CAA CAT ATC TAC GAG ACC (SEQ ID Nr.: 73).

12. Zusammensetzung, umfassend ein Nukleinsäuremolekül nach einem der Ansprüche 10-11.

13. Zusammensetzung nach einem der Ansprüche 1-9 oder 12 oder ein Nukleinsäuremolekül nach einem der Ansprüche 10-11 zur Verwendung in der Medizin.

14. Verwendung einer Zusammensetzung nach einem der Ansprüche 1-9 oder 12 oder eines Nukleinsäuremoleküls nach einem der Ansprüche 10-11 zur Herstellung eines Medikaments zur Verhinderung und/oder Behandlung von HIV-Infektionen/AIDS.

## Revendications

1. Composition comprenant au plus 20 séquences d'acides aminés choisies dans le groupe consistant en :
X₁-X₂-ADQLIH-X₃ (SEQ ID N° : 1)
où X₁ est choisi dans le groupe consistant en : G, S, E, N, D et H ; X₂ est choisi dans le groupe consistant en : L, M, I, T et V ; et X₃ est choisi dans le groupe consistant en : L, Q et M ;
SLVKHHMY- X₁ (SEQ ID N° : 2)
où X₁ est choisi dans le groupe consistant en : V, I, T et R ;
X₁-X₂-VEMGHH-X₃ (SEQ ID N° : 3)
où X₁ est choisi dans le groupe consistant en : A et I ; X₂ est choisi dans le groupe consistant en : L et M ; et X₃ est choisi dans le groupe consistant en : A, V et R ;
R-X₁-LNAWVKV (SEQ ID N° : 4)
où X₁ est choisi dans le groupe consistant en : T et L ;
FLG-X₁-IW-X₂-X₃ (SEQ ID N° : 5)
où X₁ est choisi dans le groupe consistant en K et R ; X₂ est choisi dans le groupe consistant en : P et S ; et X₃ est choisi dans le groupe consistant en : V et S ;
K-X₁-GKAGYV-X₂ (SEQ ID N° : 6)
où X₁ est choisi dans le groupe consistant en : L, M, I et T ; et X₂ est choisi dans le groupe consistant en : T et V ;
N- X₁-WATHACV (SEQ ID N° : 7)
où X₁ est choisi dans le groupe consistant en : V et I ;
VLAEAMSQ-X₁ (SEQ ID N° : 8)
où X₁ est choisi dans le groupe consistant en : V, M et A ;
N-X₁-VATLYCV (SEQ ID N° : 9)
où X₁ est choisi dans le groupe consistant en : T et L ;
Y-X₁-X₂-IFIMI- X₃ (SEQ ID N° : 10)
où X₁ est choisi dans le groupe consistant en : I et L ; X₂ est choisi dans le groupe consistant en : K et R ; et X₃ est choisi dans le groupe consistant en : V et I;
X₁-LGQHIY- X₂-T (SEQ ID N° : 11)
où X₁ est choisi dans le groupe consistant en : S, G et N ; et X₂ est choisi dans le groupe consistant en : E et N ;
où ladite composition comprend les séquences d'acides aminés suivantes :
X₁-X₂-ADQLIH-X₃ (SEQ ID N° : 1)
où X₁ est choisi dans le groupe consistant en : G, S, E, N, D et H ; X₂ est choisi dans le groupe consistant en : L, M, I, T et V ; et X₃ est choisi dans le groupe consistant en : L, Q et M ;
SLVKHHMY- X₁ (SEQ ID N° : 2)
où X₁ est choisi dans le groupe consistant en : V, I, T et R ;
X₁-X₂-VEMGHH-X₃ (SEQ ID N° : 3)
où X₁ est choisi dans le groupe consistant en : A et 1 ; X₂ est choisi dans le groupe consistant en : L et M ; et X₃ est choisi dans le groupe consistant en : A, V et R ;
R-X₁-LNAWVKV (SEQ ID N° : 4)
où X₁ est choisi dans le groupe consistant en : T et L ;
FLG-X₁-IW-X₂-X₃ (SEQ ID N° : 5)
où X₁ est choisi dans le groupe consistant en K et R ; X₂ est choisi dans le groupe consistant en : P et S ; et X₃ est choisi dans le groupe consistant en : V et S ;
K-X₁-GKAGYV-X₂ (SEQ ID N° : 6)
où X₁ est choisi dans le groupe consistant en : L, M, I et T ; et X₂ est choisi dans le groupe consistant en : T et V ; et
N- X₁-WATHACV (SEQ ID N° : 7)
où X₁ est choisi dans le groupe consistant en : V et I.

2. Composition selon la revendication 1, où au moins une desdites séquences d'acides aminés est choisie dans le groupe consistant en : GLADQLIHL (SEQ ID N° : 12), SLVKHHMYV (SEQ ID N° : 13), ALVEMGHHV (SEQ ID N° : 14), RLLNAWVKV (SEQ ID N° : 15), FLGKIWPS (SEQ ID N° : 16), KLGKAGYVV (SEQ ID N° : 17), NIWATHACV (SEQ ID N° : 18), VLAEAMSQV (SEQ ID N° : 19), NTVATLYCV (SEQ ID N° : 20), YIKIFIMIV (SEQ ID N° : 21), SLGQHIYET (SEQ ID N° : 22).

3. Composition selon l'une quelconque des revendications précédentes, ladite composition comprenant également un épitope T auxiliaire.

4. Composition selon la revendication 3, où ledit épitope T auxiliaire est choisi dans le groupe consistant en : TPPAYRPPNAPIL (SEQ ID N °: 58), KRWIILGLNKIVRMY (SEQ ID N °: 59), VWGIKQLQARVLAVERYLKD (SEQ ID N °: 60), aK(X)VAAWTLKAAa (SEQ ID N °: 61), X=cyclohexylalanine, a=D-alanine (PADRE).

5. Composition selon l'une quelconque des revendications précédentes, où au moins une desdites séquences d'acides aminés présentes dans la composition est :
a) capable de déclencher une réponse des lymphocytes T cytotoxiques chez des souris transgéniques HLA-A2 déterminée par > 10 % de lyse pour un rapport effecteur/cellules cibles (E/C) de 50/1 dans un test de relargage de chrome ; et/ou
b) capable de causer la formation de > 10 unités formant des taches (u.f.t.) par 10⁶ splénocytes provenant de souris transgéniques HLA-A2 lors d'analyses par tests ELISPOT IFN-γ ; et/ou
c) reconnue par une réponse immunitaire secondaire des lymphocytes T CD8+ chez au moins un patient HLA-A2+ et/ou HLA-A2- séropositif parmi un groupe de 17 telle que déterminée par un test FACS sur IFNγ intracellulaires.

6. Composition selon l'une quelconque des revendications précédentes, où chacune desdites séquences d'acides aminés est présente dans une quantité allant de 20 à 300 µg/dose, telle que 50 à 200 µg/dose, telle que 75 à 175 µg/dose, telle que 80 à 150 µg/dose, telle que 90 à 125 µg/dose, telle que 95 à 112,5 µg/dose.

7. Composition selon l'une quelconque des revendications précédentes, ladite composition comprenant également une substance adjuvante.

8. Composition selon la revendication 7, où la substance adjuvante est choisie dans le groupe consistant en : hydroxyde ou phosphate d'aluminium (alun), adjuvants complets ou incomplets de Freund, dérivés de QuilA et Quil A, RIBI, carbopol, véhicules huileux physiologiquement acceptables y compris le mono-oléate de mannide (Aacel A), perfluorocarbure (Fluosol-DA), bromure de diméthyldioctadécylammonium (DDA), endotoxines ou composants lipopolysaccharide ou lipide A de bactéries Gram négatives.

9. Composition selon l'une quelconque des revendications précédentes, ladite composition comprenant également des cellules dendritiques provenant de monocytes.

10. Molécule d'acide nucléique consistant en une séquence d'acides nucléiques codant pour au moins 7 et au plus 20 des séquences d'acides aminés définies dans l'une quelconque des revendications 1 et 2.

11. Molécule d'acide nucléique selon la revendication 10, ladite molécule d'acide nucléique comprenant au moins une des séquences d'acides nucléiques suivantes :
GGC CTG GCC GAC CAG CTG ATC CAC CTG (SEQ ID N° : 63) ;
AGC CTG GTG AAG CAC CAC ATG TAC GTG (SEQ ID N °: 64) ;
GCA CTA GTG GAG ATG GGA CAT CAC GTG (SEQ ID N °: 65) ;
CGC CTG CTG AAC GCC TGG GTG AAA GTG (SEQ ID N °: 66) ;
TTC CTG GGC AAG ATC TGG CCA TCT (SEQ ID N °: 67) ;
AAG CTT GGC AAG GCC GGC TAC GTG GTG (SEQ ID N °: 68) ;
AAC ATC TGG GCA ACA CAC GCC TGC GTC (SEQ ID N °: 69) ;
GTG CTG GCC GAA GCT ATG AGC CAG GTC (SEQ ID N °: 70) ;
AAC ACC GTG GCT ACT CTG TAC TGT GTC (SEQ ID N °: 71) ;
TAC ATC AAG ATT TTC ATC ATG ATC GTG (SEQ ID N °: 72) ;
AGC TTG GGA CAA CAT ATC TAC GAG ACC (SEQ ID N °: 73).

12. Composition comprenant une molécule d'acide nucléique selon l'une quelconque des revendications 10 et 11.

13. Composition selon l'une quelconque des revendications 1 à 9 ou 12 ou molécule d'acide nucléique selon l'une quelconque des revendications 10 à 11 destinée à un usage médical.

14. Utilisation d'une composition selon l'une quelconque des revendications 1 à 9 ou 12 ou d'une séquence d'acide nucléique selon l'une quelconque des revendications 10 à 11 dans la fabrication d'un médicament pour la prévention et/ou le traitement des infections par le virus de l'immunodéficience humaine/du syndrome d'immunodéficience acquise.


### SEQUENCE LISTING

<110> Statens Serum Institut
   Fomsgaard, Anders
<120> HIV vaccine
<130> P40646pc01
<150> PA 2006 00750
   <151> 2006-07-01
<160> 73
<170> FastSEQ for Windows Version 4.0
<210> 1
   <211> 9
   <212> PRT
   <213> Homo sapiens
<220>
   <221> VARIANT
   <222> 1
   <223> Amino acid residue may be Gly, Ser, Glu, Asn, Asp or His.
<221> VARIANT
   <222> 2
   <223> Amino acid residue may be Leu, Met, Ile, Thr or Val.
<221> VARIANT
   <222> 9
   <223> Amino acid residue may be Leu, Met or Gln.
<400> 1
   Xaa Xaa Ala Asp Gln Leu Ile His Xaa
    1               5
<210> 2
   <211> 9
   <212> PRT
   <213> Human Immunodeficiency Virus-1
<220>
   <221> VARIANT
   <222> 9
   <223> Amino acid residue may be Val, Ile, Thr or Arg
<400> 2
   Ser Leu Val Lys His His Met Tyr Xaa
    1               5
<210> 3
   <211> 9
   <212> PRT
   <213> Human Immunodeficiency Virus-1
<220>
   <221> VARIANT
   <222> 1
   <223> Amino acid residue may be Ala or Ile.
<221> VARIANT
   <222> 2
   <223> Amino acid residue may be Leu or Met
<221> VARIANT
   <222> 9
   <223> Amino acid residue may be Ala, Val or Arg.
<400> 3
   Xaa Xaa Val Glu Met Gly His His Xaa
    1               5
<210> 4
   <211> 9
   <212> PRT
   <213> Human Immunodeficiency Virus-1
<220>
   <221> VARIANT
   <222> 2
   <223> Amino acid residue may be Thr or Leu.
<400> 4
   Arg Xaa Leu Asn Ala Trp Val Lys Val
    1               5
<210> 5
   <211> 8
   <212> PRT
   <213> Human Immunodeficiency Virus-1
<220>
   <221> VARIANT
   <222> 4
   <223> Amino acid residue may be Lys or Arg.
<221> VARIANT
   <222> 7
   <223> Amino acid residue may be Pro or Ser.
<221> VARIANT
   <222> 8
   <223> Amino acid residue may be Val or Ser.
<400> 5
   Phe Leu Gly Xaa Ile Trp Xaa Xaa
    1               5
<210> 6
   <211> 9
   <212> PRT
   <213> Human Immunodeficiency Virus-1
<220>
   <221> VARIANT
   <222> 2
   <223> Amino acid residue may be Leu, Met, Ile or Thr.
<221> VARIANT
   <222> 9
   <223> Amino acid residue may be Thr or Val.
<400> 6
   Lys Xaa Gly Lys Ala Gly Tyr Val Xaa
    1               5
<210> 7
   <211> 9
   <212> PRT
   <213> Human Immunodeficiency Virus-1
<220>
   <221> VARIANT
   <222> 2
   <223> Amino acid residue may be Val or Ile.
<400> 7
   Asn Xaa Trp Ala Thr His Ala Cys Val
    1               5
<210> 8
   <211> 9
   <212> PRT
   <213> Human Immunodeficiency Virus-1
<220>
   <221> VARIANT
   <222> 9
   <223> Amino acid residue may be Val, Met or Ala.
<400> 8
   Val Leu Ala Glu Ala Met Ser Gln Xaa
   1 5
<210> 9
   <211> 9
   <212> PRT
   <213> Human Immunodeficiency Virus-1
<220>
   <221> VARIANT
   <222> 2
   <223> Amino acid residue may be Thr or Leu.
<400> 9
   Asn Xaa Val Ala Thr Leu Tyr Cys Val
   1 5
<210> 10
   <211> 9
   <212> PRT
   <213> Human Immunodeficiency Virus-1
<220>
   <221> VARIANT
   <222> 2
   <223> Amino acid residue may be Ile or Leu
<221> VARIANT
   <222> 3
   <223> Amino acid residue may be Lys or Arg.
<221> VARIANT
   <222> 9
   <223> Amino acid residue may be Val or Ile.
<400> 10
   Tyr Xaa Xaa Ile Phe Ile Met Ile Xaa
   1 5
<210> 11
   <211> 9
   <212> PRT
   <213> Human Immunodeficiency Virus-1
<220>
   <221> VARIANT
   <222> 1
   <223> Amino acid residue may be Ser, Gly, Asn.
<221> VARIANT
   <222> 8
   <223> Amino acid residue may be Glu or Asn.
<400> 11
   Tyr Xaa Xaa Ile Phe Ile Met Ile Xaa
   1               5
<210> 12
   <211> 9
   <212> PRT
   <213> Human Immunodeficiency Virus-1
<400> 12
   Gly Leu Ala Asp Gln Leu Ile His Leu
   1               5
<210> 13
   <211> 9
   <212> PRT
   <213> Human Immunodeficiency Virus-1
<400> 13
   Ser Leu Val Lys His His Met Tyr Val
   1               5
<210> 14
   <211> 9
   <212> PRT
   <213> Human Immunodeficiency Virus-1
<400> 14
   Ala Leu Val Glu Met Gly His His Val
   1               5
<210> 15
   <211> 9
   <212> PRT
   <213> Human Immunodeficiency Virus-1
<400> 15
   Arg Leu Leu Asn Ala Trp Val Lys Val
   1               5
<210> 16
   <211> 8
   <212> PRT
   <213> Human Immunodeficiency Virus-1
<400> 16
   Phe Leu Gly Lys Ile Trp Pro Val
   1               5
<210> 17
   <211> 9
   <212> PRT
   <213> Human Immunodeficiency Virus-1
<400> 17
   Lys Leu Gly Lys Ala Gly Tyr Val Val
   1               5
<210> 18
   <211> 9
   <212> PRT
   <213> Human Immunodeficiency Virus-1
<400> 18
   Asn Ile Trp Ala Thr His Ala Cys Val
   1               5
<210> 19
   <211> 9
   <212> PRT
   <213> Human Immunodeficiency Virus-1
<400> 19
   Val Leu Ala Glu Ala Met Ser Gln Val
   1               5
<210> 20
   <211> 9
   <212> PRT
   <213> Human Immunodeficiency Virus-1
<400> 20
   Asn Thr Val Ala Thr Leu Tyr Cys Val
   1               5
<210> 21
   <211> 9
   <212> PRT
   <213> Human Immunodeficiency Virus-1
<400> 21
   Tyr Ile Lys Ile Phe Ile Met Ile Val
   1 5
<210> 22
   <211> 9
   <212> PRT
   <213> Human Immunodeficiency Virus-1
<400> 22
   Ser Leu Gly Gln His Ile Tyr Glu Thr
   1 5
<210> 23
   <211> 9
   <212> PRT
   <213> Human Immunodeficiency Virus-1
<400> 23
   Gly Leu Ala Asp Gln Leu Ile His Met
   1 5
<210> 24
   <211> 9
   <212> PRT
   <213> Human Immunodeficiency Virus-1
<400> 24
   Ser Leu Val Lys His His Met Tyr Ile
   1 5
<210> 25
   <211> 9
   <212> PRT
   <213> Human Immunodeficiency Virus-1
<400> 25
   Ala Leu Val Glu Met Gly His His Ala
   1 5
<210> 26
   <211> 9
   <212> PRT
   <213> Human Immunodeficiency Virus-1
<400> 26
   Arg Thr Leu Asn Ala Trp Val Lys Val
   1 5
<210> 27
   <211> 8
   <212> PRT
   <213> Human Immunodeficiency Virus-1
<400> 27
   Phe Leu Gly Lys Ile Trp Pro Ser
   1 5
<210> 28
   <211> 9
   <212> PRT
   <213> Human Immunodeficiency Virus-1
<400> 28
   Lys Leu Gly Lys Ala Gly Tyr Val Thr
   1 5
<210> 29
   <211> 9
   <212> PRT
   <213> Human Immunodeficiency Virus-1
<400> 29
   Asn Val Trp Ala Thr His Ala Cys Val
   1 5
<210> 30
   <211> 9
   <212> PRT
   <213> Human Immunodeficiency Virus-1
<400> 30
   Val Leu Ala Glu Ala Met Ser Gln Ala
   1 5
<210> 31
   <211> 9
   <212> PRT
   <213> Human Immunodeficiency Virus-1
<400> 31
   Asn Thr Val Ala Thr Leu Tyr Cys Ala
   1 5
<210> 32
   <211> 9
   <212> PRT
   <213> Human Immunodeficiency Virus-1
<400> 32
   Tyr Ile Lys Ile Phe Ile Met Ile Val
   1 5
<210> 33
   <211> 9
   <212> PRT
   <213> Human Immunodeficiency Virus-1
<400> 33
   Thr Leu Gln Glu Gln Ile Gly Trp Met
   1 5
<210> 34
   <211> 9
   <212> PRT
   <213> Human Immunodeficiency Virus-1
<400> 34
   Ala Thr Leu Glu Glu Met Met Thr Ala
   1 5
<210> 35
   <211> 9
   <212> PRT
   <213> Human Immunodeficiency Virus-1
<400> 35
   Glu Met Met Thr Ala Cys Gln Gly Val
   1 5
<210> 36
   <211> 9
   <212> PRT
   <213> Human Immunodeficiency Virus-1
<400> 36
   Val Leu Ala Glu Ala Met Ser Gln Val
   1 5
<210> 37
   <211> 9
   <212> PRT
   <213> Human Immunodeficiency Virus-1
<400> 37
   Val Leu Ala Glu Ala Met Ser Gln Ala
   1 5
<210> 38
   <211> 9
   <212> PRT
   <213> Human Immunodeficiency Virus-1
<400> 38
   Ile Thr Leu Trp Gln Arg Pro Leu Val
   1 5
<210> 39
   <211> 8
   <212> PRT
   <213> Human Immunodeficiency Virus-1
<400> 39
   Phe Val Asn Thr Pro Pro Leu Val
   1 5
<210> 40
   <211> 9
   <212> PRT
   <213> Human Immunodeficiency Virus-1
<400> 40
   Tyr Leu Ala Trp Val Pro Ala His Lys
   1 5
<210> 41
   <211> 9
   <212> PRT
   <213> Human Immunodeficiency Virus-1
<400> 41
   Val Thr Val Tyr Tyr Gly Val Pro Val
   1 5
<210> 42
   <211> 9
   <212> PRT
   <213> Human Immunodeficiency Virus-1
<400> 42
   Thr Met Gly Ala Ala Ser Ile Thr Leu
   1 5
<210> 43
   <211> 9
   <212> PRT
   <213> Human Immunodeficiency Virus-1
<400> 43
   Leu Leu Gln Leu Thr Val Trp Gly Ile
   1 5
<210> 44
   <211> 9
   <212> PRT
   <213> Human Immunodeficiency Virus-1
<400> 44
   Tyr Ile Lys Ile Phe Ile Met Ile Val
   1 5
<210> 45
   <211> 9
   <212> PRT
   <213> Human Immunodeficiency Virus-1
<400> 45
   Leu Gln Leu Pro Pro Ile Glu Arg Leu
   1 5
<210> 46
   <211> 9
   <212> PRT
   <213> Human Immunodeficiency Virus-1
<400> 46
   Gly Met Gly Ser Pro Gln Ile Leu Val
   1 5
<210> 47
   <211> 9
   <212> PRT
   <213> Human Immunodeficiency Virus-1
<400> 47
   Ile Leu Val Glu Ser Pro Ala Val Leu
   1 5
<210> 48
   <211> 9
   <212> PRT
   <213> Human Immunodeficiency Virus-1
<400> 48
   Ala Leu Ala Ala Leu Ile Thr Pro Lys
   1 5
<210> 49
   <211> 9
   <212> PRT
   <213> Human Immunodeficiency Virus-1
<400> 49
   Lys Ile Lys Pro Pro Leu Pro Ser Val
   1 5
<210> 50
   <211> 9
   <212> PRT
   <213> Human Immunodeficiency Virus-1
<400> 50
   Lys Thr Lys Pro Pro Leu Pro Ser Val
   1 5
<210> 51
   <211> 9
   <212> PRT
   <213> Human Immunodeficiency Virus-1
<400> 51
   Phe Pro Arg Pro Trp Leu His Gly Leu
   1 5
<210> 52
   <211> 9
   <212> PRT
   <213> Human Immunodeficiency Virus-1
<400> 52
   Val Val Ala Ala Ile Ile Ala Ile Val
   1 5
<210> 53
   <211> 8
   <212> PRT
   <213> Human Immunodeficiency Virus-1
<400> 53
   Trp Cys Phe Lys Leu Val Pro Val
   1 5
<210> 54
   <211> 9
   <212> PRT
   <213> Human Immunodeficiency Virus-1
<400> 54
   Ser Leu Tyr Asn Thr Val Ala Thr Leu
   1 5
<210> 55
   <211> 9
   <212> PRT
   <213> Human Immunodeficiency Virus-1
<400> 55
   Val Ile Tyr Gln Tyr Met Asp Asp Leu
   1 5
<210> 56
   <211> 9
   <212> PRT
   <213> Human Immunodeficiency Virus-1
<400> 56
   Ile Leu Lys Glu Pro Val His Gly Val
   1 5
<210> 57
   <211> 9
   <212> PRT
   <213> Human Immunodeficiency Virus-1
<400> 57
   Lys Leu Thr Pro Leu Cys Val Thr Leu
   1 5
<210> 58
   <211> 13
   <212> PRT
   <213> Hepatitis B virus
<220>
   <223> Synthetic
<400> 58
   Thr Pro Pro Ala Tyr Arg Pro Pro Asn Ala Pro Ile Leu
   1 5 10
<210> 59
   <211> 15
   <212> PRT
   <213> Human Immunodeficiency Virus-1
<400> 59
   Lys Arg Trp Ile Ile Leu Gly Leu Asn Lys Ile Val Arg Met Tyr
   1 5 10 15
<210> 60
   <211> 20
   <212> PRT
   <213> Human Immunodeficiency Virus-1
<400> 60
   Val Trp Gly Ile Lys Gln Leu Gln Ala Arg Val Leu Ala Val Glu Arg
   1 5 10 15
   Tyr Leu Lys Asp
   20
<210> 61
   <211> 13
   <212> PRT
   <213> Artificial Sequence
<220> <221> MOD_RES

   <222> 1
   <223> D-alanine
<221> MOD_RES
   <222> 13
   <223> D-alanine
<221> MOD_RES
   <222> 3
   <223> Cyclohexylalanine
<223> Synthetic peptide
<400> 61
   Xaa Lys Xaa Val Ala Ala Trp Thr Leu Lys Ala Ala Xaa
   1 5 10
<210> 62
   <211> 15
   <212> PRT
   <213> Artificial Sequence
<220>
   <221> MOD_RES
   <222> 3
   <223> L-cyclohexylalanine
<221> MOD_RES
   <222> 13
   <223> D-alanine
<221> MOD_RES
   <222> 14
   <223> aminocaproic acid
<223> Synthetic peptide
<400> 62
   Ala Lys Xaa Val Ala Ala Trp Thr Leu Lys Ala Ala Xaa Xaa Cys
   1 5 10 15
<210> 63
   <211> 27
   <212> DNA
   <213> Human Immunodeficiency Virus-1
<400> 63
   ggcctggccg accagctgat ccacctg
   27
<210> 64
   <211> 27
   <212> DNA
   <213> Human Immunodeficiency Virus-1
<400> 64
   agcctggtga agcaccacat gtacgtg
   27
<210> 65
   <211> 27
   <212> DNA
   <213> Human Immunodeficiency Virus-1
<400> 65
   gcactagtgg agatgggaca tcacgtg
   27
<210> 66
   <211> 27
   <212> DNA
   <213> Human Immunodeficiency Virus-1
<400> 66
   cgcctgctga acgcctgggt gaaagtg
   27
<210> 67
   <211> 24
   <212> DNA
   <213> Human Immunodeficiency Virus-1
<400> 67
   ttcctgggca agatctggcc atct
   24
<210> 68
   <211> 27
   <212> DNA
   <213> Human Immunodeficiency Virus-1
<400> 68
   aagcttggca aggccggcta cgtggtg
   27
<210> 69
   <211> 27
   <212> DNA
   <213> Human Immunodeficiency Virus-1
<400> 69
   aacatctggg caacacacgc ctgcgtc
   27
<210> 70
   <211> 27
   <212> DNA
   <213> Human Immunodeficiency Virus-1
<400> 70
   gtgctggccg aagctatgag ccaggtc
   27
<210> 71
   <211> 27
   <212> DNA
   <213> Human Immunodeficiency Virus-1
<400> 71
   aacaccgtgg ctactctgta ctgtgtc
   27
<210> 72
   <211> 27
   <212> DNA
   <213> Human Immunodeficiency Virus-1
<400> 72
   tacatcaaga ttttcatcat gatcgtg
   27
<210> 73
   <211> 27
   <212> DNA
   <213> Human Immunodeficiency Virus-1
<400> 73
   agcttgggac aacatatcta cgagacc
   27